# FASCICULE DE BREVET EUROPEEN

(11) **EP 3 063 277 B1**
(45) Date de publication et mention de la délivrance du brevet: **12.09.2018**
(21) Numéro de dépôt: 14812611.3
(22) Date de dépôt: 31.10.2014
(51) Int. Cl.: C12N 15/62, C07K 16/28, C07K 14/47, A61K 38/17, A61P 43/00, C07K 16/18, C12N 15/12, C12N 15/13, C12N 15/79, G01N 33/68

(54) **PROTEINE CHIMERIQUE DANS LE TRAITEMENT DE L'AMYLOSE**
CHIMÄRES PROTEIN ZUR BEHANDLUNG VON AMYLOIDOSE
CHIMERIC PROTEIN FOR THE TREATMENT OF AMYLOIDOSIS

(30) Priorité: 31.10.2013 FR 1360702
(43) Date de publication de la demande: 07.09.2016
(73) Titulaire: Laboratoire Français du Fractionnement et des Biotechnologies, 91940 Les Ulis (FR); Centre National de la Recherche Scientifique, 75794 Paris Cedex 16 (FR); Université de Limoges, 87032 Limoges (FR)
(72) Inventeur: DE ROMEUF, Christophe, F-59130 Lambersart (FR); SIRAC, Christophe, F-87000 Limoges (FR)
(74) Mandataire: Cabinet Plasseraud
(86) Numéro de dépôt international: PCT/IB2014/065734
(87) Numéro de publication internationale: WO 2015/063728

(56) Documents cités:
- WO-A1-95/05394
- WO-A1-2009/000926
- WO-A1-2013/096847
- WO-A2-02/42462
- WO-A2-2008/143954
- WO-A2-2010/106180
- PICKEN MARIA M: "New insights into systemic amyloidosis: the importance of diagnosis of specific type.", CURRENT OPINION IN NEPHROLOGY AND HYPERTENSION MAY 2007, vol. 16, no. 3, mai 2007 (2007-05), pages 196-203, XP008170922, ISSN: 1062-4821 cité dans la demande

## Description

### DOMAINE DE L'INVENTION

La présente invention se rapporte au domaine de l'obtention de protéine chimérique spécifique à usage thérapeutique, notamment pour traiter l'amylose, en particulier l'amylose de type AL.

### ART ANTERIEUR

L'amylose, aussi appelée amyloïdose, est un vaste groupe de maladies appartenant au groupe des maladies conformationnelles des protéines englobant d'autres maladies telles que par exemple la maladie d'Alzheimer, les encéphalopathies spongiformes transmissibles, la maladie de Huntington ou le diabète de type II.

L'amylose est une maladie rare qui se caractérise par la présence de dépôts de protéines insolubles adoptant une conformation fibrillaire anormale dans les tissus. Le plus souvent, ce sont des fragments de protéines de précurseur sérique qui en sont la cause. De nombreux organes peuvent être affectés par ces dépôts extracellulaires, appelés « substance amyloïde ». Les principaux organes affectés par les dépôts amyloïdes sont le rein, le coeur, le tube digestif, le foie, la peau, le nerf périphérique et l'oeil. Les organes affectés par cette maladie présentent généralement un volume considérable. En définitive, l'amylose peut concerner tous les organes ainsi que le système nerveux central si bien qu'il existe des nombreux symptômes très variés.

L'amylose est une maladie sévère qui peut évoluer jusqu'à la destruction des organes atteints. Il existe plus de 25 protéines susceptibles de former les dépôts amyloïdes.

Il est admis généralement que la substance amyloïde est composée à environ 90 % de protéines fibrillaires qui sont caractéristiques de chaque variété d'amylose. Parmi les 10 % restant, environ 5 % sont composés de glycosaminoglycanes (GAG) et 5 % sont composés de glycoprotéines nommée composant-P amyloïde (ou protéine SAP), la SAP étant constamment présente quel que soit le type d'amylose.

Le tableau 1, ci-après, liste les principaux types d'amylose. Ces derniers sont nommés suivant la nature de la protéine impliquée qui elle-même sera désignée par le préfixe A (pour amylose) et un suffixe spécifique. Ainsi, la protéine amyloïde dérivée des chaînes légères d'immunoglobulines est désignée AL, et l'amylose constituée de ces chaînes est nommée « amylose AL » ; de même pour les protéines ATTR dérivées de la transthyrétine, l'amylose correspondante est nommée « amylose ATTR ».

**Tableau 1 : Nomenclature et classification des amyloses**

| **Protéine amyloïde** | **Précurseur** | **Diffusion** | **Syndromes ou tissus atteints** |
|---|---|---|---|
| AL | Chaine légère d'Ig (κ,λ) | G,L | (primitive) isolée ou associée au myélome ou à la maladie de Waldenström |
| AH | Chaine lourde d'Ig (□) | G,L | Isolée |
| AA | apoSAA | G,L | (secondaire) infections, inflammations chroniques, tumeurs, TRAPS, FMF, syndrome de Muckle et Wells |
| ATTR | transthyrétine mutée, transthyrétine notmale | G G | héréditaire sénile |
| Aβ2M rénale chronique | β2-microglobuline | G | Associée à l'insuffisance terminale |
| AAρoA1 | apoliproprotéine A1 | G L | héréditaire aortique (intima) |
| AApoA2 | apoliproprotéine A2 | G | héréditaire |
| AGel | gelsoline | G | héréditaire |
| ALys | lysosyme | G | héréditaire |
| AFib | fibrinogène | G | héréditaire |
| ACys | cystatine C | L | hémorragie cérébrale héréditaire |
| Aβ | précurseur de la protéine Aβ (AβPP) | L | Maladie d'Alzheimer trisomie 21 angiopathie amyloïde cérébrale héréditaire ou sporadique |
| APrPsc | précurseur de la protéine prion | L | Encéphalopathie spongiformes |
| ACal | procalcitonine | L | cancer médullaire de la thyroïde |
| AANF | facteur atrial natriurétique | L | Amylose auriculaire isolée |
| AIAPP | amyline | L | îlots de Langerhans du diabète de type 2, insulinome |
| Alns* | insuline | L | lactrogénique |
| APro* | prolactine | L | prolactinome, hypophyse sénile |
| AKep* | kératoépithéline | L | dystrophies cornéennes grillagées |
| Abri* | BRI-L | L | démence héréditaire britannique |
| ALact* | lactoferrine | L | vésicule séminale |
| Amed* | lactadhérine | L | Aortique (média) |
| FMF : fièvre méditerranéenne familiale ; TRAPS : *tumor necrosis factor* (TNF) *receptor-associated periodic syndrome* ; Ig : immunoglobuline ; G : amylose généralisée : L : amylose localisée ; * : nomenclature non officielle. Grateau G. Amyloses. Encyclopédie Orphanet, mai 2001, mise à jour juin 2003. | | | |

Le diagnostic de l'amylose repose sur l'identification des dépôts amyloïdes à l'examen anatomo-pathologique. Il exige donc des biopsies ciblées mais certains organes sont plus difficilement accessibles ou avec un risque non négligeable (coeur, cerveau...). Les substances amyloïdes peuvent être spécifiquement reconnues par un colorant, le rouge Congo, par l'observation d'une biréfringence dichroïque jaune-verte en lumière polarisée. Elles peuvent être également reconnues en microscopie électronique. Suite à l'identification de ces substances amyloïdes, la mise en oeuvre d'anticorps spécifiques permet d'identifier le type d'amylose considéré (Ac anti- SAA, CL kappa et lambda, fibrinogène, TTR, apoA1, apoA2, lysozyme, chaînes lourdes d'Ig) (Picken MM., Current Opin Nephrol Hypertens, 2007; 16: 196 ; Vrana JA et al., Blood, 2009; 114: 4957).

Plusieurs voies de traitement de l'amylose peuvent être envisagées, à savoir (1) l'élimination de la source de production, (2) l'inhibition de l'élongation des fibrilles ou (3) l'élimination active des dépôts amyloïdes.

Le traitement de la cause de la production de la protéine anormale (i.e. voie (1) ci-dessus) est le meilleur moyen de limiter l'évolution de la maladie amyloïde. En revanche, à ce jour, chaque type d'amylose requiert un traitement différent.

Pour l'amylose AL, forme la plus sévère où les atteintes sont le plus disséminées, le traitement consiste à réduire la formation des dépôts de substance amyloïde. Pour cela, il est nécessaire d'inhiber au maximum la production de l'immunoglobuline monoclonale responsable des dépôts. Le plus souvent, on pratique un traitement par chimiothérapie (par administration, notamment, de glucocorticoïdes (dexaméthasone) et d'antimitotiques). L'efficacité du traitement est évaluée par la mesure de la réduction du taux de la chaîne légère monoclonale dans le sang. Si cette réduction est importante, les dépôts de substance amyloïde, dont la formation est inhibée, vont être progressivement éliminés par l'organisme. Cela entraîne une amélioration progressive de l'état clinique. Le rythme d'élimination des dépôts est variable selon l'organe et selon les patients, et demande généralement plusieurs mois pour être apparent. Il n'existe pas, à l'heure actuelle, de traitement pouvant accélérer l'élimination des dépôts. Les amyloses AL peuvent être localisées ou disséminées, asymptomatiques ou au contraire de pronostic redoutable.

Parallèlement, des traitements spécifiques pour pallier la ou les insuffisances de fonctionnement du ou des organes affectés par l'amylose sont généralement mis en oeuvre. En cas d'atteinte sévère du coeur ou des reins, une transplantation peut être proposée.

Le traitement chimiothérapeutique de l'amylose AL comporte cependant de nombreux effets secondaires tels que chute des cheveux, diarrhée, nausées ou vomissements. La toxicité hématologique est le phénomène le plus récurrent et le plus grave et nécessite une surveillance constante du patient. Elle consiste en une diminution pouvant être importante de plusieurs éléments sanguins avec des risques conséquents : thrombopénie par chute des plaquettes sanguines avec un risque hémorragique, anémie, leucopénie avec un risque infectieux.

A un stade très avancé de l'amylose AL, les dégâts dans les organes sont tels que la chimiothérapie est inefficace. Une transplantation est alors nécessaire.

Pour l'amylose AA, le traitement de l'inflammation sous-jacente est la mesure thérapeutique la plus importante. L'efficacité des nouveaux traitements anti-inflammatoires (anti-TNF, anti-IL1) est en cours d'évaluation clinique. Mais pour le moment, cette amylose reste incurable et fatale, dans la mesure où il n'existe pas de traitement spécifique pouvant éliminer les dépôts plus rapidement.

Dans les amyloses de la transthyrétine (amylose héréditaire), une transplantation hépatique peut être proposée dans le but de supprimer la principale source de production de protéine TTR anormale. Ce traitement a démontré son efficacité à stopper l'évolution de la maladie dans la très grande majorité des cas de NAF traités à un stade précoce (90 %) et ce avec un recul moyen de 8 ans. De plus, ce traitement n'est pas efficace quand il est débuté à un stade pathologique avancé, en particulier quand il existe une atteinte cardiaque.

Concernant la voie de traitement consistant à inhiber l'élongation des fibrilles (i.e. voie (2) ci-dessus), il est connu de mettre en oeuvre le DMSO et la colchicine, ainsi que l'I-Dox, anthracycline interagissant avec les dépôts amyloïdes. Cependant, il semble que ces traitements, bien qu'ils induisent un retard dans l'apparition des dépôts amyloïdes, n'inhibent cependant pas ou peu leur élongation (Merlini G, Blood 93, 1999 : 1112).

Concernant la voie de traitement consistant à éliminer activement les dépôts amyloïdes (i.e. voie (3) ci-dessus), il est connu de mettre en oeuvre des anticorps monoclonaux murins reconnaissant un épitope conformationnel de l'amylose (11-1F4) (Solomon, Am. J. Path., 2000, 157 : 1239). Toutefois, il apparaît que ces anticorps semblent peu efficaces.

Par ailleurs, le document WO 2009/000926 propose l'administration, notamment, d'anticorps anti-SAP. Cette stratégie permet de recruter les macrophages et de conduire à l'élimination des dépôts amyloïdes. Cependant, cette stratégie a pour inconvénient de nécessiter une étape préalable d'administration de l'acide (R)-1-[6-[(R)-2-carboxy-pyrrolidine-1-yl]-6-oxo-hexanoyl]pyrrolidine-2-carboxylique (CPHPC) de manière à éliminer la SAP circulante (Pepys MB et al., Nature, 2010,468(7320): 93-7).

Par ailleurs, NEOD001 est un anticorps monoclonal en cours de développement par Prothena, qui cible spécifiquement les substances amyloïdes de type amylose AL ou AA

Cet anticorps réagit seulement avec la forme agrégée des protéines à l'origine de cette amylose AL.

D'autres alternatives thérapeutiques dans le traitement des amyloses sont à ce jour à l'étude. Peut à ce titre être cite le tafamidis qui permettrait de stabiliser la protéine circulante et empêcher la formation des dépôts, la doxycycline qui pourrait briser les fibrilles d'amyloïdes et empêchera la formation des dépôts (déjà testée sur les animaux (Ward J.E., Blood 2011, 118(25): 6610) et en phase II chez l'homme (Obici L. et al., Amyloid 2012: Suppl. 1 : 34)) ou encore le TUDCA, molécule anti-apoptose en cours de test.

En conséquence, et pour des raisons évidentes, la mise en oeuvre d'alternatives thérapeutiques efficaces dans le traitement des amylases demeure un objectif constant avec notamment la nécessité de disposer de traitements ayant pour but de ralentir la formation des dépôts OU à accélérer leur élimination, qui plus est dénués des inconvénients mentionnés ci-dessus.

La présente invention a précisément pour objectif de répondre à cette attente.

WO2009000926A1 porte sur l'utilisation d'un composé qui appauvrit un composant d'amyloïde P sérique de la circulation, en combinaison avec un anticorps spécifique pour un composant-P amyloïde sérique, pour le traitement ou la prophylaxie de l'amyloïdose.

WO2013/096847A1 concerne des protomères de pentraxine-2 (PTX-2) (aussi appelée composant-P amyloïde) fonctionnalisés et des pentamères de PTX-2 fonctionnalisés.

### RESUME DE L'INVENTION

Selon un premier de ses aspects, la présente invention concerne une protéine chimérique comprenant au moins un composant-P amyloïde humain et au moins un fragment d'une région Fc d'un anticorps humain, le composant-P amyloïde humain et le fragment d'une région Fc auquel il est rattaché étant liés l'un a l'autre par l'intermédiaire d'une région charnière.
Sous réserve d'une architecture appropriée, telle que définie ci-après, une telle protéine chimérique met en effet à profit, d'une part, l'affinité naturelle de la SAP pour les dépôts de substance amyloïde, conduisant ainsi à une amélioration de l'état clinique du patient traité.

La SAP étant présente dans tous les types d'amyloses, comme indiqué précédemment, la présente invention est particulièrement avantageuse en ce qu'elle est efficace quel que soit le type d'amylose considéré, systémique ou localisé.

Une protéine chimérique selon l'invention offre donc une alternative thérapeutique efficace dans le traitement des amyloses, avec en outre pour avantage d'être dénuée des effets secondaires indésirables attachés aux traitements conventionnels de l'amylose, tels que mentionnées ci-dessus.

Qui plus est, contrairement à la stratégie décrite dans WO 2009/000926, la présente invention ne nécessite pas une étape préliminaire consistant à administrer du CPHPC de manière à éliminer la SAP circulante.

Enfin, la mise en oeuvre de matériel d'origine humaine pour une protéine chimérique selon l'invention permet de garantir, d'une part, une innocuité maximale à l'égard des patients traités et, d'autre part, un niveau d'interaction avec les cibles considérées (figurées par les dépôts d'amyloïdes et les cellules effectrices) optimales.

En effet, l'utilisation de séquences humaines ou présentant une forte similarité avec les séquences humaines pour une protéine chimérique selon l'invention permet de diminuer le risque d'immunogénicité après administration, assurant ainsi une bonne tolérance des protéines chimériques administrées par l'organisme traité.

Selon un mode de réalisation particulier, le composant-P amyloïde humain présent au niveau d'une protéine chimérique selon l'invention peut être figuré par une séquence en acides aminés ayant au moins 80 % d'identité avec la séquence SEQ ID NO : 1.

Selon un autre mode de réalisation particulier, la région Fc de l'anticorps humain à l'origine de chacun des fragments de région Fc considérés au niveau d'une protéine chimérique selon l'invention peut être une région Fc d'une immunoglobuline humaine, de préférence une région Fc d'une IgG, de préférence une région Fc d'une IgG1 ou d'une IgG2, et plus particulièrement une région Fc d'une IgG1.

Selon un autre mode de réalisation particulier, le fragment de région Fc d'un anticorps humain considéré au niveau d'une protéine chimérique selon l'invention peut être représenté par au moins une séquence en acides aminés ayant au moins 80 % d'identité avec la séquence SEQ ID NO : 3.

Selon un mode de réalisation particulier, le fragment de région Fc d'un anticorps humain considéré au niveau d'une protéine chimérique selon l'invention peut présenter une affinité améliorée pour FcRn comparée à un fragment d'une région Fc d'un anticorps humain parent.

A ce titre, le fragment de région Fc d'un anticorps humain considéré au niveau d'une protéine chimérique selon l'invention peut comprendre au moins deux modifications au niveau de la séquence en acides aminées telles que définies dans WO 2010/106180, à savoir :
(i) une modification au niveau de la séquence en acides aminées choisie parmi le groupe consistant en 378V, 378T, 434Y et 434S, et
(ii) au moins une modification au niveau de la séquence en acides aminées choisie parmi le groupe consistant en 226G, 230S, 230T, 230L, 241 L, 264E, 307P, 315D, 330V, 362R, 378V, 378T, 389T, 389K, 434Y et 434S,
étant entendu que la numérotation des acides aminés de la région Fc est celle de l'index UE proposé par Kabat et à la condition que la modification (i) ne se produise pas à la même position en acides aminés que la modification (ii).

Ce mode de réalisation est avantageux en ce qu'il contribue à améliorer encore la capacité de recrutement des cellules effectrices, en particulier les polynucléaires neutrophiles et les monocytes macrophages impliqués, et donc l'élimination des dépôts amyloïdes.

D'autres modes de réalisation particuliers, décrits ci-après, permettent notamment d'améliorer encore la capacité de recrutement des cellules effectrices, plus particulièrement via une liaison améliorée entre les fragments de région Fc considérés aux récepteurs Fcgamma R, en particulier Fcgamma RIII ou CD16, exprimés en particulier à la surface des polynucléaires et macrophages.

Ainsi, selon un autre mode de réalisation particulier, un fragment de région Fc d'un anticorps humain considéré au niveau d'une protéine chimérique selon l'invention peut présenter une faible fucosylation au niveau des chaines oligosaccharidiques portées par Asn297 (numérotation Kabat) caractérisée par une teneur en fucose inférieure à 65 % par rapport à la teneur totale des structures glycanniques au niveau des chaines oligosaccharidiques portées par Asn297.

Dans un autre mode de réalisation, un fragment de région Fc d'un anticorps humain considéré au niveau d'une protéine chimérique selon l'invention peut présenter une faible fucosylation caractérisée par des structures glycanniques ayant une teneur inférieure à 50 % de formes G0F+G1F décrites ci-après par rapport à la teneur totale des structures glycanniques. Par exemple, un fragment de région Fc d'un anticorps humain considéré au niveau d'une protéine chimérique selon l'invention peut comprendre en outre une teneur supérieure à 60 % pour les formes G0+G1+G0F+G1F, les formes G0F+G1F étant inférieures à 50 %.

Dans un autre mode de réalisation particulier, les anticorps peuvent comprendre une teneur supérieure à 60 % pour les formes G0+G1+G0F+G1F, la teneur en fucose étant inférieure à 65 %.

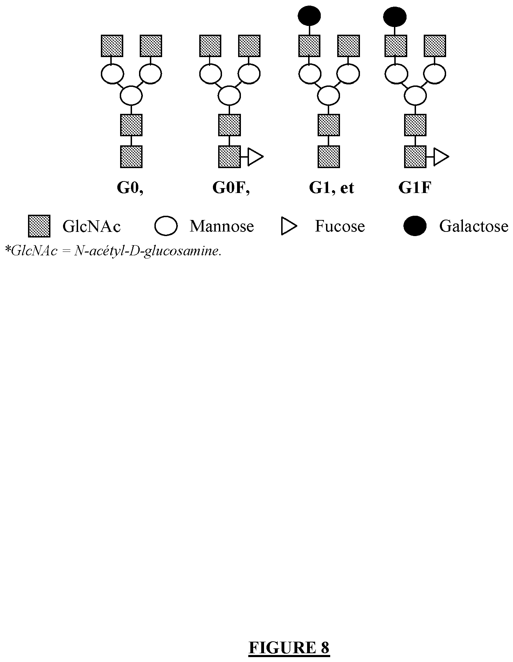

Ces formes G0, G0F, G1 et G1F sont plus particulièrement illustrées en figure 8 ci-après.

Selon le dernier mode de réalisation décrit ci-dessus, le fragment de région Fc d'un anticorps humain considéré au niveau d'une protéine chimérique selon l'invention peut comprendre en outre, sur un site de glycosylation Asn297, une structure glycannique présentant des mannoses terminaux et/ou des N-acétylglucosamines terminaux non intercalaires.

Le fragment de région Fc d'un anticorps humain considéré au niveau d'une protéine chimérique selon l'invention peut comprendre, sur le site de glycosylation Asn297, une structure glycannique de type biantenné, avec des chaînes courtes, une faible sialylation, et une teneur supérieure à 60 % pour les formes G0+G1+G0F+G1F, les formes G0F+G1F étant inférieures à 50 %. Par exemple, le fragment de région Fc d'un anticorps humain considéré au niveau d'une protéine chimérique selon l'invention peut posséder une teneur en acide sialique inférieure à 25 %, 20 %, 15 %, ou 10 %, de préférence 5 %, 4 %, 3 % ou 2 %.

Le fragment de région Fc d'un anticorps humain considéré au niveau d'une protéine chimérique selon l'invention peut comprendre une teneur supérieure à 60 %, de préférence supérieure à 80 % pour les formes G0+G1+G0F+G1F étant entendu que les formes G0F+G1F sont inférieure à 50 %, de préférence inférieure à 30 %.

Le fragment de région Fc d'un anticorps humain considéré au niveau d'une protéine chimérique selon l'invention peut comprendre des structures glycanniques de type biantennées, avec des chaînes courtes, une faible sialylation, des mannoses et des N-acétylglucosamines du point d'attache terminaux non intercalaires, les structures glycanniques ayant une teneur supérieure à 60 % pour les formes G0+G1+G0F+G1F, et une faible fucocylation, les structures glycanniques ayant une teneur inférieure à 50 % de formes G0F+G1F.

Certains des modes de réalisation particuliers décrits ci-dessus sont notamment décrits plus en détails dans WO 01/77181 et WO 2012/175874.

Comme indiqué ci-dessus, une protéine chimérique selon l'invention comprend une région charnière entre le composant-P amyloïde humain et le fragment d'une région Fc d'un anticorps humain auquel ledit composant-P amyloïde humain est rattaché.

Cette construction a pour avantage d'assurer une plus grande accessibilité aussi bien au composant-P amyloïde humain qu'au fragment d'une région Fc d'un anticorps humain et confère également une plus grande flexibilité à une protéine chimérique selon l'invention. Ce mode de réalisation est avantageux en ce qu'il contribue donc à améliorer la capacité de la SAP humaine à reconnaître les dépôts amyloïdes ainsi que la capacité des fragments de régions Fc à recruter des cellules effectrices, en particulier les polynucléaires neutrophiles et les monocytes macrophages impliqués, et donc l'élimination des dépôts amyloïdes.

Sous réserve d'une architecture appropriée, telle que définie ci-après, l'association de deux fragments de région Fc d'un anticorps humain telle que rencontrée dans les immunoglobulines confère à une protéine chimérique selon l'invention une capacité d'interaction avec les récepteurs Fc comparable à celle des immunoglobulines. Cette association résulte donc en une région fonctionnelle capable de recruter des cellules effectrices et d'assurer l'élimination des plaques amyloïdes.

La nature de la région charnière relève des connaissances de l'homme du métier.

Plus particulièrement, une région charnière selon l'invention peut être une séquence peptidique spécifique comprenant au moins un résidu cystéine ou une molécule non-peptidique telle que du Polyéthylène Glycol (PEG).

De préférence, une région charnière conforme à l'invention peut être choisie parmi les régions charnières de l'IgG1 Humaine, l'IgG2 Humaine, l'IgG3 Humaine ou l'IgG4 Humaine.

Encore plus préférentiellement, une région charnière peut comprendre au moins une séquence en acides aminés ayant au moins 60 % d'identité, de préférence au moins 80 % d'identité, avec une séquence choisie parmi les séquences SEQ ID NO : 13 et 15 à 18, en particulier la séquence SEQ ID NO : 13.

Une région charnière selon l'invention peut également être figurée par des variants des séquences SEQ ID NO : 13 et 15 à 18.

A titre illustratif de variants de région charnière comprenant au moins une séquence en acides aminés ayant au moins 60 % d'identité, de préférence au moins 80 % d'identité, avec les séquences SEQ ID NO : 13 et 15 à 18, on peut citer la séquence SEQ ID NO : 13 dans laquelle la première Cystéine est mutée par une Sérine.

Selon un mode de réalisation particulièrement préféré, une région charnière selon l'invention peut être figurée par la séquence en acides aminés SEQ ID NO: 19.

Selon un mode de réalisation particulier, une région charnière selon l'invention peut en outre inclure une séquence peptidique non structurante entre la région charnière et le composant-P amyloïde.

En particulier, une telle séquence peptidique non structurante peut être représentée par une séquence en acides aminés SEQ ID NO : 5.

Cette séquence SEQ ID NO : 5, de motif (GGGGS)n (ou G₄S)n, est donc plus ou moins longue en fonction de la flexibilité recherchée. A cet égard, « n » est de préférence compris entre 1 et 5, et de préférence est égal à 3.

Comme indiqué précédemment, la capacité d'une protéine chimérique selon l'invention à présenter une affinité naturelle de la SAP humaine pour les dépôts amyloïdes mais surtout à recruter les cellules effectrices, en particulier les polynucléaires neutrophiles et les monocytes macrophages impliqués dans l'élimination des dépôts amyloïdes via le fragment de région Fc d'un anticorps humain, est conditionnée par une architecture appropriée, telle que définie ci-après.

Ainsi, selon une première variante de réalisation, la protéine chimérique selon l'invention peut comprendre au moins deux motifs, chaque motif comprenant au moins un composant-P amyloïde humain et au moins un fragment d'une région Fc d'un anticorps humain liés l'un à l'autre par l'intermédiaire d'une région charnière, les deux motifs étant liés l'un à l'autre de manière covalente par au moins un pont disulfure.

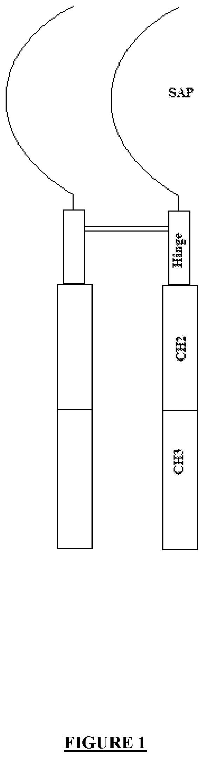

Cette architecture spécifique est notamment désignée dans la suite de la présente demande par l'expression « *SAP-Fc dimérique* » et est illustrée en figure 1.

Cette première variante de réalisation conduit à la formation d'une protéine chimérique dimérique. Cette architecture spécifique est avantageuse en ce qu'elle améliore la capacité de recrutement des cellules effectrices, en particulier les polynucléaires neutrophiles et les monocytes macrophages impliqués, et donc l'élimination des dépôts amyloïdes.

Pour chaque motif, ledit composant-P amyloïde humain et ledit fragment d'une région Fc d'un anticorps humain sont liés l'un à l'autre par une région charnière, telle que définie précédemment.

Selon cette variante de réalisation, les fragments de région Fc d'un anticorps humain des différents motifs considérés peuvent être identiques ou différents.

A titre d'alternative, une protéine chimérique SAP-Fc dimérique selon l'invention peut comprendre au moins deux motifs, chaque motif comprenant au moins un composant-P amyloïde humain et au moins un fragment d'une région Fc d'un anticorps humain liés l'un à l'autre par l'intermédiaire d'une région charnière, mais les deux motifs étant liés l'un à l'autre par une liaison formée d'au moins deux groupements de poly(éthylène glycol) (PEG).

Une protéine chimérique de type SAP-Fc dimérique résultant de la liaison de deux motifs tels que définis ci-dessus, chaque motif étant lié l'un à l'autre par au moins un pont disulfure ou par une liaison formée d'au moins deux groupements PEG, la présente invention concerne également une protéine chimérique figurée par un unique motif à titre de composé intermédiaire.

Ainsi, la présente invention concerne également, à titre de composé intermédiaire, une protéine chimérique comprenant un unique composant-P amyloïde humain et un unique fragment d'une région Fc d'un anticorps humain, le composant-P amyloïde humain et le fragment d'une région Fc étant liés l'un à l'autre par l'intermédiaire d'une région charnière.

Un tel composé intermédiaire peut également être désigné dans la suite de la présente description par l'expression « *SAP-Fc monomérique ».*

Un tel composé intermédiaire peut être représenté par une séquence en acides aminés ayant au moins 80 %, de préférence au moins 90 %, d'identité avec la séquence SEQ ID NO:7.

Un protocole de préparation d'une protéine chimérique SAP-Fc dimérique telle que définie précédemment en partant d'une protéine chimérique intermédiaire « *SAP-Fc monomérique* » relève des connaissances générales de l'homme du métier.

Un tel aspect est notamment décrit ultérieurement dans les exemples.

Selon une autre variante de réalisation, la protéine chimérique selon l'invention peut comprendre au moins un composant-P amyloïde humain et au moins deux fragments d'une région Fc d'un anticorps humain (i.e. premier et second fragments d'une région Fc d'un anticorps humain), identiques ou différents, ledit composant-P amyloïde humain et ledit premier fragment d'une région Fc d'un anticorps humain étant liés l'un à l'autre par l'intermédiaire d'une région charnière, les premier et second fragments d'une région Fc étant liés l'un à l'autre de manière covalente par l'intermédiaire d'une liaison formée d'une chaine espaceur et d'une région charnière identique ou différente à celle évoquée précédemment, et forment une seule chaîne polypeptidique constituant une région Fc dimérique fonctionnelle.

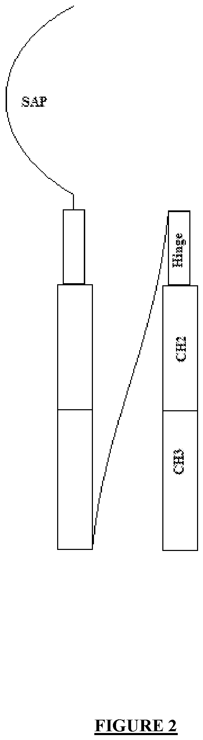

Cette architecture spécifique est notamment désignée dans la suite de la présente demande par l'expression « *SAP-ScFc monomérique* » et est illustrée en figure 2.

En d'autres termes, une protéine SAP-ScFc monomérique selon l'invention comprend, de la partie N-terminale à la partie C-terminale, (1) un composé-P amyloïde humain, (2) une région charnière, (3) un premier fragment de région Fc d'un anticorps humain, (4) une chaine espaceur, (5) une région charnière, identique ou différente à celle considérée en (2) ci-dessus, et (6) un second fragment de région Fc d'un anticorps humain, identique ou différent à celui considéré en (3) ci-dessus.

Des protéines possédant cette architecture particulière, qui peut être dénommée *« single chain Fc* » ou « *ScFc* », comprenant au moins deux fragments d'une région Fc d'un anticorps humain, identiques ou différents, et étant liés l'un à l'autre de manière covalente pour former une seule chaîne polypeptidique constituant une région Fc dimérique fonctionnelle, est notamment décrite dans WO 2008/143954.

A cet égard, la chaine espaceur considérée dans la liaison entre les premier et second fragments d'une région Fc peut de préférence être représentée par une séquence en acides aminés SEQ ID NO : 5.

A ce titre, cette séquence SEQ ID NO : 5, de motif (GGGGS)n (ou G₄S)n, est donc plus ou moins longue en fonction de la flexibilité recherchée. A cet égard, « n » est de préférence compris entre 1 et 5, et de préférence égal à 3.

Par ailleurs, le composant-P amyloïde humain et le premier fragment d'une région Fc d'un anticorps humain auquel ledit composant-P amyloïde humain est attaché sont séparés l'un de l'autre par l'intermédiaire d'une région charnière telle que définie précédemment.

Selon encore une autre variante de réalisation, une protéine chimérique selon l'invention peut résulter de l'appariement naturel de deux protéines chimériques SAP-ScFc monomériques, liées l'une à l'autre par l'intermédiaire d'au moins deux ponts disulfure ou d'au moins deux liaisons, une liaison étant formée d'au moins deux groupements poly(éthylène glycol) (PEG).

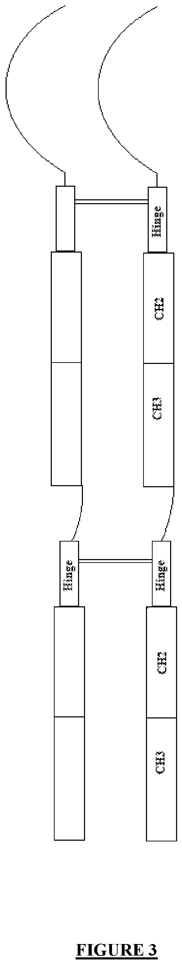

Cette architecture spécifique est notamment désignée dans la suite de la présente demande par l'expression « *SAP-ScFc dimérique* » et est illustrée en figure 3.

Ces trois variantes de réalisations SAP-Fc dimérique, SAP-ScFc monomérique et SAP-ScFc dimérique d'une protéine chimérique selon l'invention comprennent toutes au moins deux fragments de région Fc, qui résulte donc en une région fonctionnelle à l'origine de la capacité de ces architectures particulières à recruter des cellules effectrices et ainsi d'assurer l'élimination des plaques amyloïdes.

Par ailleurs, les protéines chimériques SAP-Fc monomérique et SAP-ScFc monomérique selon l'invention et telles que décrites précédemment peuvent s'apparier pour former une structure pentamérique, ou encore décamérique (i.e. résultant de deux structures pentamériques interagissant face à face). En effet, il a été montré que la protéine SAP sérique peut s'associer pour former des structures pentamérique et dimérique (Hutchinson et al., 2000, Molecular Medicine, 6 :482).

Selon un autre de ses aspects, la présente invention concerne un acide nucléique comprenant au moins une séquence de polynucléotides codant au moins une protéine chimérique selon l'invention.

La formation des structures SAP-Fc dimérique et SAP-ScFc dimérique résultant d'un phénomène d'appariement naturel, la séquence de polynucléotides code de préférence au moins la protéine chimérique SAP-Fc monomérique ou SAP-ScFc monomérique.

Selon encore un autre de ses aspects, la présente invention concerne un vecteur dans lequel est inséré un acide nucléique tel que défini ci-dessus.

Selon un autre de ses aspects, la présente invention concerne une cellule hôte transfectée avec un vecteur tel que défini précédemment.

Selon encore un autre de ses aspects, la présente invention concerne un procédé de fabrication d'une protéine chimérique telle que définie précédemment, ledit procédé comprenant au moins les étapes consistant à :
a) transfecter une cellule hôte avec un vecteur tel que défini ci-dessus;
b) cultiver ladite cellule hôte dans des conditions telles que la protéine chimérique est produite; et
c) collecter ladite protéine chimérique produite par ladite cellule hôte à l'issue de l'étape b).

Eu égard à la protéine chimérique SAP-Fc dimérique, il est à noter que l'étape consistant à lier, de manière covalente par l'intermédiaire d'au moins un pont disulfure, les deux motifs comprenant chacun au moins un composant-P amyloïde humain et au moins un fragment d'une région Fc d'un anticorps humain avec, pour chaque motif, ledit composant-P amyloïde humain étant lié audit fragment d'une région Fc auquel il est rattaché par l'intermédiaire d'une région charnière, est une étape se produisant naturellement dans la cellule hôte.

De la même façon, l'appariement de deux protéines SAP-ScFc monomérique pour l'obtention de la protéine chimérique SAP-ScFc dimérique se fait naturellement dans la cellule hôte, via la formation d'au moins deux ponts disulfure.

Selon encore un autre de ses aspects, la présente invention concerne une composition pharmaceutique comprenant une protéine chimérique selon l'invention et un excipient pharmaceutiquement acceptable.

Selon encore un autre de ses aspects, la présente invention concerne une protéine chimérique telle que définie ci-dessus, pour son utilisation comme médicament.

Selon encore un autre de ses aspects, la présente invention concerne une protéine chimérique telle que définie ci-dessus, pour son utilisation pour éliminer les dépôts d'amylose au niveau des organes.

Selon encore un autre de ses aspects, la présente invention concerne une protéine chimérique telle que définie ci-dessus, pour induire une activation de la phagocytose du ligand reconnu par le composant-P amyloïde humain par les polynucléaires neutrophiles et/ou les monocytes macrophages.

Selon encore un autre de ses aspects, la présente invention concerne une protéine chimérique telle que définie ci-dessus, pour son utilisation pour traiter l'amylose, en particulier l'amylose de type AL.

Selon encore un autre de ses aspects, il est décrit un procédé pour traiter l'amylose, en particulier l'amylose de type AL, comprenant au moins une étape d'administration à un individu en ayant besoin d'une quantité efficace d'au moins un premier agent actif représenté par une protéine chimérique ou une composition telle que définie précédemment.

Selon un mode de réalisation particulier, ledit procédé peut en outre comprendre une étape d'administration, antérieurement, concomitamment et/ou postérieurement à l'étape d'administration du premier agent actif, d'au moins un second agent actif distinct dudit premier agent actif, de préférence le second agent actif étant choisi parmi le groupe comprenant le 4-[bis(chloroéthyl)amino]phénylalanine (melphalan) ; le 9-fluoro-11β,17,21-trihydroxy-16a-méthylprégna-1,4-diène-3,20-dione (dexaméthasone) ; la prednisone ; le diméthylsulfoxyde (DMSO) ; le N-[(7S)-5,6,7,9-tétrahydro-1,2,3,10-tétraméthoxy-9-oxobenzo[a]heptalen-7-yl)acétamide](colchicine) ; le 7S,9S)-7-[(2R,4S,5S,6S)-4-amino-5-hydroxy-6-methyloxan-2-yl]oxy-6,9,11-trihydroxy-9-(2-hydroxyacetyl)-4-methoxy-8,10-dihydro-7H-tetracene-5,12-dione (Doxorubicin) ; des actifs aptes à réagir avec le composant-P amyloïde, en particulier de type bis-d-proline, et plus particulièrement le composé (R)-1-[6-[(R)-2-carboxy-pyrrolidin-1-yl]-6-oxo-hexa-noyl]pyrrolidine-2 carboxylic acid) ; les inhibiteurs du protéasome, par exemple le bortezomib, le carfilzomib, le marizomib, l'ixazomib, le delanzomib, l'ONX-912 ou le Revlimid ; et un de leur mélange.

Selon encore un autre de ses aspects, la présente invention concerne l'utilisation d'au moins une protéine chimérique telle que définie ci-dessus, à titre d'outil de caractérisation *in vitro* ou *ex vivo* de la présence d'un dépôt d'amylose.

Selon encore un autre de ses aspects, la présente invention concerne l'utilisation d'au moins une protéine chimérique telle que définie ci-dessus, à titre d'outil de caractérisation *in vitro* ou *ex vivo* de la présence d'un dépôt d'amylose.

Selon une première variante de réalisation, la protéine chimérique est radiomarquée, de préférence le composant-P amyloïde humain est figuré par ¹²³I-SAP.

Selon une seconde variante de réalisation, la protéine chimérique est identifiée au moyen d'un anticorps anti-Fc radiomarqué, par exemple ledit anticorps anti-Fc est couplé à la péroxydase.

Une protéine chimérique selon l'invention peut encore être identifiée au moyen de toute technique connue de l'homme de l'art. A ce titre peut notamment être cité la technique via le couplage avec des fluorophores et des microbulles telle que décrite dans Neri et al. (1997) Nat Biotech, ou encore les techniques décrites dans Brack et al. (2005) EJNM, Santimaria (2003) Clinical cancer res, Borsi et al. (2002) Int J cancer, Berndorff (2006) J Nucl Med et Joseph et al. (2004) Pham Res.

### DESCRIPTION DES FIGURES

La **Figure** 1 illustre une protéine chimérique selon l'invention comprenant deux motifs comprenant chacun un composant-P amyloïde humain et un fragment d'une région Fc d'un anticorps humain liés l'un à l'autre par une région charnière, les deux motifs étant liés l'un à l'autre de manière covalente par un pont disulfure (= protéine chimérique SAP-Fc dimérique).
La **Figure** 2 illustre une protéine chimérique selon l'invention comprenant un composant-P amyloïde humain et deux fragments d'une région Fc d'un anticorps humain (i.e. premier et second fragments d'une région Fc d'un anticorps humain), le composant-P amyloïde humain étant rattaché au premier fragment d'une région Fc par une région charnière et les deux fragments d'une région Fc, identiques ou différents, étant liés l'un à l'autre de manière covalente par une liaison formée d'une chaine espaceur et d'une région charnière et forment une seule chaîne polypeptidique constituant une région Fc dimérique fonctionnelle (= protéine chimérique SAP-ScFc monomérique).
La **Figure** 3 illustre une protéine chimérique selon l'invention résultant de l'appariement de deux protéines chimériques SAP-ScFc monomériques liées l'une à l'autre par deux ponts disulfure (= protéine chimérique SAP-ScFc dimérique).

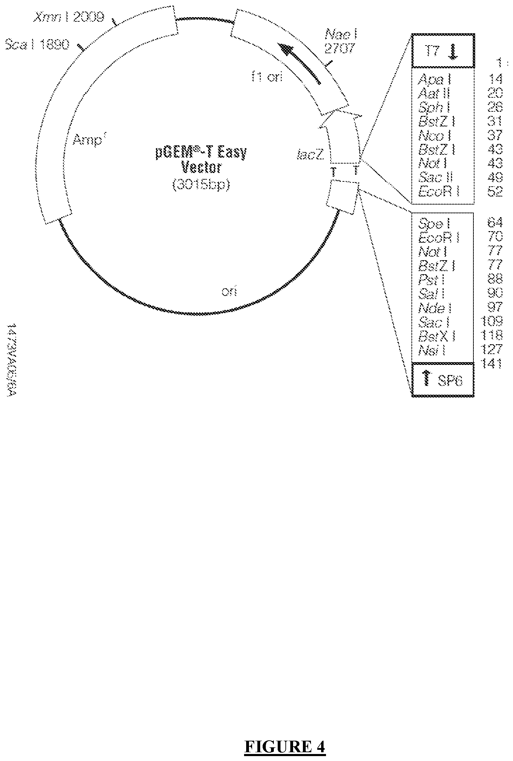

La **Figure** 4 illustre le vecteur de clonage commercial pGEM®-T Easy (Promega).

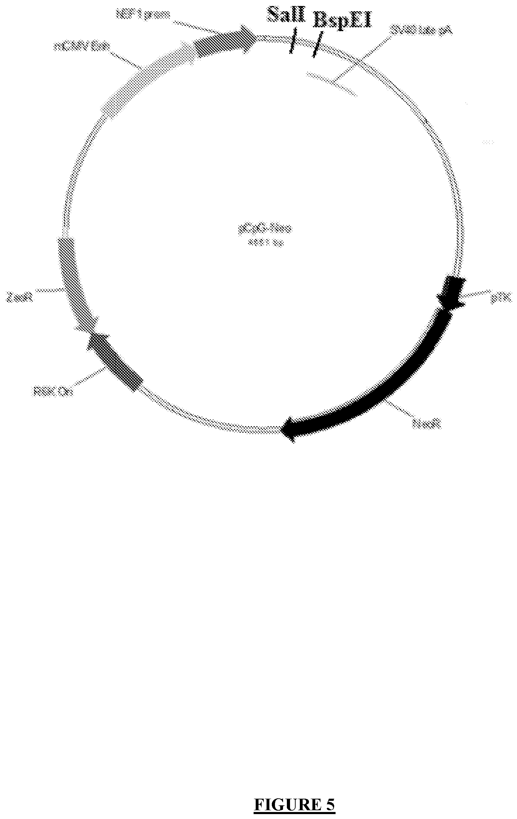

La **Figure** 5 illustre vecteur d'expression commerciale pCpG (invivogen).

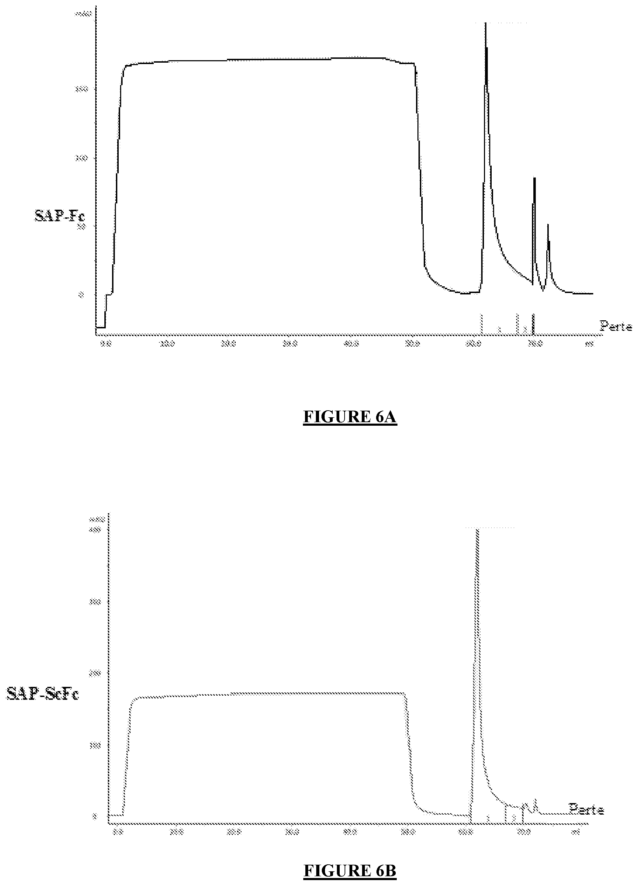

La **Figure** 6 illustre les résultats de l'étape de purification sur protéine A selon l'invention considérée en exemple 3 ci-après, pour les protéines chimériques SAP-Fc dimériques **(****Figure** 6A) et SAP-ScFc monomériques **(****Figure** 6B) (Abscisse : volume injecté en ml ; Ordonnée : Mesure de la densité optique à 280 nm en Unité Arbitraire). Sur les figures 6A et 6B, les pics localisés entre 0 et 60 ml correspondent au volume d'exclusion. Sur les figures 6A et 6B, les pics localisés entre 60 et 70 ml correspondent aux protéines chimériques selon l'invention.

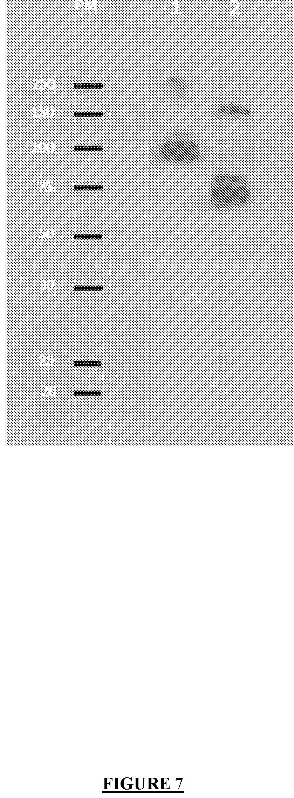

La **Figure** 7 illustre les résultats de l'étape de purification par Western blot considérée en exemple 3 ci-après, pour les protéines chimériques SAP-Fc et SAP-ScFc. Échantillon 1: caractérise la SAP-Fc : Échantillon 2: caractérise la SAP-ScFc. La bande à 75 kDa correspond à l'architecture SAP-ScFc monomérique. La bande à 100 kDa correspond à l'architecture SAP-Fc dimérique.
La **Figure** 8 illustre les structures glycaniques susceptibles d'être considérées dans le cadre de la fucosylation d'une région Fc d'un anticorps humain.

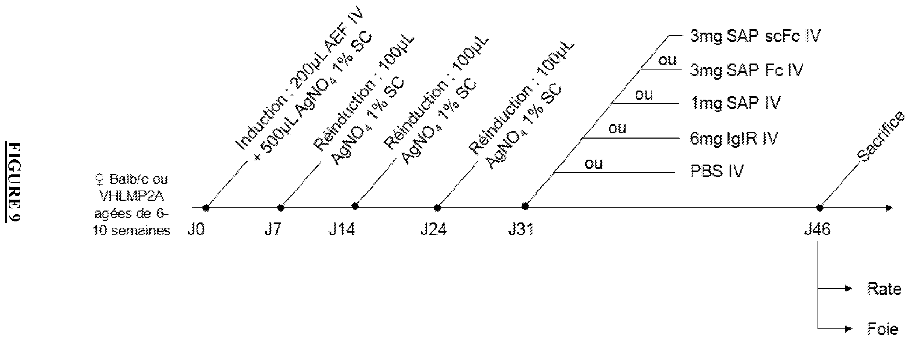

La **Figure** 9 illustre le protocole d'induction de l'amylose AA chez des souris Balb/c et VH-LMP2A. Dans cette figure 9, AEF IV = « Amyloid Enhancing Factor » en Intra-Veineuse ; SC = « Sous-cutané» ; IgIR = « Immunoglobuline Irrelevante anti-FVIII » (IgG1 humaine).

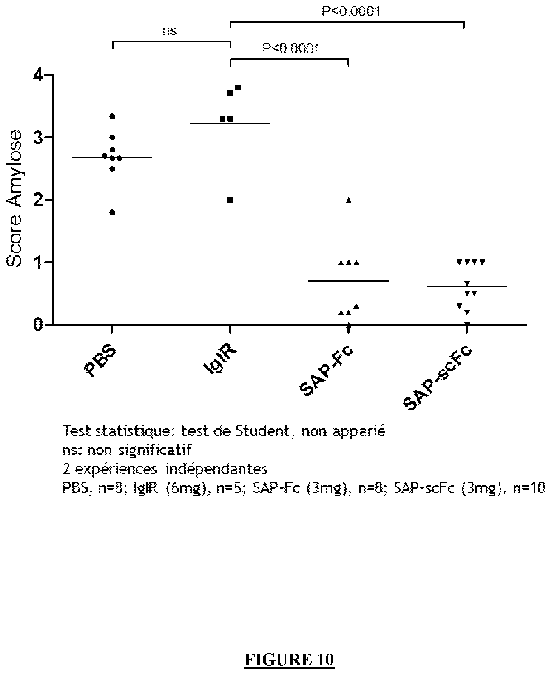

La **Figure 10** illustre l'effet de différents composés, dont les protéines chimériques SAP-Fc et SAP-ScFc selon l'invention, sur les dépôts d'amylose induits chez des souris Balb/c traitées selon le protocole détaillé en figure 9. La figure 10 illustre plus particulièrement le taux de dépôts d'amylose observés chez des souris Balb/c ayant subies des injections de PBS, de protéines IgG1 irrelevantes IgIR (Contrôle) ou de protéines chimériques SAP-Fc ou SAP-ScFc selon l'invention. Ces résultats sont obtenus à partir des rates desdites souris.

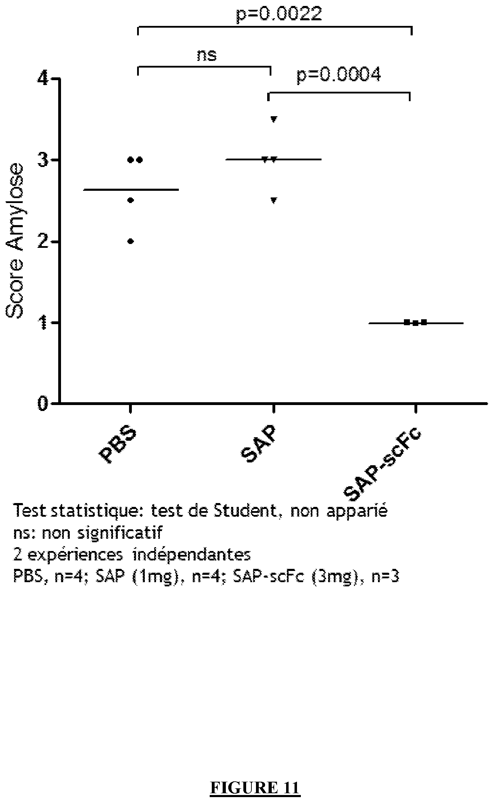

La **Figure 11** illustre l'effet de différents composés, dont les protéines chimériques SAP-ScFc selon l'invention, sur les dépôts d'amylose induits chez des souris VH-LMP2A traitées selon le protocole détaillé en figure 9. La figure 11 illustre plus particulièrement le taux de dépôts d'amylose observés chez des souris VH-LMP2A ayant subies des injections de PBS, de protéines SAP en tant que telles (i.e. dénuée du fragment de région Fc d'un anticorps humain) ou de protéines chimériques SAP-ScFc selon l'invention. Ces résultats sont obtenus à partir des rates desdites souris.

### DESCRIPTION DETAILLEE DE L'INVENTION

Comme indiqué ci-dessus et illustré dans les exemples ci-après, les inventeurs ont montré qu'une protéine chimérique, en mettant à profit, d'une part, l'affinité naturelle de la SAP pour les dépôts amyloïdes et, d'autre part, la capacité de la région Fc des anticorps à recruter les cellules effectrices, en particulier les polynucléaires neutrophiles et les monocytes macrophages, est particulièrement avantageuse sur le plan de l'élimination des dépôts amyloïdes et ce, quel que soit le type d'amylose considéré.

### Définitions

Préliminairement, pour que la demande puisse être mieux comprise, plusieurs définitions sont énoncées ci-dessous. Ces définitions sont censées englober les équivalents grammaticaux.

Par « protéine chimérique », on entend désigner, au sens de la présente invention, une protéine constituée d'une première séquence d'acides aminés dérivée d'une première source, liée, de manière covalente, à une seconde séquence d'acides aminés dérivée d'une seconde source, dans laquelle la première et la deuxième source ne sont pas identiques ou, en d'autres termes, caractérisent deux entités biologiques différentes. En l'espèce, l'une parmi la première ou seconde séquence d'acides aminés représente au moins un composant-P amyloïde humain et l'autre représente au moins un fragment d'une région Fc d'un anticorps humain. Dans le cadre de la présente invention, la première et la seconde séquence d'acides aminés sont liées l'une à l'autre, par l'intermédiaire d'une région charnière, notamment telle que définie précédemment.

Dans le cadre de la présente invention, une protéine chimérique entend désigner également des formes dimériques d'une protéine telle que définie ci-dessus, ou d'un de ses dérivés.

Par « polynucléotide », on entend selon l'invention un composé comprenant un polymère de nucléotides, ce qui englobe un polymère de ribonucléotides (ARN) et un polymère de désoxyribonucléotides (ADN), lesdits nucléotides étant éventuellement modifiés chimiquement. Selon l'invention, un polynucléotide peut avoir avantageusement une longueur allant de 5 à 3 000 nucléotides de longueur. Un polynucléotide englobe classiquement les poly-ribonucléotides et les poly-désoxyribonucléotides, le cas échéant chimiquement modifiés. Dans certains modes de réalisation, un polynucléotide consiste en un polymère de nucléotides, ribonucléotides ou désoxyribonucléotides. Dans d'autres modes de réalisation, un acide nucléique consiste essentiellement en un polymère de nucléotides et comprend une partie non-nucléotidique, ladite partie non-nucléotidique étant préférentiellement de longueur réduite, comparée à la longueur de la partie nucléotidique, par exemple une longueur linéaire inférieure à la longueur occupée par une chaîne de cinq nucléotides, ribonucléotides ou désoxyribonucléotides. Préférentiellement, la partie non-nucléotidique n'est pas de nature polypeptidique, ou alternativement ne contient pas de résidu d'acide aminé. Un polynucléotide peut comprendre un ou plusieurs nucléotides modifiés, tels que des nucléotides méthylés, les modifications pouvant être réalisées avant, pendant, ou après l'assemblage du polymère de nucléotides. Dans certains modes de réalisation, la partie non nucléotidique peut consister en une chaîne espaceur comprenant un groupe carboxyle libre ou en une chaîne espaceur comprenant un groupe amine libre.

Aux fins de la présente description, les termes « entité polynucléotidique » et « polynucléotide » sont utilisés indifféremment pour désigner le même objet.

Par « polypeptide », on entend selon l'invention une chaîne d'au moins deux résidus d'acides aminés, ce qui englobe une chaîne allant de 2 à 1 000 résidus d'acides aminés de longueur, lesdits résidus d'acides aminés étant liés entre eux par des liaisons covalentes, y compris par des liaisons peptidiques. Dans la présente description, le terme « polypeptide » peut être utilisé pour désigner indifféremment une protéine, un peptide ou un oligopeptide. Le terme « polypeptide » englobe les polypeptides chimiquement modifiés par un ou plusieurs groupes fonctionnels ou par une ou plusieurs molécules non peptidiques, à l'exception d'une modification par un polynucléotide.

Aux fins de la présente description, les termes « entité polypeptidique » et « polypeptide » sont utilisés indifféremment pour désigner le même objet.

Par « acide aminé », on englobe l'un quelconque des 22 résidus d'acides aminés naturels, ainsi que leurs analogues non naturels. Les acides aminés englobent les résidus Alanine (Ala; A), Arginine (Arg; R), Asparagine (Asp; N), acide Aspartique (Asp; D), Cystéine (Cys; C), acide Glutamique (Glu; E), Glutamine (Gln; Q), Glycine (Gly; G), Histidine (His; H), Isoleucine (Ile; I), Leucine (Leu; L), Lysine (Lys; K), Methionine (Met; M), Phénylalanine (Phe; F), Proline (Pro; P), Sérine (Ser; S), Thréonine (Thr; T), Tryptophane (Trp; W), Tyrosine (Tyr; Y), Valine (Val; V), Pyrrolysine et Sélénocystéine. Les acides aminés englobent le L-acides aminés et les D-acides aminés. Préférentiellement, les acides aminés sont les L-acides aminés.

Par « composant-P amyloïde » ou « protéine SAP », on entend désigner, au sens de la présente invention, une glycoprotéine, appartenant à la famille des protéines pentraxine, qui a une organisation caractéristique pentamérique. C'est une glycoprotéine constituant tous les dépôts amyloïdes, notamment ceux isolés du pancréas de diabétiques à long terme. Elle est formée de 10 sous-unités polypeptidiques glycosylées identiques, associées de façon non covalente pour former deux pentamères disposés en deux filaments hélicoïdaux. Chaque sous-unité de la structure pentagonale obtenue a un poids moléculaire de 23 à 25000 Daltons. La SAP fait partie de la famille des pentraxines comme le CRP (c-reactive protein) avec qui elle présente une homologie de structure, les parties N-terminale étant homologues. La SAP est présente naturellement dans le sang et ce, à hauteur environ de 20 à 30 mg/L de sang (Pepys, M.B., et al., Ann Rev Med, 2006 ; 57:223-41.)

Par « Fc » ou « région Fc », tel qu'il est utilisé ici, on entend le fragment cristallisable généré lors de la digestion enzymatique des immunoglobulines par la papaïne. Le fragment Fc se réfère aux deux dernières régions constantes des immunoglobulines IgA, IgD, et IgG, aux trois dernières régions constantes des IgE et IgM. Pour IgA et IgM, Fc peut comprendre la chaîne de J. Pour les IgG, Fc comprend les domaines d'immunoglobuline Cgamma2 et Cgamma3 (Cγ2 et Cγ3 qui sont les domaines CH2 et CH3, respectivement). La chaîne lourde de la région Fc de l'IgG1 humaine est définie ici comme comprenant des résidus C226 à son extrémité carboxyle, où la numérotation des acides aminés de la région Fc est celle de l'index UE proposé par Kabat. Dans le cadre de l'IgG1 humaine, le domaine CH2 se réfère aux positions 237 à 340 et le domaine CH3 se réfère aux positions 341 à 447 selon l'index UE proposé par Kabat.

Le terme « anticorps » est utilisé ici dans le sens le plus large. Par « anticorps », on entend donc désigner tout polypeptide qui comprend au moins: (i) une région Fc et (ii) un domaine de liaison polypeptidique dérivé d'une région variable d'une immunoglobuline. Ledit domaine de liaison polypeptidique est capable de se lier spécifiquement à un antigène cible donnée ou d'un groupe d'antigènes cibles. Un domaine de liaison polypeptidique qui dérive d'une région variable d'une immunoglobuline comprend une ou plusieurs CDR (« *complementarity determining regions* »). Les anticorps comprennent, mais ne sont pas limités, aux immunoglobulines pleine longueur, aux anticorps monoclonaux, aux anticorps multi-spécifiques, aux protéines de fusion Fc comprenant au moins une région variable, aux anticorps synthétiques (parfois appelés « anticorps mimétiques »), aux anticorps chimériques, aux anticorps humanisés, aux anticorps humains entier, aux protéines de fusion-anticorps, aux conjugués d'anticorps et à leur fragments respectif.

Par « anticorps pleine longueur » ou « immunoglobuline » tel qu'il est utilisé ici, on entend la structure qui constitue la forme biologique naturelle d'un anticorps, comprenant les régions variables et constantes. Les « anticorps pleine longueur » couvrent les anticorps pleine longueur monoclonaux, les anticorps pleine longueur de type sauvage, les anticorps pleine longueur chimériques, des anticorps pleine longueur humanisés, la liste n'étant pas limitative.

Chez la plupart des mammifères, dont les humains et les souris, la structure des anticorps pleine longueur est généralement un tétramère. Ledit tétramère est composé de deux paires identiques de chaînes polypeptidiques, chaque paire ayant une chaîne « lourde » (ayant typiquement une masse moléculaire d'environ 50-70 kDa) et une chaîne « légère » (ayant typiquement une masse moléculaire d'environ 25 kDa). Chez certains mammifères, par exemple chez les chameaux et des lamas, les anticorps pleine longueur peuvent n'être constitués que de deux chaînes lourdes, chaque chaîne lourde comprenant un domaine variable attachée à la région Fc.

La partie amino-terminale de chaque chaîne comprend une région variable d'environ 100 à 110 acides aminés responsable de la reconnaissance de l'antigène. Dans la région variable, trois boucles sont rassemblées pour chacun des domaines V de la chaîne lourde et de la chaîne légère pour former un site de liaison a l'antigène. Chacune de ces boucles se rapporte à une CDR, dans laquelle la variation de la séquence d'acides amines est la plus importante.

La partie carboxy-terminale de chaque chaîne définit une région constante principalement responsable de la fonction effectrice. Kabat *et al.* a collecté de nombreuses séquences primaires de régions variables, de chaînes lourdes et de chaînes légères. Sur la base du degré de conservation des séquences, ils ont classé les séquences primaires individuels dans les CDR et ont fait une liste de celles-ci (voir Sequences of immunological Interest, 5e édition, NIH Publication, n ° 91 -3242, EA Kabat *et al.*).

Dans le cas des immunoglobulines humaines, les chaînes légères sont classées comme des chaînes légères kappa et lambda. Les chaînes lourdes sont classées comme mu, delta, gamma, alpha, ou epsilon, et définissent l'isotype de l'anticorps tel que IgM, IgD, IgG, IgA et IgE, respectivement. IgG a plusieurs sous-classes, y compris, mais sans s'y limiter, IgG1, IgG2, IgG3, et IgG4. IgM a plusieurs sous-classes, y compris, mais sans s'y limiter, IgM1 et IgM2. Ainsi, par « isotype » tel qu'il est utilisé ici, on entend l'une des sous-classes d'immunoglobulines définies par les caractéristiques chimiques et antigéniques et de leurs régions constantes. Les isotypes d'immunoglobulines humaines connues sont IgG1, IgG2, IgG3, IgG4, IgAI, IgA2, IgM1, IgM2, IgD, et IgE.

Par "IgG" tel qu'utilisé ici, on entend un polypeptide appartenance à la classe des anticorps qui est substantiellement codé par un gène gamma d'immunoglobuline reconnue. Chez l'homme, l'IgG comprend les sous-classes ou isotypes IgG1, IgG2, IgG3, et IgG4.

Les IgG pleine longueur sont des tétramères et se composent de deux paires identiques de deux chaînes d'immunoglobuline, chaque paire ayant une chaîne légère et une chaîne lourde, chaque chaîne légère comprenant les domaines VL et CL, et chaque chaîne lourde comprenant les domaines VH, CγI (également appelé CH1), Cγ2 (également appelé CH2) et Cy3 (appelée aussi CH3). Dans le cadre de l'IgG1 humaine, «CH1» renvoie aux positions 118 a 220, le domaine CH2 se réfère aux positions 237 a 340 et le domaine CH3 se réfère aux positions 34I a 447 selon l'index UE propose par Kabat. La chaîne lourde d'IgG comprend également une région charnière (« hinge domain ») qui se réfère aux positions 221 a 236 dans le cas de l'IgG1.

Par « fragments » de région Fc, on entend désigner, au sens de la présente invention, des polypeptides, identiques ou différents, qui comprennent un ou plusieurs polypeptides dérivés d'une région Fc de type sauvage, de préférence à partir du domaine CH2-CH3 se trouvant sous la région charnière d'une IgG de type sauvage. Les fragments de région Fc selon l'invention sont tels que, lorsqu'ils sont sous forme dimérique telle que celles décrites précédemment (i.e. au sein des protéines chimériques SAP-Fc dimériques, SAP-ScFc monomériques et SAP-ScFc dimériques) conservent leur capacité à recruter les cellules effectrices, en particulier les polynucléaires neutrophiles et les monocytes macrophages. A cet effet, Les fragments de région Fc selon l'invention ont une constante de dissociation pour FcRn inférieure à 1 microM selon le dosage SPR

Par « FcRn » ou « récepteur Fc néonatal », on entend désigner, au sens de la présente invention, une protéine qui se lie à la région Fc des anticorps IgG et est codé au moins en partie par un gène FCRN. Le FcRn peut être de n'importe quel organisme, y compris mais non limité à l'homme, souris, rats, lapins et singes. Au vu des indications thérapeutiques considérées dans la présente demande, le FcRn est celui de l'homme. Comme il est connu dans l'art, la protéine de FcRn fonctionnelle comprend deux polypeptides, souvent désignés comme la chaîne lourde et la chaîne légère. La chaîne légère est la bêta-2-microglobuline et la chaîne lourde est codée par le gène RRFC. Sauf indication contraire, FcRn ou la protéine FcRn se réfère au complexe des α-chaîne avec bêta-2-microglobuline. Chez l'homme, le gène codant pour FcRn est appelé FCGRT.

Comme indiqué ci-dessus, selon un mode de réalisation particulier, la région Fc ou le fragment de région Fc d'un anticorps humain d'une protéine chimérique selon l'invention peut présenter une affinité améliorée pour FcRn comparée à la région Fc d'un fragment d'une région Fc d'un anticorps humain parent.

Par « parent » tel qu'utilisé ici, on entend un polypeptide non modifié qui est ensuite modifié pour produire un variant. Ledit polypeptide parent peut être un polypeptide existant naturellement, un variant d'un polypeptide naturel, la version d'ingénierie ou un polypeptide synthétique. Le polypeptide parent peut se référer au polypeptide lui-même, ou à la séquence d'acides aminés qui le code. Dans le cadre de la présente invention, le polypeptide parent est un anticorps humain qui comprend une région Fc. Le polypeptide parent peut éventuellement comporter des modifications d'acides aminés pré-existantes dans sa région Fc (par exemple un mutant Fc) par rapport à des régions Fc de type sauvage.

Par « affinité améliorée pour FcRn » tel qu'il est utilisé ici, on entend l'augmentation de l'affinité de liaison, *in vivo* ou *in vitro,* d'un fragment de région Fc pour FcRn par rapport à un fragment de région Fc issu d'un anticorps humain parent.

L'affinité pour FcRn d'un fragment de région Fc d'un anticorps humain selon l'invention peut être évaluée par des méthodes bien connues de l'art antérieur. Par exemple, l'homme de l'art peut déterminer la constante de dissociation (Kd) en utilisant la résonance plasmonique de surface (SPR), comme décrit par exemple par Yeung YA, et al. (2009, J. Immunol, vol. 182 : 7663-7671). Dans le cas d'un fragment de région Fc d'un anticorps humain selon l'invention comprenant certaines modifications au niveau de sa séquence en acides aminées comparée à un fragment d'une région Fc d'un anticorps humain parent, à des fins d'améliorer son affinité pour FcRn, la présence d'une valeur de Kd 1,5 à 3 fois plus faible (affinité plus forte) que celle du fragment d'une région Fc d'un anticorps humain parent confirme cette amélioration.

Comme technique alternative, l'homme de l'art peut effectuer un test ELISA approprié, comme décrit par exemple par Deng et al. (2012, Landes Bioscience, vol. 4(1) : 101-109). Un test ELISA approprié permet de comparer la force de liaison entre un fragment de région Fc d'un anticorps humain selon l'invention comprenant certaines modifications et un fragment d'une région Fc d'un anticorps humain parent. Un fragment de région Fc d'un anticorps humain selon l'invention comprenant certaines modifications présente ainsi une affinité améliorée pour FcRn si son signal spécifique est au moins 1,2 fois plus fort, de préférence au moins 4 fois plus fort que celui mesuré avec un fragment d'une région Fc d'un anticorps humain parent.

A des fins d'amélioration de l'affinité pour FcRn, la région Fc de l'anticorps de la protéine chimérique selon l'invention peut comprendre certaines modifications au niveau de sa séquence en acides aminées.

Par « modifications au niveau de la séquence en acides aminés », on entend désigner, au sens de la présente invention, un changement dans la séquence d'acides aminés d'un polypeptide. Une modification au niveau de la séquence en acides aminés, ou changement d'acides aminés, comprend une substitution, une insertion et/ou une suppression d'un acide aminé compris dans une séquence de polypeptide.

Par « substitution d'un acide aminé » ou « substitution », on entend ici le remplacement d'un acide aminé en une position particulière dans une séquence de polypeptide parent par un autre acide aminé. Par exemple, la substitution N434S se réfère à un polypeptide dans lequel l'asparagine en position 434 est remplacée par la sérine.

Par « insertion d'un acide aminé » ou « insertion », on entend ici l'ajout d'un acide aminé à une position particulière dans une séquence de polypeptide parent. Par exemple, insert G> 235-236 désigne une insertion de glycine entre les positions 235 et 236.

Par « délétion d'un acide aminé » ou « suppression », tel qu'utilisé ici, on entend la suppression d'un acide aminé à une position particulière dans une séquence de polypeptide parent. Par exemple, E294del désigne la suppression de l'acide glutamique en position 294.

Par exemple, la forme suivante de modifications est préférentiellement utilisée : 434S, ou N434S, veut dire que l'acide aminé parent en position 434, soit l'asparagine, est remplacé par la sérine. Dans le cas d'une combinaison de substitutions, le format préféré est le suivant: 259I/315D/434Y ou V259I/N315D/N434Y. Cela signifie qu'il existe trois substitutions, une en position 259, une en position 315 et une en position 434, et que l'acide aminé en position 259 du polypeptide parent, c'est à dire la valine, est remplacé par l'isoleucine, que l'acide aminé en position 315 du polypeptide parent, soit l'asparagine, est remplacé par l'acide aspartique et que l'acide aminé en position 434 du polypeptide parent, soit l'asparagine, est remplacé par la tyrosine.

### Protéine chimérique selon l'invention

Comme indiqué précédemment, selon un premier de ses aspects, la présente invention concerne une protéine chimérique comprenant au moins un composant-P amyloïde humain et au moins un fragment d'une région Fc d'un anticorps humain, le composant-P amyloïde humain et le fragment d'une région Fc auquel il est rattaché étant liés l'un à l'autre par l'intermédiaire d'une région charnière.

Selon un mode de réalisation particulier, le composant-P amyloïde humain présent au niveau d'une protéine chimérique selon l'invention peut être figuré par une séquence en acides aminés ayant au moins 80 % d'identité avec la séquence SEQ ID NO : 1, de préférence au moins 90 % d'identité avec la séquence SEQ ID NO : 1.

Au sens de la présente invention, une séquence en acides aminés ayant au moins 80 % d'identité avec une séquence de référence possède une identité d'au moins 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % ou 99,5 % avec ladite séquence de référence.

Pour déterminer le pourcentage d'identité de deux séquences d'acides aminés, des méthodes d'alignement de séquences sont bien connues de l'homme du métier. Les séquences sont alignées à des fins de comparaison optimale. Par exemple, les écarts peuvent être introduits dans une ou les deux première et seconde séquences d'acides aminés ou de nucléotides pour un alignement optimal et les séquences non-homologues peuvent être écartées aux fins de comparaison. A titre d'exemple et sans être limitatif, le pourcentage d'identité de deux séquences d'acides aminés ou de nucléotides peut être réalisé avec CLUSTAL W (version 1.82, version 2), CLUSTAL OMEGA, BLAST ou MULTALIN.

Selon un autre mode de réalisation particulier, le composant-P amyloïde humain présent au niveau d'une protéine chimérique selon l'invention peut être figuré par une séquence en acides aminés codée par une séquence en acides nucléiques ayant au moins 80 % d'identité avec la séquence SEQ ID NO : 2, de préférence au moins 90 % d'identité avec la séquence SEQ ID NO : 2.

Ainsi, au sens de la présente invention, une séquence en acides nucléiques ayant au moins 80% d'identité avec une séquence de référence possède une identité d'au moins 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % ou 99,5 % avec ladite séquence de référence.

Selon un autre mode de réalisation particulier, la région Fc à l'origine de chacun des fragments de région Fc d'un anticorps humain de la protéine chimérique selon l'invention peut être une région Fc d'une immunoglobuline humaine, de préférence une région Fc d'une IgG, mieux une région Fc d'une IgG1 ou d'une IgG2, et plus particulièrement une région Fc d'une IgG1.

Selon un autre mode de réalisation particulier, le fragment de région Fc d'un anticorps humain d'une protéine chimérique selon l'invention peut être représenté par au moins une séquence en acides aminés ayant au moins 80 % d'identité avec la séquence SEQ ID NO : 3.

Selon un encore autre mode de réalisation particulier, le fragment de région Fc d'un anticorps humain d'une protéine chimérique selon l'invention peut être représenté par au moins une séquence en acides aminés codée par une séquence en acides nucléiques ayant au moins 80 % d'identité avec la séquence SEQ ID NO : 4.

Selon un mode de réalisation particulier, le fragment de région Fc d'un anticorps humain d'une protéine chimérique selon l'invention peut présenter une affinité améliorée pour FcRn, comparée à un fragment d'une région Fc d'un anticorps humain parent.

A ce titre, la région Fc de l'anticorps de la protéine chimérique selon l'invention peut comprendre au moins deux modifications au niveau de la séquence en acides aminées, à savoir :
(i) une modification au niveau de la séquence en acides aminées choisie parmi le groupe consistant en 378V, 378T, 434Y et 434S, et
(ii) au moins une modification au niveau de la séquence en acides aminées choisie parmi le groupe consistant en 226G, 230S, 230T, 230L, 241 L, 264E, 307P, 315D, 330V, 362R, 378V, 378T, 389T, 389K, 434Y et 434S,
étant entendu que la numérotation des acides aminés de la région Fc est celle de l'index UE proposé par Kabat et à la condition que la modification (i) ne se produise pas à la même position en acides aminés que la modification (ii).

Ce mode de réalisation est avantageux en ce que la combinaison de différentes mutations contribue à améliorer également la capacité de recrutement des cellules effectrices, en particulier les polynucléaires neutrophiles et les monocytes macrophages impliqués, et donc l'élimination des dépôts amyloïdes.

Comme désigné précédemment, un composant-P amyloïde humain est rattaché au fragment d'une région Fc d'un anticorps humain par l'intermédiaire d'une région charnière.

Cette construction a pour avantage d'assurer une plus grande accessibilité aussi bien au composant-P amyloïde qu'au fragment d'une région Fc d'un anticorps et confère également une plus grande flexibilité à une protéine chimérique selon l'invention. Ce mode de réalisation est avantageux en ce qu'il contribue à améliorer la capacité de la SAP à reconnaître les dépôts amyloïdes ainsi que le recrutement des cellules effectrices, en particulier les polynucléaires neutrophiles et les monocytes macrophages impliqués, et donc l'élimination des dépôts amyloïdes.

La nature de la région charnière peut être choisie selon les connaissances de l'homme du métier. De préférence, la région charnière est une séquence peptidique spécifique comprenant au moins un résidu cystéine ou une molécule non-peptidique telle que du Polyéthylène Glycol (PEG).

De préférence, une région charnière conforme à l'invention peut être choisie parmi les régions charnières de l'IgG1 humaine, l'IgG2 humaine, l'IgG3 humaine ou l'IgG4 humaine.

Avantageusement, une région charnière selon l'invention est celle de l'IgG1 humaine.

A cet égard, une région charnière selon l'invention peut comprendre au moins une séquence en acides aminés ayant au moins 60 % d'identité, de préférence au moins 80 % d'identité, avec une séquence choisie parmi les séquences SEQ ID NO : 13 et 15 à 18, de préférence avec la séquence SEQ ID NO : 13.

Une région charnière conforme à l'invention peut par exemple être représentée par la séquence SEQ ID NO: 19.

Selon un mode de réalisation particulier, une séquence peptidique non structurante peut en outre être présente entre la région charnière et le composant-P amyloïde.

En particulier, une telle séquence peptidique non structurante peut être représentée par la séquence SEQ ID NO : 5.

Cette séquence, de motif (G₄S)n, est donc plus ou moins longue en fonction de la flexibilité recherchée. A cet égard, « n » est de préférence compris entre 1 et 5.

Selon une première variante de réalisation, la protéine chimérique selon l'invention peut comprendre au moins deux motifs, chaque motif comprenant au moins un composant-P amyloïde humain et au moins un fragment d'une région Fc d'un anticorps humain liés l'un à l'autre par l'intermédiaire d'une région charnière, les deux motifs étant liés l'un à l'autre de manière covalente par au moins un pont disulfure.

Cette première variante de réalisation, caractérisant l'architecture SAP-Fc dimérique, est notamment illustrée en figure 1 ci-après.

Cette première variante de réalisation conduit à la formation d'une protéine chimérique dimérique. Cette architecture spécifique est avantageuse en ce qu'elle conduit à recruter efficacement les cellules effectrices, en particulier les polynucléaires neutrophiles et les monocytes macrophages impliqués. Il en résulte que cette protéine chimérique dimérique pourrait être particulièrement efficace dans l'élimination des dépôts amyloïdes.

Au vu de ce qui précède, une protéine chimérique selon cette première variante de réalisation peut donc comprendre au moins deux séquences en acides aminés, chaque séquence, identique ou différente, pouvant être représentée par une séquence en acides aminés ayant au moins 80 % d'identité, de préférence au moins 90 %, avec la séquence SEQ ID NO : 7, chaque séquences étant liées l'une à l'autre de manière covalente par au moins un pont disulfure.

Selon une seconde variante de réalisation, la protéine chimérique selon l'invention peut comprendre au moins un composant-P amyloïde humain et au moins deux fragments d'une région Fc d'un anticorps humain (i.e. premier et second fragments d'une région Fc d'un anticorps humain), avec une région charnière entre ledit composant-P amyloïde humain et le premier fragment d'une région Fc d'un anticorps humain auquel il est rattaché, les deux fragments d'une région Fc, identiques ou différents, étant liés l'un à l'autre de manière covalente par l'intermédiaire d'une liaison formée d'une chaine espaceur et d'une région charnière, identique ou différente à celle susmentionnée, et forment une seule chaîne polypeptidique constituant une région Fc dimérique fonctionnelle.

Cette seconde variante de réalisation, caractérisant l'architecture SAP-ScFc monomérique, est notamment illustrée en figure 2 ci-après.

A cet égard, les fragments d'une région Fc peuvent être de préférence liés l'un à l'autre par l'intermédiaire d'une chaine espaceur représentée par au moins une séquence en acides aminés représentée par la séquence SEQ ID NO : 5.

Cette séquence SEQ ID NO : 5, de motif (GGGGS)n (ou G₄S)n, est donc plus ou moins longue en fonction de la flexibilité recherchée. A cet égard, « n » est de préférence compris entre 1 et 5, et de préférence égal à 3.

En d'autres termes, une protéine SAP-ScFc monomérique comprend, de la partie N-terminale à la partie C-terminale, (1) un composé-P amyloïde humain, (2) une région charnière, (3) un premier fragment de région Fc d'un anticorps humain, (4) une chaine espaceur, (5) une région charnière, identique ou différente à celle considérée en (2) ci-dessus, et (6) un second fragment de région Fc d'un anticorps humain, identique ou différent à celui considéré en (3) ci-dessus.

Selon cette seconde variante de réalisation, l'architecture, de la partie N-terminale à la partie C-terminale, comprenant (i) une région charnière, (ii) un premier fragment de région Fc d'un anticorps humain, (iii) une chaine espaceur, (iv) une région charnière, identique ou différente à celle considérée en (i) ci-dessus, et (v) un second fragment de région Fc d'un anticorps humain, identique ou différent à celui considéré en (ii) ci-dessus peut être figurée par au moins une séquence en acides aminés ayant au moins 80 % d'identité avec la séquence SEQ ID NO:11.

A cet égard, l'architecture, de la partie N-terminale à la partie C-terminale, comprenant (i) une région charnière, (ii) un premier fragment de région Fc d'un anticorps humain, (iii) une chaine espaceur, (iv) une région charnière, identique ou différente à celle considérée en (i) ci-dessus, et (v) un second fragment de région Fc d'un anticorps humain, identique ou différent à celui considéré en (ii) ci-dessus peut être figurée par une séquence en acides aminés codée par une séquence en acide nucléique ayant au moins 80 % d'identité avec la séquence SEQ ID NO : 12.

Au vu de ce qui précède, une protéine chimérique selon cette seconde variante de réalisation peut comprendre au moins une séquence en acides aminés ayant au moins 80 % d'identité avec la séquence SEQ ID NO : 9.

Une protéine chimérique selon cette seconde variante de réalisation peut également comprendre au moins une séquence en acides aminés codée par une séquence en acides nucléiques ayant au moins 80 % d'identité avec la séquence SEQ ID NO : 10.

Selon une troisième variante de réalisation, la protéine chimérique selon l'invention peut résulter de l'appariement naturel de deux protéine SAP-ScFc monomériques, liées l'une avec l'autre par l'intermédiaire d'au moins deux ponts disulfure ou d'au moins deux liaisons, une liaison étant formée d'au moins deux groupements poly(éthylène glycol) (PEG).

Cette troisième variante de réalisation, caractérisant l'architecture SAP-ScFc dimérique, est notamment illustrée en figure 3 ci-après.

Eu égard aux protéines chimériques SAP-Fc dimérique et SAP-ScFc dimérique, la/les liaison(s) covalente(s) entre chaque motif SAP-Fc monomérique ou SAP-ScFc monomérique se situe(nt) de préférence au niveau des résidus Cystéine des régions charnières.

### Acides nucléiques vecteurs et cellules hôtes selon l'invention

Selon un autre de ses aspects, la présente invention concerne un acide nucléique comprenant au moins une séquence de polynucléotides codant au moins une protéine chimérique selon l'invention.

La formation des structures SAP-Fc dimérique et SAP-ScFc dimérique résultant d'un phénomène d'appariement naturel, la séquence de polynucléotides code de préférence au moins la protéine chimérique SAP-Fc monomérique ou SAP-ScFc monomérique.

Selon encore un autre de ses aspects, la présente invention concerne un vecteur, de préférence un vecteur d'expression dans lequel est inséré un acide nucléique tel que défini ci-dessus.

De préférence, un vecteur particulièrement adapté est le vecteur pCpG (Cayla, Invivogen) qui permet de très bons résultats sur production de chaînes légères d'Ig en SP2/0 et CHO.

Selon un autre de ses aspects, la présente invention concerne une cellule hôte transfectée avec un vecteur tel que défini précédemment, en particulier une cellule de bactéries, levures, champignons ou une cellule de mammifères, ou encore une cellule d'animaux transgéniques.

De préférence, à titre de cellule hôte particulièrement adaptée, peut être cité les cellules eucaryotes, plus particulièrement les cellules choisies parmi le groupe consistant en les cellules YB2/0, en particulier la lignée déposée à la « American Type Culture Collection » sous le n° ATCC n°CRL-1662), SP2/0, YE2/0, 1R983F, Namalwa, PER.C6, les lignées cellulaires CHO, en particulier CHO-K-1, CHO-Lecl0, CHO-Lecl, CHO-Lecl3, CHO Pro-5, CHO dhfr-, Wil-2, Jurkat, Vero, Molt-4, COS-7, 293-HEK, BHK, KGH6, NSO, SP2/0-Ag 14, P3X63Ag8.653, C127, JC, LA7, ZR-45-30, hTERT, NM2C5, UACC-812, DG44 et DXB11, DHFR, HELA, CVI, COS, R1610, BALBC/3T3, HAK, BF1-1c1BPT, RAJI, HEK, EB66, ou BHK

### Procédé de fabrication d'une protéine chimérique selon l'invention

Selon encore un autre de ses aspects, la présente invention concerne un procédé de fabrication d'une protéine chimérique telle que définie précédemment, ledit procédé comprenant au moins les étapes consistant à :
a) transfecter une cellule hôte avec un vecteur tel que défini ci-dessus;
b) cultiver ladite cellule hôte dans des conditions telles que la protéine chimérique est produite; et
c) collecter ladite protéine chimérique produite par ladite cellule hôte à l'issue de l'étape b).

### 1) Etape de transfection

Au sens de la présente invention, on appelle « transfection » le processus d'introduction d'ADN exogène (par exemple un vecteur d'expression tel que défini ci-dessus) dans des cellules eucaryotes.

Il existe différentes méthodes pour introduire un ADN exogène dans une cellule, ces méthodes relevant des techniques classiques connues de l'homme du métier.

A titre d'exemple de méthodes de transfection, on peut citer la transfection à l'aide du phosphate de calcium, l'incorporation de l'ADN à transfecter dans des liposomes (pour les cellules eucaryotes, une transfection basée sur les lipides et les polycations est préférentiellement utilisée, du fait de la plus grande sensibilité des cellules), la mise en oeuvre d'agents polycationiques hautement branchés, appelés dendrimères, comme le polyéthylènimine (PEI), pour lier l'ADN et le transporter dans la cellule (du Tris est souvent inclus dans la solution de transfection pour améliorer la perméabilité membranaire), l'électroporation, le choc thermique (heat shock), les propriétés particulières de réactifs comme le GeneCellin, et le gene gun.

De préférence, une méthode de transfection appropriée dans le cadre de la présente invention est l'électroporation, comme décrit par exemple par Potter et al. (2003, Curr Protoc. Mol. Biol., Chapter Unit-9.3., 1-12).

Au vu de ce qui précède, le vecteur mis en oeuvre comprend de préférence au moins un acide nucléique comprenant au moins une séquence de polynucléotides codant au moins une protéine chimérique SAP-Fc monomérique ou SAP-ScFc monomérique.

### 2) Etape de culture de la cellule transfectée

Une fois l'étape de transfection réalisée, les cellules ainsi obtenues sont ensuite placées dans des conditions environnementales, et notamment un milieu de culture, apte à assurer leur survie et donc leur multiplication, l'amplification du vecteur, l'induction de l'expression génique et la production de la protéine chimérique selon l'invention.

Cette étape relève également des connaissances classiques de l'homme du métier.

### 3) Etape de récupération de la protéine chimérique

Lors de l'expression génique, la présence d'un peptide signal d'adressage en amont de la séquence génique codant la protéine chimérique permet la sécrétion de ladite protéine chimérique dans le milieu de culture.

La protéine chimérique selon l'invention secrétée ne contient donc plus la séquence relative au peptide signal.

Les critères de choix du peptide signal relèvent des connaissances générales de l'homme du métier et, notamment, peuvent être basées sur le D-score et l'Y-max définis par le logiciel Signal IP.

Un peptide signal selon l'invention peut être représenté par une séquence en acides aminés ayant au moins 80 % d'identité avec au moins une des séquences décrites dans WO 2011/114063.

Plus particulièrement un peptide signal selon l'invention peut encore être représenté par une séquence en acides aminés ayant au moins 80 % d'identité avec une séquence choisie parmi les séquences SEQ ID NO : 20 et 22 à 27.

De préférence, le peptide signal est représenté par la séquence SEQ ID NO:25.

Après récupération du surnageant cellulaire, une étape de dosage par la technique ELISA de la protéine chimérique peut être effectuée. Ensuite, une étape de séparation/visualisation des protéines est requise. Cette étape peut être effectuée au moyen d'une électrophorèse SDS-PAGE ou Western-Blot.

Toutes ces techniques relèvent des connaissances classiques de l'homme du métier.

### 4) Etapes additionnelles

Dans le cas où on cherche à obtenir une protéine chimérique SAP-Fc dimérique ou SAP-ScFc dimérique telles que définies précédemment, il est à noter que ces architectures spécifiques sont réalisées naturellement dans la cellule hôte.

De préférence, un procédé selon l'invention peut en outre comprendre une étape d) de purification.

Une telle étape de purification est réalisée par les techniques classiques connues de l'homme du métier. Peut à ce titre être cité la purification sur colonne d'affinité couplée à l'antigène, sur Protéine A ou sur Protéine G, ou encore une colonne d'affinité couplée à des aptamères anti-SAP et/ou anti-fragment d'une région Fc d'un anticorps humain considéré.

Dans l'hypothèse où un procédé de fabrication d'une protéine chimérique selon l'invention considère la mise en oeuvre d'une étape c), l'étape d) peut intervenir antérieurement ou postérieurement à cette étape c).

Dans le cadre d'une purification sur colonne d'affinité couplée à l'antigène, on peut imaginer un système comprenant un anticorps anti-SAP de manière à isoler les protéines chimériques selon l'invention.

La méthode de purification considérée peut impliquer également la mise en oeuvre d'une étape d'élution de manière à récupérer les complexes formés, et donc les protéines chimériques selon l'invention. Cette étape d'élution relève également des techniques classiques connues de l'homme du métier.

La protéine chimique selon l'invention peut encore être produite à partir d'animaux transgéniques. Selon un mode de réalisation préféré, ladite protéine est produite dans le lait de mammifères transgéniques non humain, modifiés génétiquement pour produire cette protéine chimique. Un animal transgénique selon l'invention peut être choisi parmi le lapin, la chèvre, la vache, le chameau, le hamster, la souris, le rat, le cheval, la truie, le dromadaire, la brebis, le lama, la liste n'étant pas limitative. De préférence, il s'agit du lait d'une lapine ou d'une chèvre transgénique. La sécrétion de ladite protéine par les glandes mammaires, permettant sa sécrétion dans le lait du mammifère transgénique, implique le contrôle de son expression de manière tissu-dépendante. De telles méthodes de contrôle sont bien connues de l'homme du métier. Le contrôle de l'expression est effectué grâce à des séquences permettant l'expression de la protéine vers un tissu particulier de l'animal. Il s'agit notamment des séquences promotrices WAP, bêta-caséine, bêta-lactoglobuline et des séquences peptides signal. Le procédé d'extraction des protéines d'intérêt à partir du lait d'animaux transgénique est notamment décrit dans EP 0 264 166.

### Utilisation d'une protéine chimérique selon l'invention

Comme indiqué précédemment, la présente invention se rapporte au domaine de l'obtention de protéine chimérique spécifique à usage thérapeutique, notamment pour traiter l'amylose, en particulier l'amylose de type AL.

En conséquence, selon encore un autre de ses aspects, la présente invention concerne une composition pharmaceutique comprenant une protéine chimérique selon l'invention et un excipient pharmaceutiquement acceptable.

Comme indiqué ci-dessus, une protéine chimérique selon l'invention peut revêtir des architectures différentes. A ce titre, une composition pharmaceutique selon l'invention peut comprendre un mélange de protéines chimériques selon l'invention ayant des architectures différentes. Plus particulièrement, parmi l'ensemble des protéines chimériques selon l'invention pouvant être comprises dans une composition pharmaceutique selon l'invention, on peut citer :
- la protéine chimérique comprenant deux motifs, chaque motif comprenant un composant-P amyloïde humain et un fragment d'une région Fc d'un anticorps humain liés entre eux par une région charnière, les deux motifs étant liés l'un à l'autre de manière covalente par au moins un pont disulfure (Caractérise l'architecture SAP-Fc dimérique) ;
- la protéine chimérique comprenant un composant-P amyloïde humain et deux fragments d'une région Fc d'un anticorps humain (i.e. premier et second fragments d'une région Fc d'un anticorps humain), le composant-P amyloïde humain étant rattaché au premier fragment d'une région Fc par une région charnière et les deux fragments d'une région Fc, identiques ou différents, étant liés l'un à l'autre de manière covalente par l'intermédiaire d'une liaison formée d'une chaine espaceur et d'une région charnière, et forment une seule chaîne polypeptidique constituant une région Fc dimérique fonctionnelle (Caractérise l'architecture SAP-ScFc monomérique) ; et
- la protéine chimérique comprenant au moins deux motifs SAP-ScFc monomérique liés l'un à l'autre de manière covalente par au moins deux ponts disulfure (caractérise l'architecture SAP-ScFc dimérique).

De préférence, une composition pharmaceutique selon l'invention est majoritairement formée de protéines chimériques comprenant deux motifs comprenant chacun un composant-P amyloïde humain et un fragment d'une région Fc d'un anticorps humain, les deux motifs étant liés l'un à l'autre de manière covalente par une chaine espaceur (Caractérise l'architecture SAP-Fc dimérique), avec, pour chaque motif, le composant-P amyloïde humain et le fragment d'une région Fc d'un anticorps humain étant liés l'un à l'autre par l'intermédiaire d'une région charnière.

Dans certains modes de réalisation, une composition pharmaceutique selon l'invention peut être sous forme liquide.

Dans certains modes de réalisation, une composition pharmaceutique selon l'invention peut être sous forme solide, forme qui comprend une forme lyophilisée.

Une composition pharmaceutique selon l'invention peut être formulée selon les méthodes standard telles que celles décrites dans Remington: The Science and Practice of Pharmacy (Lippincott Williams & Wilkins; Twenty first Edition, 2005).

A titre d'excipient pharmaceutiquement acceptable, on peut citer, en particulier, ceux décrits Handbook of Pharmaceuticals Excipients, American Pharmaceutical Association (Pharmaceutical Press; 6th revised edition, 2009).

Afin de traiter un patient en ayant besoin, c'est-à-dire un patient étant atteint d'au moins une des indications considérées dans la présente demande, une dose thérapeutiquement efficace de la composition selon l'invention peut être administrée.

Par "dose thérapeutiquement efficace", on entend une dose qui produit des effets attendus et pour lesquels une composition selon l'invention est administrée. La dose exacte dépendra du but du traitement, et sera vérifiable par l'homme de l'art en utilisant des techniques connues. Les doses pouvant être considérées peuvent varier de 0,001 à 100 mg de protéines chimériques selon l'invention, par kg de poids corporel (mg / kg) ou plus, par exemple 0, 1, 1,0, 10 ou 50 mg / kg de poids corporel, avec 1 à 10 mg / kg étant préféré. Le dosage et la fréquence d'administration peuvent être adaptés en fonction de la réponse du patient traité, ainsi que la fréquence d'injection.

De préférence, la protéine chimérique peut être administrée à une dose variant de 0,1 à 1000 µg/kg de poids corporel.

Comme il est connu dans l'art, des ajustements compte-tenu de la dégradation des protéines, de l'administration systémique versus localisée, ainsi que de l'âge, du poids, de la santé générale, du sexe, l'alimentation, du temps d'administration, des interactions médicamenteuses et de la gravité de l'état du patient peuvent être nécessaires, et sont peuvent être aisément déterminés par l'homme de l'art au moyen d'expérience de routine.

L'administration d'une composition pharmaceutique selon l'invention peut être réalisée de plusieurs façons, y compris, mais sans s'y limiter, par voie locale et cutanéo-muqueuse, par voie entérale ou par voie parentérale.

Par « voie locale et cutanéo-muqueuse », on entend notamment désigner la voie topique, intra-auriculaire, intravaginal, intrautérin, en inhalation, transdermique, oculaire, ou intravésicale.

Par « voie entérale », on entend notamment désigner la voie, intrarectal, sublingual, buccal, nasal, intrastomacal ou intrajéjunal.

Par « voie parentérale », on entend notamment désigner la voie intradermique, sous-cutané, intramusculaire, intracardiaque, intravasculaire, intraveineuse, intra-oculaire, intra-artériel, épidural, intrarachidien, extracorporel, intrathécal, intrapéritionéal, intrapleural, intraluminal, intravitréal, intracaverneux, intraventriculaire, intraosseux, palatin, intra-articlaire, intracellulaire, pulmonaire ou intrafoetal.

De préférence, l'administration d'une composition pharmaceutique selon l'invention peut être faite par voie intraveineuse (IV) ou sous-cutanée (SC) selon un schéma d'administration issu de protocole standard utilisés dans le traitement par immunothérapie passive. D'autres modes d'administrations comme une injection locale ou d'une administration par aérosols peuvent également être utilisés.

La composition de l'invention peut être administrée de façon concomitante avec d'autres agents thérapeutiques, notamment des traitements spécifiques pour pallier la ou les insuffisances de fonctionnement du ou des organes touchés par les dépôts amyloïdes.

Selon encore un autre de ses aspects, la présente invention concerne une protéine chimérique telle que définie ci-dessus, pour son utilisation comme médicament.

Selon encore un autre de ses aspects, la présente invention concerne une protéine chimérique telle que définie ci-dessus, pour son utilisation pour éliminer les dépôts d'amylose au niveau des organes.

Selon encore un autre de ses aspects, la présente invention concerne une protéine chimérique telle que définie ci-dessus qui, en s'intégrant au dépôt amyloïde humain, va induire un recrutement des cellules effectrices comme les monocytes-macrophages et des polynucléaires et induire de ce fait l'élimination par phagocytose des dépôts amyloïdes

Selon encore un autre de ses aspects, la présente invention concerne une protéine chimérique telle que définie ci-dessus, pour son utilisation pour traiter l'amylose, en particulier l'amylose de type AL.

Selon encore un autre de ses aspects, il est décrit un procédé pour traiter l'amylose, en particulier l'amylose de type AL, comprenant au moins une étape d'administration à un individu en ayant besoin d'une quantité efficace d'au moins un premier agent actif représenté par une protéine chimérique telle que définie précédemment.

Selon un mode de réalisation particulier, ledit procédé peut en outre comprendre une étape d'administration, antérieurement, concomitamment et/ou postérieurement à l'étape d'administration du premier agent actif, d'un second agent actif distinct dudit premier agent actif, de préférence le second agent actif étant choisi parmi le groupe comprenant le 4-[bis(chloroéthyl)amino]phénylalanine (melphalan) ; le 9-fluoro-11β,17,21-trihydroxy-16a-méthylprégna-1,4-diène-3,20-dione (dexaméthasone) ; la prednisone ; le diméthylsulfoxyde (DMSO) ; le N-[(7S)-5,6,7,9-tétrahydro-1,2,3,10-tétraméthoxy-9-oxobenzo[a]heptalen-7-yl)acétamide] (colchicine) ; le 7S,9S)-7-[(2R,4S,5S,6S)-4-amino-5-hydroxy-6-methyloxan-2-yl]oxy-6,9,11-trihydroxy-9-(2-hydroxyacetyl)-4-methoxy-8,10-dihydro-7H-tetracene-5,12-dione (Doxorubicin) ; des actifs aptes à réagir avec le composant-P amyloïde, en particulier de type bis-d-proline, et plus particulièrement le composé (R)-1-[6-[(R)-2-carboxy-pyrrolidin-1-yl]-6-oxo-hexa-noyl]pyrrolidine-2 carboxylic acid) ;les inhibiteurs du protéasome, par exemple le bortezomib, le carfilzomib, le marizomib, l'ixazomib, le delanzomib, l'ONX-912 ou le Revlimid ; et un de leur mélange.

Une protéine chimérique selon l'invention peut encore être identifiée au moyen de toute technique connue de l'homme de l'art. A ce titre peut notamment être cité la technique via le couplage avec les fluorophores et les microbulles telle que décrite dansNeri et al (1997) Nat Biotech, ou encore les techniques décrites dans Brack et al (2005) EJNM, Santimaria (2003) Clinical cancer res, Borsi et al. (2002) Int J cancer, Berndorff (2006) J Nucl Med et Joseph et al (2004) Pham Res.

### Procédé d'identification des dépôts amyloïdes

Selon encore un autre de ses aspects, une protéine chimérique selon l'invention est en outre avantageuse en ce que elle peut être mise à profit pour son utilisation à titre d'outil de caractérisation *in vitro* ou *ex vivo* de la présence d'un dépôt d'amylose.

Selon cet aspect de l'invention, ladite protéine chimérique peut être marquée à l'aide d'une molécule détectable. Par exemple, selon une première variante, ladite protéine chimérique peut être radiomarquée, de préférence le composant-P amyloïde humain est figuré par ¹²³I-SAP. Selon une seconde variante, la protéine chimérique peut être identifiée au moyen d'un anticorps anti-Fc radiomarqué, par exemple ledit anticorps anti-Fc est couplé à la péroxydase.

De préférence, pour des raisons évidentes, ledit anticorps anti-Fc est d'origine humaine.

A titre d'anti-Fc couplé à la péroxydase, il est possible d'utiliser tout anticorps disponible sur le marché, montrant une bonne spécificité et une interaction avec la partie Fc de la protéine de fusion Fc-SAP. A titre d'exemple, on peut citer celui commercialisé sous la dénomination A0170 par la société Sigma-Aldrich.

Une protéine chimérique selon l'invention peut encore être identifiée au moyen de toute technique connue de l'homme de l'art. A ce titre peut notamment être cité la technique via le couplage avec des fluorophores et des microbulles telle que décrite dansNeri et al. (1997) Nat Biotech, ou encore les techniques décrites dans Brack et al. (2005) EJNM, Santimaria (2003) Clinical cancer res, Borsi et al. (2002) Int J cancer, Berndorff (2006) J Nucl Med et Joseph et al. (2004) Pham Res.

**Tableau 2 : séquences**

| **SEQ ID NO°** | **Type** | **Description** |
|---|---|---|
| 1 | peptide | composant-P amyloïde humain |
| 2 | acide nucléique | composant-P amyloïde humain |
| 3 | peptide | fragment d'une région Fc d'un anticorps humain |
| 4 | acide nucléique | fragment d'une région Fc d'un anticorps humain |
| 5 | peptide | Séquence peptidique non structurante entre SAP et une région charnière, ou chaine espaceur entre deux fragments d'une région Fc d'un anticorps humain au niveau de la SAP-ScFc |
| 6 | acide nucléique | Séquence peptidique non structurante entre SAP et une région charnière, ou chaine espaceur entre deux fragments d'une région Fc d'un anticorps humain au niveau de la SAP-ScFc |
| 7 | peptide | SAP-Fc monomérique sécrétée |
| 8 | acide nucléique | SAP-Fc monomérique sécrétée |
| 9 | peptide | SAP-ScFc monomérique sécrétée |
| 10 | acide nucléique | SAP-ScFc monomérique sécrétée |
| 11 | peptide | Région charnière + les deux fragments d'une région Fc d'un anticorps humain liés entre eux par une chaine espaceur et une région charnière de SAP-ScFc |
| 12 | acide nucléique | Région charnière + les deux fragments d'une région Fc d'un anticorps humain liés entre eux par une chaine espaceur et une région charnière de SAP-ScFc |
| 13 | peptide | Région charnière de l'IgG1 humaine |
| 14 | acide nucléique | Région charnière de l'IgG1 humaine |
| 15 | peptide | Région charnière de l'IgG2 humaine |
| 16 | peptide | Région charnière de l'IgG3 humaine |
| 17 | peptide | Région charnière de l'IgG3 humaine |
| 18 | peptide | Région charnière de l'IgG4 humaine |
| 19 | peptide | Région charnière de l'IgG1 humaine raccourcie |
| 20 | peptide | Peptide signal naturel de la SAP |
| 21 | acide nucléique | Peptide signal naturel de la SAP |
| 22 | peptide | Peptide signal TIMP humain MMP1 |
| 23 | peptide | Peptide signal de l'insuline humaine |
| 24 | peptide | Peptide signal de l'EPO humaine |
| 25 | peptide | Peptide signal MB7 |
| 26 | peptide | Peptide signal AMHRII |
| 27 | peptide | Peptide signal XXII49 |
| 28 | acide nucléique | SAP de l'exemple 1 |
| 29 | acide nucléique | Fragment de région Fc d'un anticorps humain de l'exemple 1 |
| 30 | acide nucléique | SAP-Fc monomérique de l'exemple 1 |
| 31 | peptide | SAP-Fc monomérique de l'exemple 1 |
| 32 | acide nucléique | Premier fragment de région Fc d'un anticorps humain de l'exemple 2 |
| 33 | acide nucléique | Second fragment de région Fc d'un anticorps humain de l'exemple 2 |
| 34 | acide nucléique | Chaine espaceur entre les premier et second fragments de région Fc d'un anticorps humain de l'exemple 2 |
| 35 | acide nucléique | SAP-ScFc monomérique de l'exemple 2 |
| 36 | peptide | SAP-ScFc monomérique de l'exemple 2 |
| 37 | acide nucléique | Première amorce pour l'amplification de la SAP en exemple 1 |
| 38 | acide nucléique | Seconde amorce pour l'amplification de la SAP en exemple 1 |
| 39 | acide nucléique | Première amorce pour l'amplification du fragment de région Fc d'un anticorps humain en exemple 1 |
| 40 | acide nucléique | Seconde amorce pour l'amplification du fragment de région Fc d'un anticorps humain en exemple 1 |
| 41 | acide nucléique | Première amorce pour l'amplification du premier fragment de région Fc d'un anticorps humain en exemple 2 |
| 42 | acide nucléique | Seconde amorce pour l'amplification du premier fragment de région Fc d'un anticorps humain en exemple 2 |
| 43 | acide nucléique | Première amorce pour l'amplification du second fragment de région Fc d'un anticorps humain en exemple 2 |
| 44 | acide nucléique | Seconde amorce pour l'amplification du second fragment de région Fc d'un anticorps humain en exemple 2 |
| 45 | acide nucléique | Première amorce pour l'amplification de la chaine espaceur en exemple 2 |
| 46 | acide nucléique | Seconde amorce pour l'amplification de la chaine espaceur en exemple 2 |

Les exemples et figures qui suivent sont présentés à titre illustratif et non limitatif de l'invention.

### EXEMPLES

Préliminairement, il est à noter que la séquence correspondant au composant-P amyloïde humain utilisée ci-après est identifiée, notamment, au moyen de son numéro de séquence tel que considéré ci-après, auquel est ajouté en partie N-terminal une séquence spécifique correspondant au peptide signal naturel dudit composant. En l'espèce, le peptide signal considéré est celui représenté par la séquence en acide aminés SEQ ID NO : 20, voire la séquence en acides nucléiques SEQ ID NO : 21.

### Exemple 1 : Construction du vecteur SAP-Fc monomérique

### A) Amplification du fragment d'ADNc codant la SAP humaine :

Pour la construction du vecteur SAP-Fc monomérique, le composant-P amyloïde humain (SAP) a été amplifié à partir d'ADNc humain avec la Taq polymérase Phusion. Le codon STOP n'a pas été amplifié. Une séquence Kozak a été ajoutée en amont de l'ATG de départ de la séquence. La séquence a été encadrée de 2 sites de restrictions SalI, ajoutés dans les amorces utilisées pour l'amplification. Qui plus est, dans la séquence considérée, seuls les 2 exons composant la SAP sont présents, l'intron n'étant pas dans cette construction.

La séquence d'ADNc de la SAP humaine utilisée, correspondante à la séquence SEQ ID NO : 28 ci-après, est donc la suivante :

Les amorces de PCR utilisées pour amplifier la SAP sont :
hSAPfor**Sal** : acttg**GTCGAC**accatgaacaagccgctgctttg (SEQ ID NO : 37)
hSAPrevfus**Sal** : actag**GTCGAC**ccacaccaagggtttga (SEQ ID NO : 38)

L'amplification PCR est réalisée par les techniques classiques connues de l'homme du métier. Peut à ce titre être cité les protocoles standards décrits dans Current Protocols in Molecular Biology (Frederick M. AUSUBEL, 2000, Wiley and son Inc, Library ofCongress, USA).

### B) Amplification du fragment d'ADNc codant le fragment de région Fc d'un anticorps humain

L'acide nucléique codant le fragment de région Fc d'un anticorps humain (avec son codon STOP), incluant en outre une séquence nucléique correspondante à une région charnière en région N-terminale dudit acide nucléique, a été amplifié à partir d'ADNc totaux extraits de moelle osseuse humaine. Le domaine Fc considéré est celui du d'une IgG1 humaine (IGHG1*03). L'amplification de la séquence du domaine Fc a été réalisée avec la Taq polymérase Phusion. Le fragment Fc a été encadré de 2 sites de restrictions, XhoI et BspEI, ajoutés dans les amorces lors de l'amplification.

La séquence d'ADNc codant le fragment considéré de région Fc d'un anticorps humain, correspondante à la séquence SEQ ID NO : 29 ci-après, est donc la suivante :

Les amorces de PCR utilisées pour amplifier le fragment considéré de région Fc d'un anticorps humain sont :
hG1hingeforfus**Xho** : **CTCGAG**cccaaatcttgtgacaa (SEQ ID NO : 39)
hG1CH3rev **BspE I : TCCGGA**gcactcatttacccggagac (SEQ ID NO : 40)

### C) Construction du vecteur SAP-Fc monomérique

Les fragments SAP et Fc sont dans un premier temps clonés dans le vecteur commercial pGEM®-T Easy (Promega) (voir figure 4 ci-après) de manière à les séquencer (pGEMT-SAP et pGEMT-Fc).

Le fragment codant pour la SAP humaine a été coupé et isolé à partir du vecteur pGEMT-SAP par l'enzyme de restriction SalI (un site de chaque coté du fragment) puis inséré dans le vecteur pGEMT-Fc en amont du fragment codant pour le domaine Fc préalablement coupé par l'enzyme de restriction XhoI (Compatible avec SalI).

Une fois la SAP-Fc monomérique établie, une coupure par les enzymes de restrictions SalI et BspEI a permis d'insérer le fragment SAP-Fc monomérique dans le vecteur d'expression pCpGfree (invivogen), (voir figure 5 ci-après) digéré par le même couple d'enzymes, ledit le vecteur d'expression ayant été préalablement modifié pour contenir les sites de restriction SalI et BspEI dans le site multiple de clonage originel ainsi qu'une cassette fonctionnelle de résistance à la néomycine.

Puis, un séquençage complet a été effectué par une méthode dérivée de la méthode de Sanger (Sanger F, Nicklen S, Coulson AR, PNAS, 1977). L'ADN est préparé de façon à avoir des fragments de toutes les tailles dont la dernière base et une des 4 ddNTP, marquée par un fluorochrome, qui bloque la réaction de polymérisation. Les fragments migrent selon leurs tailles dans le capillaire, et les différents fluorochromes sont excités par un laser qui permet d'établir la séquence. La réaction de séquençage nécessite, en plus de l'ADN à séquencer, l'amorce sens ou anti-sens, le tampon et le BigDye® (Applied Biosystems) qui contient l'ADN polymérase, les dNTP et les ddNTP. Le programme de PCR est le suivant: 96°C pendant 1 minute, puis 25 cycles composés d'une étape de déshybridation à 96°C pendant 10 secondes, puis une étape d'hybridation des amorces à 50°C pendant 5 secondes suivie d'une étape d'élongation à 60°C durant 4 min.

Afin d'éliminer tous les constituants pouvant interférer lors de la migration dans les capillaires, les produits de séquences sont purifiés sur gel Sephadex™ G50 (GE Healthcare). La poudre Sephadex est distribuée dans les puits de la plaque de filtration (Millipore) puis laissée 3 heures avec 300 µl d'eau. L'excès d'eau est éliminé par centrifugation 3 min à 910 g,

Ensuite, les produits de séquences sont déposés dans les puits sur le gel, centrifugés 3 min à 910 g et récupérés dans une plaque de séquençage. Le séquençage est réalisé sur un séquenceur automatique ABI 3130 (Applied Biosystems).

La séquence en acides nucléiques de SAP-Fc monomérique, correspondante à la séquence SEQ ID NO : 30, est la suivante :

La séquence protéique correspondante de la SAP-Fc monomérique, correspondante à la séquence SEQ ID NO : 31, est la suivante :

La séquence protéique correspondante de la SAP-Fc monomérique sécrétée est la séquence SEQ ID NO:7 suivante :

### Exemple 2 : Construction du vecteur SAP-ScFc monomérique

### A) Amplification du fragment d'ADN codant la SAP humaine :

Voir exemple 1.A) ci-dessus.

### B) Amplification du premier fragment d'ADNc codant le premier fragment de région Fc d'un anticorps humain

L'acide nucléique codant le premier fragment de région Fc d'un anticorps humain a été amplifié à partir d'un vecteur du laboratoire contenant l'ADNc d'une IgG1 humaine (IGHG1*03). L'amplification de la séquence du domaine Fc a été réalisée avec la Taq polymérase Phusion. Le fragment Fc a été encadré de 2 sites de restrictions, XhoI et HindIII, ajoutés dans les amorces lors de l'amplification. Le codon STOP n'a pas été amplifié.

La séquence d'ADNc codant le premier fragment de région Fc d'un anticorps humain, correspondante à la séquence SEQ ID NO : 32 ci-après, est la séquence suivante:

Les amorces de PCR utilisées pour amplifier la séquence du fragment de région Fc d'un anticorps humain :
hGlhingeforfus**Xho** : atact**CTCGAG**cccaaatcttgtgacaa (SEQ ID NO : 41)
hG1CH3revfus**Hind :** atctg**AAGCTT**acccggagacagggaga (SEQ ID NO : 42)

### C) Amplification du second fragment d'ADN codant le second fragment de région Fc d'un anticorps humain:

L'acide nucléique codant le second fragment de région Fc d'un anticorps humain, incluant en outre une séquence nucléique correspondante à une région charnière en région N-terminale dudit acide nucléique, a été encadré de 2 sites de restrictions, BglII et BspEI, ajoutés dans les amorces lors de l'amplification. Le codon STOP a été amplifié pour ce second fragment de région Fc d'un anticorps humain.

La séquence d'ADNc codant le second fragment de région Fc d'un anticorps humain, correspondante à la séquence SEQ ID NO : 33 ci-après, est donc la suivante :

Les amorces de PCR utilisées pour amplifier la séquence du second fragment de région Fc d'un anticorps humain sont:
hGlhingeforfus**Bgl** : aagta**AGATCT**gagcccaaatcttgtgacaa (SEQ ID NO : 43)
hG1CH3rev**BspE :** atctg**TCCGGA**gcactcatttacccggagac (SEQ ID NO : 44)

### D) Amplification de la chaine espaceur entre les deux fragments de région Fc d'un anticorps humain :

La séquence de la chaine espaceur a été amplifiée à partir d'un vecteur du laboratoire contenant la séquence d'un ScFv. Cette séquence code un peptide de 15 acides aminés (Gly4Ser répétés 3 fois). L'amplification de la chaine espaceur a été réalisée grâce à la Taq polymérase Phusion. La séquence de la chaine espaceur se termine par un site de restriction BamHI déjà présent dans le vecteur du laboratoire. Le site de restriction HindIII est ajouté par l'amorce Forward lors de l'amplification.

La séquence codant de la chaine espaceur, correspondante à la séquence SEQ ID NO : 34 ci-après, est donc la suivante:

Les amorces de PCR utilisées pour amplifier la séquence du linker sont :
Linkfor**Hind** III: **AAGCTT**ggtggaggcggttcagg (SEQ ID NO:45)
Linkrev**BHI** : **GGATCC**gccaccgccagagcca (SEQ ID NO : 46)

### E) Construction du vecteur SAP-ScFc monomérique

Les différents fragments composant la SAP-ScFc monomérique ont dans un premier temps été clonés indépendamment dans le vecteur commercial pGEMT-easy (Promega) (voir figure 4 ci-après) de manière à les séquencer. Il s'agit des vecteurs respectivement nommés : pGEMT-SAP, pGEMT-premier Fc, pGEMT-linker et pGEMT-second Fc.

Les vecteurs d'expression considérés ont été préalablement modifiés pour contenir les sites de restriction Xho/Hind (pour le premier fragment Fc) et Bg1/BspE1 (pour le second fragment Fc) dans le site multiple de clonage originel ainsi qu'une cassette fonctionnelle de résistance à la néomycine.

Le fragment codant pour la SAP humaine présent dans le vecteur pGEMT-SAP a été coupé et isolé par l'enzyme de restriction SalI (un site de chaque coté du fragment) puis inséré dans le vecteur pGEMT-premierFc contenant la séquence du premier fragment de région Fc d'un anticorps humain, en amont de ce dernier préalablement coupé par l'enzyme de restriction XhoI.

Le linker coupé HindIII/NotI (le site NotI étant présent en 3' du site multiple de clonage du vecteur pGEM-T Easy) a ensuite été ajouté en aval du premier fragment de région Fc d'un anticorps humain dans le vecteur pGEMT-SAP-premierFc suite à une digestion par les enzymes de restrictions HindIII et NotI.

Le second fragment de région Fc d'un anticorps humain a ensuite été coupé et isolé du vecteur pGEMT-second Fc par les enzymes BglII/NotI puis inséré dans le vecteur pGEMT-SAP-premierFc-linker digéré par les enzymes de restriction BamHI/NotI.

Une fois la SAP-ScFc établie, une coupure par les enzymes de restrictions SalI et BspeI a permis d'insérer le fragment SAP-ScFc dans le vecteur d'expression pCpG (invivogen) préalablement modifié pour contenir les sites de restriction SalI et BspEI dans le site multiple de clonage originel ainsi qu'une cassette fonctionnelle de résistance à la néomycine (voir figure 4 ci-après) digéré par le même couple d'enzymes.

Puis un séquençage complet a été effectué.

La séquence d'acides nucléiques de la SAP-ScFc monomérique, correspondante à la séquence SEQ ID NO : 35 ci-après, est la suivante :

La séquence protéique correspondante de la SAP-ScFc monomérique, correspondante à la séquence SEQ ID NO : 36 ci-après, est la suivante :

La séquence protéique correspondante de la SAP-ScFc monomérique secrétée, correspondante à la séquence SEQ ID NO : 9 ci-après, est la suivante :

### Exemple 3 : production de protéines chimériques SAP-Fc/ScFc selon l'invention

### A) Transformation de cellules SP2/0, CHO, YB2/0 par électroporation

La transfection est réalisé par le protocole d'électroporation Nucleofector™ (Lonza). Les cellules en phase exponentielle sont centrifugées 5 min à 300g et resuspendues dans du PBS. Après comptage, la quantité nécessaire est culotée par centrifugation (300g, 5 min) et reprise dans 100µl de tampon Nucleofector™ Solution V. 2,5x10⁶ cellules SP2/0 ou 1.10⁶ cellules CHO sont transfectées par 2,5 µg de vecteur linéarisé. Après électroporation, les cellules sont remises dans du milieu complet et réparties dans des plaque 96 puits à 1000 cellules par puits pour les SP2/0 et 100 cellules par puits pour les CHO. Les conditions optimales de clonalité ont été établies en fonction de l'efficacité du programme d'électroporation choisi et de la nature de la lignée utilisée. La sélection des clones ayant intégrés de façon stable le vecteur d'expression est réalisé par ajout de néomycine (G418) à 1 mg/ml final 24h après la transfection.

A noter que la dimérisation des protéines SAP-Fc et SAP-ScFc monomériques s'effectue naturellement dans les cellules transformées, comme mentionné précédemment.

Après 15-20 jours de culture, les surnageants des clones résistants au G418 sont prélevés puis testés par ELISA pour évaluer le niveau de production de la protéine recombinante (détection du Fc humain). Les clones sélectionnés sont amplifiés en plaques 24 puits puis flasques T25 pour d'autres tests ELISA et une partie est congelées en l'azote liquide dans du milieu de congélation contenant 10 % de DMSO et 20 % de SVF.

Protocole de dosage de la SAP-Fc et SAP-scFc par ELISA :
Dans un premier temps, des plaques MaxiSorp™ (NUNC) sont « coatées » par un anticorps primaire anti-IgG humaines (1µg/ml, Beckman Coulter) toute la nuit à 4°C. Après 3 lavages au PBS/tween20 0,05 %, 50 µl des échantillons dilués dans du milieu complet sont incubés 2 h à 37°C. En même temps, est incubée une gamme étalon d'IgG humains dilué dans du milieu complet. Après 3 lavages, un anticorps anti-IgG (Beckman Coulter) couplé à la phosphatase alcaline (1µg/ml) est ajouté et incubé 1 h 30 à 37°C. Après une dernière série de lavage, un volume de 100 µl du substrat de la phosphatase alcaline est ajouté puis la réaction est arrêtée après quelques minutes par du NAOH 3M. La lecture de la plaque est réalisée par spectrométrie à 405 nm.

### B) Purification des protéines chimériques SAP-Fc/SAP-ScFc obtenues

### B-1) Purification sur protéine A

Les protéines chimériques SAP-Fc/SAP-ScFc obtenues sont ensuite purifiées par chromatographie d'affinité sur un système ÄKTA_{FPLC} (GE Healthcare) en utilisant des colonnes d'affinité Protéine A (Pierce) du fait de la forte affinité de la protéine A pour les segments Fc et utilisable du fait de la présence de fragments de région Fc dans les protéines chimériques selon l'invention. Le détecteur UV situé en aval de la colonne permet de suivre la progression; quand l'absorption de la phase non retenue descend à une valeur seuil, la protéine est éluée par de la Glycine 0,1 M à pH 2,6. Les différentes fractions sont collectées en fonction des pics du chromatogramme. Les éluats obtenus sont alors neutralisés par du Tris à pH 8,8.

Les protéines purifiées sont concentrées grâce aux filtres Amicon® Ultra4 30k (Millipore), qui permettent d'éliminer les molécules d'une taille inférieur à 30 kDa. Les solutions sont placées dans les filtres et centrifugées à 4000g le temps nécessaire pour obtenir le volume et la concentration souhaités.

### Résultats :

Les résultats de cette étape de purification sur protéine A sont illustrés en figure 6 ci-après.

B-2) Détection des protéines chimériques SAP-Fc/SAP-ScFc par Western Blot Gel dénaturant

Les protéines purifiées (ou surnageants de culture) sont mélangées à un volume de bleu de dépôt contenant du β-mercaptoéthanol (Bio Rad) et mises à bouillir 5 min. Elles sont ensuite migrées par électrophorèse sur gel de polyacrylamide (SDS-PAGE) composé d'une phase de concentration à 7,5 % et d'une phase de séparation à 10 %. Les protéines sont alors transférées sur une membrane de PVDF et saturées au lait 5 %. Ensuite, elles sont incubées 1h à température ambiante en présence de l'anticorps de souris anti-SAP (Abcam) ou anti-IgG couplé HRP (Beckman Coulter) (1 µg/ml). L'anti-SAP est révélé par un anticorps secondaire de chèvre anti-IgG de souris couplé HRP (Santa Cruz) (0,2 µg/ml). La réaction de chimiluminescence est déclenchée par l'ajout du substrat ECL (Pierce) et révélée sur film autoradiographique (Kodak).

### Gel semi-natif

Les protéines sont mélangées à un volume de bleu de charge sans β-mercaptoéthanol puis mises à migrer dans un gel SDS-PAGE 12 %. La suite du protocole est la même que pour le gel dénaturant.

### Résultats :

Les résultats sont illustrés en figure 7.

La bande à 75 kDa correspond à l'architecture SAP-ScFc monomérique. La bande à 100 kDa correspond à l'architecture SAP-Fc dimérique.

### Exemple 4 : Reconnaissance de la substance amyloïde

### A) Test de fixation in vitro des protéines chimériques SAP-Fc/SAP-ScFc sur des coupes de coeur amyloïde humain

Les protéines chimériques SAP-Fc/ScFc selon l'invention sont mises en contact avec des coupes de coeur amyloïde humain, en présence de calcium et de DNAse I.

Les organes amyloïdes sont fournis par le centre de référence des amyloses AL et autres maladies de dépôts d'Ig monoclonales. Les fragments d'organes sont fixées sur un gel Cryomatrix™ (Thermo Scientific), puis coupées à -20°C sur des lames SuperFrost® Plus (Thermo Scientific) à une épaisseur de 8 µm. Les lames sont conservées à -80°C.

Les lames sont ensuite séchées à température ambiante puis fixées dans de l'acétone à -20°C pendant 15 min. Un traitement à la DNAse I (4U/µl) est effectué à température ambiante durant 15 min suivie de deux lavages rapides et de deux lavages de 5 min au PBS. Le traitement DNAse permet d'éviter un accrochage de la SAP qui a une affinité naturelle pour l'ADN. Les anticorps primaires, qui serviront de témoin positif, sont alors ajoutés à 5 µg/ml en PBS sur les lames et incubés 1 h en chambre humide à 37°C. Les protéines SAP-Fc et SAP-scFc sont diluées dans du PBS (5µg/ml) contenant du CaCl₂ à 2,2 mM. Après lavage, les anticorps secondaires (rabbit anti-human IgG Fc, Dako ou mouse anti-human SAP, Abcam) couplés aux fluorochromes et dilués en PBS à 1 µg/ml sont ajoutés et incubés 30 min à température ambiante.

### Résultats :

Les résultats obtenus confirment le fait que les protéines chimériques SAP-Fc/SAP-ScFc selon l'invention conservent leur capacité de fixation sur les dépôts d'amylose et ne se fixent pas sur des tissus sains.

### B) Test de fixation in vivo des protéines chimériques SAP-Fc/SAP-ScFc sur un modèle murin d'amylose AA

### B-1) Préparation d'un modèle murin d'amylose AA

A J0, on injecte par intraveineuse à une souris (Balb/c ou C57/B16) une solution comprenant I.V 200 µL d'AEF (i.e. un broyat de rate d'amylose SaM ApoAII) et par voie sous-cutanée 200 µL de nitrate d'argent 1 %.

A J7 et J14, on injecte par voie sous-cutanée une solution comprenant 100 µL de nitrate d'argent 1 %.

A J21, on procède au sacrifice de l'animal et on prélève la rate et le foie.

A partir de ces organes, on réalise des coupes sur lesquelles on réalise une reconnaissance des dépôts d'amylose grâce à une coloration au rouge congo.

### Résultats :

Les résultats obtenus caractérisent sans aucun doute la présence de dépôts d'amylose sur les coupes d'organes issues du modèle murin décrit précédemment et absent sur des souris saines.

### B-2) Test de fixation des protéines chimériques SAP-Fc/SAP-ScFc selon l'invention

On considère le même modèle murin d'amylose AA que celui décrit au point B-1) ci-dessus.

A J21, à la place de l'étape de sacrifice, on injecte par intraveineuse 2 mg d'une solution comprenant les protéines chimériques SAP-Fc ou SAP-ScFc selon l'invention ou 1 mg de SAP humaine. En parallèle, on injecte à d'autres souris du PBS à titre de contrôle.

A J24, on procède au sacrifice de l'animal et on prélève la rate et le foie.

Les lames sont préparées comme précédemment décrit. Les dépôts amyloïdes sont révélés par une coloration au rouge Congo.

La présence de SAP-Fc ou SAP-ScFc associées aux dépôts amyloïdes est observée grâce à un marquage avec l'anti-human IgG Fc (Dako) couplé à la FITC. Lorsque cela est possible, le double marquage avec le rouge Congo est effectué pour s'assuré de la co-localisation des dépôts.

Enfin, un marquage anti-human SAP a été aussi réalisé sur certaines lames des différents organes/animaux.

### Résultats :

Les résultats obtenus confirment :
- la présence de dépôts amyloïdes caractéristiques dans la rate et le foie des animaux où l'amylose AA a été déclenchée,
- la capacité des protéines chimériques SAP-Fc/SAP-ScFc selon l'invention à se fixer sur les dépôts amyloïdes (avec colocalisation sur le rouge Congo),
- la spécificité du marquage anti-human Fc IgG (absence de marquage sur les souris amyloïdes mais injectées avec de la SAP humaine sans Fc),
- la spécificité de la fixation de la SAP-Fc/SAP-ScFc sur les dépôts amyloïdes (absence totale de marquage sur les lames de souris saines).

### Exemple 5 : Evaluation de l'efficacité thérapeutique in vivo des protéines chimériques SAP-Fc/SAP-ScFc sur l'amylose AA chez des souris Balb/c

### 1) Matériel et méthodes

L'induction de l'amylose AA chez des souris Balb/c est réalisée selon le protocole défini en figure 9 ci-après.

Comme il ressort de cette figure 9, différents composés sont testés. A chaque fois, on injecte le composé testé chez les souris Balb/c une semaine après la dernière injection d'AgNO₄.

Les composés testés sont une protéine chimérique SAP-ScFc selon l'invention (3 mg), une protéine chimérique SAP-Fc selon l'invention (3 mg), une Immunoglobuline IgG1 irrelevante anti-FVIII (« IgIR », contrôle isotypique) (6 mg) ou encore du tampon PBS (contrôle négatif).

A partir des organes des souris décrites ci-dessus, notamment de la rate, on réalise des coupes sur lesquelles on réalise une reconnaissance des dépôts d'amylose grâce à une coloration au rouge congo, de manière à quantifier ces derniers.

### 2) Résultats :

Comme il ressort de la figure 10, les dépôts d'amylose sont significativement plus faibles chez les souris Balb/c traitées avec SAP-Fc ou SAP-ScFc.

Les résultats obtenus confirment donc la capacité des protéines chimériques SAP-Fc/SAP-ScFc selon l'invention :
- à se fixer sur les dépôts amyloïdes, et
- à éliminer les dépôts d'amylose AA.

Les résultats obtenus attestent également que c'est bien le fragment SAP présent dans les protéines chimériques selon l'invention qui permet leur fixation effective sur les dépôts amyloïdes. Cela ressort de l'incapacité du composé IgIR à fixer sur les dépôts amyloïdes. En effet, ce composé IgIR comprend certes au moins un fragment de région Fc d'un anticorps humain similaire à celui présent chez les protéines chimériques selon l'invention, mais il est en revanche dénué du fragment SAP.

### Exemple 6 : autre évaluation de l'efficacité thérapeutique in vivo des protéines chimériques SAP-Fc/SAP-ScFc sur l'amylose AA chez des souris VH-LMP2A

### 1) Matériel et méthodes

L'induction de l'amylose AA chez des souris VH-LMP2A est réalisée selon le protocole défini en figure 9 ci-avant.

Les composés testés sont une protéine chimérique selon l'invention (SAP-ScFc), une protéine SAP en tant que telle (i.e. dénuée du fragment de région Fc d'un anticorps humain), ou encore du tampon PBS.

Les souris VH-LMP2A ont la particularité d'être dépourvues de la capacité de production d'anticorps. De fait, une diminution des dépôts d'amylose consécutivement à l'injection d'un composé spécifique traduira la capacité propre dudit composé spécifique à recruter efficacement les cellules effectrices, en particulier les polynucléaires neutrophiles et les monocytes macrophages, impliquées dans l'élimination des dépôts amyloïdes.

A partir des organes des souris décrites ci-dessus, notamment de la rate, on réalise des coupes sur lesquelles on réalise une reconnaissance des dépôts d'amylose grâce à une coloration au rouge congo, de manière à quantifier ces derniers.

### 2) Résultats :

Comme il ressort de la figure 11, les dépôts d'amylose sont significativement réduits chez les souris VH-LMP2A traitées avec les protéines chimériques SAP-ScFc selon l'invention.

En revanche, comme il ressort de la figure 11, aucune diminution des dépôts d'amylose n'est observée dans le cas de la protéine SAP en tant que telle, c'est-à-dire dénuée du fragment de région Fc d'un anticorps humain.

Les résultats obtenus confirment donc la capacité des protéines chimériques SAP-Fc/SAP-ScFc selon l'invention :
- à se fixer sur les dépôts amyloïdes, et
- à éliminer les dépôts d'amylose AA, et donc à recruter efficacement les cellules effectrices, en particulier les polynucléaires neutrophiles et les monocytes macrophages, impliquées dans l'élimination de ces dépôts amyloïdes.

Les résultats obtenus attestent également que c'est bien le fragment de région Fc d'un anticorps humain présent dans les protéines chimériques selon l'invention qui permet ce recrutement efficace des cellules effectrices.

### SEQUENCES :

SEQ ID NO : 1 : ***(Protéine** = **SAP humaine)***
SEQ ID NO : 2: ***(ADN = SAP humaine)***
SEQ ID NO : 3 : ***(Protéine = fragment de région Fc d'un anticorps humain)***
SEQ ID NO : 4 : ***(ADN = fragment de région Fc d'un anticorps humain)***
SEQ ID NO : 5 : ***(Protéine = Séquence peptidique non structurante entre SAP et une région charnière, ou chaine espaceur entre deux fragments d'une région Fc au niveau de la SAP-ScFc)***
   (GGGGS)n, avec n compris entre 1 et 5
SEQ ID NO : 6 : ***(ADN = Séquence peptidique non structurante entre SAP et une région charnière, ou chaine espaceur entre deux fragments d'une région Fc au niveau de la Caractérise SAP-ScFc)***
   (CTTGGTGGAGGCGGTTCAGGCGGAGGTGGCTCTGGCGGTGGCG)m, avec m compris entre 1 et 5
SEQ ID NO : 7 : ***(Protéine = SAP-Fc monomérique sécrétée)***
SEQ ID NO : 8 : ***(ADN = SAP-Fc monomérique sécrétée)***
SEQ ID NO : 9 : ***(Protéine =SAP-ScFc monomérique sécrétée)***
SEQ ID NO : 10 : ***(ADN = SAP-ScFc monomérique sécrétée)***
SEQ ID NO : 11 : ***(Protéine = région charnière* + *deux fragments d'une région Fc d'un anticorps humain liés entre eux par une liaison (chaine espaceur + région charnière) de SAP-ScFc)***
SEQ ID NO : 12 : ***(ADN = région charnière* + *deux fragments d'une région Fc d'un anticorps humain liés entre eux par une liaison (chaine espaceur* + *région charnière) de SAP-ScFc)***
SEQ ID NO : 13 : ***(Protéine = Région charnière de l'IgG1 humaine)***
   EPKSCDKTHTCPPCP
SEQ ID NO : 14 : ***(ADN = Région charnière de l'IgG1 humaine)***
   CTCACACATGCCCACCGTGCCCA
SEQ ID NO : 15 ; ***(Protéine = Région charnière de l'IgG2 humaine)***
   ERKCCVECPPCP
SEQ ID NO : 16 ; ***(Protéine = Région charnière de l'IgG3 humaine)***
   ELKTPLGDTTHTCPRCP
SEQ ID NO : 17 ; ***(Protéine = Région charnière de l'IgG3 humaine)***
   EPKSCDTPPPCPRCP
SEQ ID NO : 18 ; ***(Protéine = Région charnière de l'IgG4 humaine)***
   ESKYGPPCPSCP
SEQ ID NO : 19 ; ***(Protéine = Région charnière de l'IgG1 (SEQ ID NO 13) raccourcie)***
   DKTHTCPPCP
SEQ ID NO 20 ***(Protéine = peptide signal naturel de la SAP)***
   MNKPLLWISVLTSLLEAFA
SEQ ID NO 21 ***(ADN = peptide signal naturel de la SAP)***
SEQ ID NO 22 ***(Protéine = peptide signal TIMP humain MMP1 (=P01033 d'uniprot))***
   MAPFEPLASGILLLLWLIAPSRA
SEQ ID NO 23 ***(Protéine = peptide signal insuline humaine (=P01308 d'uniprot))***
   MALWNIRLLPLLALLALWGPDPAAA
SEQ ID NO 24 ***(Protéine=peptide signal EPO humaine (=P01588 d'uniprot))***
   MGVHECPAWLWLLLSLLSLPLGLPVLG
SEQ ID NO 25 ***(Protéine = peptide signal MB7 issue de*** WO 2011/114063***)***
   MRWSWIFLLLLSITSANA
SEQ ID NO 26 ***(Protéine = peptide signal AMHRII issue de*** WO 2011/114063***)***
   MRWSWIFLFLLSITASVHC
SEQ ID NO 27 ***(Protéine = peptide signal XXII49 issue de*** WO 2011/114063***)***
   MAWVWTLLFLMAAAQSAQA
SEQ ID NO 28 ***(ADN = SAP de l'exemple 1)***
SEQ ID NO 29 ***(ADN = fragment de région Fc d'un anticorps humain de l'exemple 1)***
SEQ ID NO 30 ***(ADN = SAP-Fc monomérique de l'exemple 1)***
SEQ ID NO 31 ***(Protéine = SAP-Fc monomérique de l'exemple 1)***
SEQ ID NO 32 ***(ADN = premier fragment de région Fc d'un anticorps humain de l'exemple 2)***
SEQ ID NO 33 ***(ADN = second fragment de région Fc d'un anticorps humain de l'exemple 2)***
SEQ ID NO 34 ***(ADN = chaine espaceur entre les premier et second fragments de région Fc d'un anticorps humain de l'exemple 2)***
SEQ ID NO 35 ***(ADN = SAP-ScFc monomérique de l'exemple 2)***
SEQ ID NO 36 ***(Protéine = SAP-ScFc monomérique de l'exemple 2)***
SEQ ID NO 37 ***(acide nucléique = Première amorce pour l'amplification de la SAP en exemple 1)***
   ACTTGGTCGACACCATGAACAAGCCGCTGCTTTG
SEQ ID NO 38 ***(acide nucléique = Seconde amorce pour l'amplification de la SAP en exemple 1)***
   ACTAGGTCGACCCACACCAAGGGTTTGA
SEQ ID NO 39 ***(acide nucléique = Première amorce pour l'amplification du fragment de région Fc d'un anticorps humain en exemple 1)***
   CTCGAGCCCAAATCTTGTGACAA
SEQ ID NO 40 ***(acide nucléique = Seconde amorce pour l'amplification du fragment de région Fc d'un anticorps humain en exemple 1)***
   TCCGGAGCACTCATTTACCCGGAGAC
SEQ ID NO 41 ***(acide nucléique = Première amorce pour l'amplification du premier fragment de région Fc d'un anticorps humain en exemple 2)***
   ATACTCTCGAGCCCAAATCTTGTGACAA
SEQ ID NO 42 ***(acide nucléique = Seconde amorce pour l'amplification du premier fragment de région Fc d'un anticorps humain en exemple 2)***
   ATCTGAAGCTTACCCGGAGACAGGGAGA
SEQ ID NO 43 ***(acide nucléique = Première amorce pour l'amplification du second fragment de région Fc d'un anticorps humain en exemple 2)***
   AAGTAAGATCTGAGCCCAAATCTTGTGACAA
SEQ ID NO 44 ***(acide nucléique = Seconde amorce pour l'amplification du second fragment de région Fc d'un anticorps humain en exemple 2)***
   ATCTGTCCGGAGCACTCATTTACCCGGAGAC
SEQ ID NO 45 ***(acide nucléique = Première amorce pour l'amplification de la chaine espaceur en exemple 2)***
   AAGCTTGGTGGAGGCGGTTCAGG
SEQ ID NO 46 ***(acide nucléique = Seconde amorce pour l'amplification de la chaine espaceur en exemple 2)***
   GGATCCGCCACCGCCAGAGCCA

## Revendications

1. Protéine chimérique comprenant au moins un composant-P amyloïde humain et au moins un fragment d'une région Fc d'un anticorps humain, le composant-P amyloïde humain et le fragment d'une région Fc auquel il est rattaché étant liés l'un à l'autre par l'intermédiaire d'une région charnière.

2. Protéine chimérique selon la revendication 1, **caractérisée en ce que** le composant-P amyloïde humain comprend une séquence en acides aminés ayant au moins 80 % d'identité avec la séquence SEQ ID NO : 1.

3. Protéine chimérique selon la revendication 1 ou 2, **caractérisée en ce que** la région Fc de l'anticorps est la région Fc d'une immunoglobuline humaine, de préférence la région Fc d'une IgG, mieux la région Fc d'une IgG1ou d'une IgG2, et plus particulièrement la région Fc d'une IgG1.

4. Protéine chimérique selon l'une quelconque des revendications précédentes, **caractérisée en ce que** le fragment d'une région Fc d'un anticorps humain comprend au moins une séquence en acides aminés ayant au moins 80 % d'identité avec la séquence SEQ ID NO : 3.

5. Protéine chimérique selon l'une quelconque des revendications précédentes, **caractérisée en ce que** ladite région Fc comprend au moins deux modifications au niveau de la séquence en acides aminées, à savoir :
(i) une modification au niveau de la séquence en acides aminées choisie parmi le groupe consistant en 378V, 378T, 434Y et 434S, et
(ii) au moins une modification au niveau de la séquence en acides aminées choisie parmi le groupe consistant en 226G, 230S, 230T, 230L, 241 L, 264E, 307P, 315D, 330V, 362R, 378V, 378T, 389T, 389K, 434Y et 434S,
étant entendu que la numérotation des acides aminés de la région Fc est celle de l'index UE proposé par Kabat et à la condition que la modification (i) ne se produise pas à la même position en acides aminés que la modification (ii).

6. Protéine chimérique selon l'une quelconque des revendications précédentes, **caractérisée en ce que** la région charnière comprend au moins une séquence en acides aminés ayant au moins 60 % d'identité, de préférence au moins 80 % d'identité, avec une séquence choisie parmi les séquences SEQ ID NO : 13 et 15 à 18, de préférence est représentée par la séquence SEQ ID NO : 13.

7. Protéine chimérique selon l'une quelconque des revendications précédentes, **caractérisée en ce que** ladite protéine chimérique comprend en outre, entre la région charnière et le composant-P amyloïde, au moins une séquence peptidique non structurante, de préférence représentée par une séquence en acides aminés SEQ ID NO : 5.

8. Protéine chimérique selon l'une quelconque des revendications précédentes, **caractérisée en ce que** ladite protéine chimérique comprend au moins deux motifs, chaque motif comprenant au moins un composant-P amyloïde humain et au moins un fragment d'une région Fc d'un anticorps humain liés l'un à l'autre par l'intermédiaire d'une région charnière, les deux motifs étant liés l'un à l'autre par au moins un pont disulfure et les fragments d'une région Fc d'un anticorps humain étant identiques ou différents.

9. Protéine chimérique selon l'une quelconque des revendications 1 à 7, **caractérisée en ce que** ladite protéine chimérique comprend au moins un composant-P amyloïde humain et au moins deux fragments d'une région Fc d'un anticorps humain, identiques ou différents, ledit composant-P amyloïde humain et le premier fragment étant liés l'un à l'autre par l'intermédiaire d'une région charnière, les premier et second fragments d'une région Fc étant liés l'un à l'autre de manière covalente par l'intermédiaire d'une liaison formée d'une chaine espaceur et d'une région charnière, identique ou différente à celle précédemment citée, et forment une seule chaîne polypeptidique constituant une région Fc dimérique fonctionnelle.

10. Protéine chimérique selon la revendication précédente, **caractérisée en ce que** la chaine espaceur est représentée par au moins une séquence en acides aminés SEQ ID NO : 5.

11. Acide nucléique comprenant au moins une séquence de polynucléotides codant au moins une protéine chimérique telle que définie en revendications 1 à 7 et 9 à 10.

12. Vecteur dans lequel est inséré un acide nucléique tel que défini en revendication 11.

13. Cellule hôte transfectée avec un vecteur tel que défini en revendication 12.

14. Procédé de fabrication d'une protéine chimérique telle que définie en revendications 1 à 10, ledit procédé comprenant au moins les étapes consistant à :
a) transfecter une cellule hôte avec un vecteur tel que défini en revendication 12;
b) cultiver ladite cellule hôte dans des conditions telles que la protéine chimérique est produite; et
c) collecter ladite protéine chimérique produite par ladite cellule hôte à l'issue de l'étape b).

15. Composition pharmaceutique comprenant une protéine chimérique telle que définie en revendications 1 à 10, et un excipient pharmaceutiquement acceptable.

16. Protéine chimérique telle que définie en revendications 1 à 10, pour son utilisation comme médicament.

17. Protéine chimérique telle que définie en revendications 1 à 10, pour son utilisation pour éliminer les dépôts d'amylose au niveau des organes.

18. Protéine chimérique telle que définie en revendications 1 à 10, pour son utilisation pour traiter les amyloses, en particulier l'amylose de type AL.

19. Utilisation d'une protéine chimérique telle que définie en revendications 1 à 7, pour la fabrication d'une protéine chimérique telle que définie en revendication 8.

20. Utilisation d'au moins une protéine chimérique telle que définie en revendications 1 à 10, à titre d'outil de caractérisation *in vitro* ou *ex vivo* de la présence d'un dépôt d'amylose, ladite protéine chimérique étant radiomarquée, de préférence le composant-P amyloïde humain est figuré par ¹²³I-SAP, et/ou identifiée au moyen d'un anticorps anti-Fc radiomarqué, par exemple ledit anticorps anti-Fc est couplé à la péroxydase.

## Patentansprüche

1. Chimäres Protein, welches wenigstens eine menschliche Amyloid-P-Komponente und wenigstens ein Fragment einer Fc-Region eines menschlichen Antikörpers umfasst, wobei die menschliche Amyloid-P-Komponente und das Fragment einer Fc-Region, mit dem sie verbunden ist, miteinander mittels einer Gelenkregion verbunden sind.

2. Chimäres Protein nach Anspruch 1, **dadurch gekennzeichnet, dass** die menschliche Amyloid-P-Komponente eine Aminosäuresequenz umfasst, die wenigstens 80% Identität mit der Sequenz SEQ ID NO: 1 aufweist.

3. Chimäres Protein nach Anspruch 1 oder 2, **dadurch gekennzeichnet, dass** die Fc-Region des Antikörpers die Fc-Region eines menschlichen Immunglobulins ist, bevorzugt die Fc-Region eines IgG, besser die Fc-Region eines IgG1 oder eines IgG2 und insbesondere die Fc-Region eines IgG1.

4. Chimäres Protein nach einem der vorhergehenden Ansprüche, **dadurch gekennzeichnet, dass** das Fragment einer Fc-Region eines menschlichen Antikörpers wenigstens eine Aminosäuresequenz umfasst, die wenigstens 80% Identität mit der Sequenz SEQ ID NO: 3 aufweist.

5. Chimäres Protein nach einem der vorhergehenden Ansprüche, **dadurch gekennzeichnet, dass** die Fc-Region wenigstens zwei Modifikationen bezüglich der Aminosäuresequenz aufweist, nämlich:
(i) eine Modifikation bezüglich der Aminosäuresequenz, gewählt aus der Gruppe, umfassend 378V, 378T, 434Y und 434S, und
(ii) wenigstens eine Modifikation bezüglich der Aminosäuresequenz, gewählt aus der Gruppe, umfassend 226G, 230S, 230T, 230L, 241L, 264E, 307P, 315D, 330V, 362R, 378V, 378T, 389T, 389K, 434Y und 434S,
vorausgestzt, dass die Nummerierung der Aminosäuren der Fc-Region dem EU-Index von Kabat entspricht und unter der Bedingung, dass die Modifikation (i) nicht an derselben Aminosäureposition erfolgt wie die Modifikation (ii).

6. Chimäres Protein nach einem der vorhergehenden Ansprüche, **dadurch gekennzeichnet, dass** die Gelenkregion wenigstens eine Aminosäuresequenz umfasst, die wenigstens 60% Identität, bevorzugt wenigstens 80% Identität, mit einer Sequenz aufweist, die gewählt ist aus den Sequenzen SEQ ID NO: 13 und 15 bis 18, und bevorzugt durch die Sequenz SEQ ID NO: 13 repräsentiert ist.

7. Chimäres Protein nach einem der vorhergehenden Ansprüche, **dadurch gekennzeichnet, dass** das chimäre Protein ferner zwischen der Gelenkregion und der Amyloid-P-Komponente wenigstens eine nicht strukturierende Peptidsequenz umfasst, die bevorzugt durch eine Aminosäuresequenz SEQ ID NO: 5 repräsentiert ist.

8. Chimäres Protein nach einem der vorhergehenden Ansprüche, **dadurch gekennzeichnet, dass** das chimäre Protein wenigstens zwei Gruppen umfasst, wobei jede Gruppe wenigstens eine menschliche Amyloid-P-Komponente und wenigstens ein Fragment einer Fc-Region eines menschlichen Antikörpers umfasst, die miteinander mittels einer Gelenkregion verbunden sind, wobei die beiden Gruppen miteinander durch wenigstens eine Disulfidbrücke verbunden sind und wobei die Fragmente einer Fc-Region eines menschlichen Antikörpers identisch oder unterschiedlich sind.

9. Chimäres Protein nach einem der Ansprüche 1 bis 7, **dadurch gekennzeichnet, dass** das chimäre Protein wenigstens eine menschliche Amyloid-P-Komponente und wenigstens zwei identische oder unterschiedliche Fragmente einer Fc-Region eines menschlichen Antikörpers umfasst, wobei die menschliche Amyloid-P-Komponente und das erste Fragment miteinander mittels einer Gelenkregion verbunden sind, wobei das erste und zweite Fragment einer Fc-Region miteinander auf kovalente Weise mittels einer Bindung verbunden sind, die durch eine Abstandskette und eine Gelenkregion gebildet und zu der zuvor genannten identisch oder verschieden ist, und eine einzige Polypeptidkette bilden, die eine funktionelle dimere Fc-Region bildet.

10. Chimäres Protein nach dem vorhergehenden Anspruch, **dadurch gekennzeichnet, dass** die Abstandskette durch wenigstens eine Aminosäuresequenz SEQ ID NO: 5 gebildet wird.

11. Nukleinsäure, die wenigstens eine Polynukleotidsequenz umfasst, die für wenigstens ein chimäres Protein gemäß der Definition in den Ansprüchen 1 bis 7 und 9 bis 10 kodiert.

12. Vektor, in den eine Nukleinsäure gemäß der Definition in Anspruch 11 eingefügt ist.

13. Mit einem Vektor gemäß der Definition in Anspruch 12 transfizierte Wirtszelle.

14. Herstellungsverfahren für ein chimäres Protein gemäß der Definition in den Ansprüchen 1 bis 10, wobei das Verfahren wenigstens die folgenden Schritte umfasst:
a) Transfektion einer Wirtszelle mit einem Vektor gemäß der Definition in Anspruch 12;
b) Kultivierung der Wirtszelle unter derartigen Bedingungen, dass das chimäre Protein hergestellt wird; und
c) Gewinnung des durch die Wirtszelle hergestellten chimären Proteins am Ende von Schritt b).

15. Pharmazeutische Zusammensetzung, welche ein chimäres Protein gemäß den Definitionen in den Ansprüchen 1 bis 10 und einen akzeptablen pharmazeutischen Hilfsstoff umfasst.

16. Chimäres Protein gemäß der Definition in den Ansprüchen 1 bis 10 zur Verwendung als Medikament.

17. Chimäres Protein gemäß der Definition in den Ansprüchen 1 bis 10 zur Verwendung für die Elimination von Amyloseablagerungen in Organen.

18. Chimäres Protein gemäß der Definition in den Ansprüchen 1 bis 10 zur Verwendung in der Behandlung von Amylosen, insbesondere Amylosen vom AL-Typ.

19. Verwendung eines chimären Proteins gemäß der Definition in den Ansprüchen 1 bis 7 zur Herstellung eines chimären Proteins gemäß der Definition in Anspruch 8.

20. Verwendung wenigstens eines chimären Proteins gemäß den Definitionen in den Ansprüchen 1 bis 10 als in-vitro- oder in-vivo-Charakterisierungswerkzeug für das Vorhandensein einer Amylose-Ablagerung, wobei das chimäre Protein radioaktiv markiert ist, bevorzugt die menschliche Amyloid-P-Komponente durch ¹²¹I-SAP dargestellt wird und/oder mittels eines radioaktiv markierten anti-Fc-Antikörpers identifiziert wird, wobei beispielsweise der anti-Fc Antikörper an Peroxydase gekoppelt ist.

## Claims

1. A chimeric protein comprising at least one human amyloid P component and at least one fragment of an Fc region of a human antibody, the human amyloid P component and the fragment of an Fc region to which it is attached being bonded to each other by means of a hinge region.

2. The chimeric protein as claimed in claim 1, **characterized in that** the human amyloid P component comprises an amino acid sequence having at least 80% identity with the sequence SEQ ID NO: 1.

3. The chimeric protein as claimed in claim 1 or 2, **characterized in that** the Fc region of the antibody is the Fc region of a human immunoglobulin, preferably the Fc region of an IgG, better still the Fc region of an IgG1 or of an IgG2, and more particularly the Fc region of an IgG1.

4. The chimeric protein as claimed in any one of the preceding claims, **characterized in that** the fragment of an Fc region of a human antibody comprises at least one amino acid sequence having at least 80% identity with the sequence SEQ ID NO: 3

5. The chimeric protein as claimed in any one of the preceding claims, **characterized in that** said Fc region comprises at least two modifications in the amino acid sequence, namely:
(i) a modification in the amino acid sequence chosen from the group consisting of 378V, 378T, 434Y and 434S, and
(ii) at least one modification in the amino acid sequence chosen from the group consisting of 226G, 230S, 230T, 230L, 241 L, 264E, 307P, 315D, 330V, 362R, 378V, 378T, 389T, 389K, 434Y and 434S,
it being understood that the numbering of the amino acids of the Fc region is that of the EU index proposed by Kabat and on the condition that the modification (i) does not occur at the same amino acid position as the modification (ii).

6. The chimeric protein as claimed in any one of the preceding claims, **characterized in that** the hinge region comprises at least one amino acid sequence having at least 60% identity, preferably at least 80% identity, with a sequence chosen from the sequences SEQ ID NOs: 13 and 15 to 18, and preferably is represented by the sequence SEQ ID NO: 13.

7. The chimeric protein as claimed in any one of the preceding claims, **characterized in that** said chimeric protein also comprises, between the hinge region and the amyloid P component, at least one non-structuring peptide sequence, preferably represented by an amino acid sequence SEQ ID NO: 5.

8. The chimeric protein as claimed in any one of the preceding claims, **characterized in that** said chimeric protein comprises at least two units, each unit comprising at least one human amyloid P component and at least one fragment of an Fc region of a human antibody, bonded to each other by means of a hinge region, the two units being bonded to each other by at least one disulfide bridge and the fragments of an Fc region of a human antibody being identical or different.

9. The chimeric protein as claimed in any one of claims 1 to 7, **characterized in that** said chimeric protein comprises at least one human amyloid P component and at least two fragments of an Fc region of a human antibody, which may be identical or different, said human amyloid P component and the first fragment being bonded to each other by means of a hinge region, the first and second fragments of an Fc region being bonded to each other covalently by means of a bond formed from a spacer chain and a hinge region, identical to or different from that previously mentioned, and form a single polypeptide chain constituting a functional dimeric Fc region.

10. The chimeric protein as claimed in the preceding claim, **characterized in that** the spacer chain is represented by at least one amino acid sequence SEQ ID NO: 5.

11. A nucleic acid comprising at least one polynucleotide sequence encoding at least one chimeric protein as defined in claims 1 to 7 and 9 and 10.

12. A vector into which a nucleic acid as defined in claim 11 is inserted.

13. A host cell transfected with a vector as defined in claim 12.

14. A process for producing a chimeric protein as defined in claims 1 to 10, said process comprising at least the steps consisting in:
a) transfecting a host cell with a vector as defined in claim 12;
b) culturing said host cell under conditions such that the chimeric protein is produced; and
c) collecting said chimeric protein produced by said host cell at the end of step b).

15. A pharmaceutical composition comprising a chimeric protein as defined in claims 1 to 10, and a pharmaceutically acceptable excipient.

16. The chimeric protein as defined in claims 1 to 10, for use thereof as a medicament.

17. The chimeric protein as defined in claims 1 to 10, for use thereof for eliminating the amyloidosis deposits in the organs.

18. The chimeric protein as defined in claims 1 to 10, for use thereof for treating amyloidosis, in particular amyloidosis of AL type.

19. The use of a chimeric protein as defined in claims 1 to 7, for producing a chimeric protein as defined in claim 8.

20. The use of at least one chimeric protein as defined in claims 1 to 10, as a tool for *in vitro* or *ex vivo* characterization of the presence of an amyloidosis deposit, said chimeric protein being radiolabeled; preferably, the human amyloid P component is represented by ¹²³I-SAP, and/or identified by means of a radiolabeled anti-Fc antibody; for example, said anti-Fc antibody is coupled to peroxidase.


### SEQUENCE LISTING

<110> Laboratoire français du Fractionnement et des Biotechnologies
<120> FUSION Fc-SAP
<130> S15/BR70884/DC1/BAJ/11
<160> 46
<170> BiSSAP 1.0
<210> 1
   <211> 204
   <212> PRT
   <213> Homo sapiens
<220>
   <221> SOURCE
   <222> 1..204
   <223> /mol_type="protein" /note="Human SAP" /organism="Homo sapiens"
<400> 1
   His Thr Asp Leu Ser Gly Lys Val Phe Val Phe Pro Arg Glu Ser Val
   1               5                   10                  15
   Thr Asp His Val Asn Leu Ile Thr Pro Leu Glu Lys Pro Leu Gln Asn
               20                  25                  30
   Phe Thr Leu Cys Phe Arg Ala Tyr Ser Asp Leu Ser Arg Ala Tyr Ser
           35                  40                  45
   Leu Phe Ser Tyr Asn Thr Gln Gly Arg Asp Asn Glu Leu Leu Val Tyr
       50                  55                  60
   Lys Glu Arg Val Gly Glu Tyr Ser Leu Tyr Ile Gly Arg His Lys Val
   65                  70                  75                  80
   Thr Ser Lys Val Ile Glu Lys Phe Pro Ala Pro Val His Ile Cys Val
                   85                  90                  95
   Ser Trp Glu Ser Ser Ser Gly Ile Ala Glu Phe Trp Ile Asn Gly Thr
               100                 105                 110
   Pro Leu Val Lys Lys Gly Leu Arg Gln Gly Tyr Phe Val Glu Ala Gln
           115                 120                 125
   Pro Lys Ile Val Leu Gly Gln Glu Gln Asp Ser Tyr Gly Gly Lys Phe
       130                 135                 140
   Asp Arg Ser Gln Ser Phe Val Gly Glu Ile Gly Asp Leu Tyr Met Trp
   145                 150                 155                 160
   Asp Ser Val Leu Pro Pro Glu Asn Ile Leu Ser Ala Tyr Gln Gly Thr
                   165                 170                 175
   Pro Leu Pro Ala Asn Ile Leu Asp Trp Gln Ala Leu Asn Tyr Glu Ile
               180                 185                 190
   Arg Gly Tyr Val Ile Ile Lys Pro Leu Val Trp Val
           195                 200
<210> 2
   <211> 612
   <212> DNA
   <213> Homo sapiens
<220>
   <221> source
   <222> 1..612
   <223> /mol_type="DNA" /note="Huamn SAP" /organism="Homo sapiens"
<400> 2
   cacacagacc tcagtgggaa ggtgtttgta tttcctagag aatctgttac tgatcatgta     60
   aacttgatca caccgctgga gaagcctcta cagaacttta ccttgtgttt tcgagcctat    120
   agtgatctct ctcgtgccta cagcctcttc tcctacaata cccaaggcag ggataatgag    180
   ctactagttt ataaagaaag agttggagag tatagtctat acattggaag acacaaagtt    240
   acatccaaag ttatcgaaaa gttcccggct ccagtgcaca tctgtgtgag ctgggagtcc    300
   tcatcaggta ttgctgaatt ttggatcaat gggacacctt tggtgaaaaa gggtctgcga    360
   cagggttact ttgtggaagc tcagcccaag attgtcctgg ggcaggaaca ggattcctat    420
   gggggcaagt ttgataggag ccagtccttt gtgggagaga ttggggattt gtacatgtgg    480
   gactctgtgc tgcccccaga aaatatcctg tctgcctatc agggtacccc tctccctgcc    540
   aatatcctgg actggcaggc tctgaactat gaaatcagag gatatgtcat catcaaaccc    600
   ttggtgtggg tc                                                        612
<210> 3
   <211> 217
   <212> PRT
   <213> Homo sapiens
<220>
   <221> SOURCE
   <222> 1..217
   <223> /mol_type="protein" /note="Fragment of Fc region of a human antibody" /organism="Homo sapiens"
<400> 3
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Glu Glu Met
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
                 195                 200                 205
   Lys Ser Leu Ser Leu Ser Pro Gly Lys
       210                 215
<210> 4
   <211> 654
   <212> DNA
   <213> Homo sapiens
<220>
   <221> source
   <222> 1..654
   <223> /mol_type="DNA" /note="Fragment of Fc region of a human antibody" /organism="Homo sapiens"
<400> 4
   gcacctgaac tcctgggggg accgtcagtc ttcctcttcc ccccaaaacc caaggacacc 60
   ctcatgatct cccggacccc tgaggtcaca tgcgtggtgg tggacgtgag ccacgaagac 120
   cctgaggtca agttcaactg gtacgtggac ggcgtggagg tgcataatgc caagacaaag 180
   ccgcgggagg agcagtacaa cagcacgtac cgtgtggtca gcgtcctcac cgtcctgcac 240
   caggactggc tgaatggcaa ggagtacaag tgcaaggtct ccaacaaagc cctcccagcc 300
   cccatcgaga aaaccatctc caaagccaaa gggcagcccc gagaaccaca ggtgtacacc 360
   ctgcccccat cccgggagga gatgaccaag aaccaggtca gcctgacctg cctggtcaaa 420
   ggcttctatc ccagcgacat cgccgtggag tgggagagca atgggcagcc ggagaacaac 480
   tacaagacca cgcctcccgt gctggactcc gacggctcct tcttcctcta cagcaagctc 540
   accgtggaca agagcaggtg gcagcagggg aacgtcttct catgctccgt gatgcatgag 600
   gctctgcaca accactacac gcagaagagc ctctccctgt ctccgggtaa atga 654
<210> 5
   <211> 5
   <212> PRT
   <213> artificial sequences
<220>
   <221> SOURCE
   <222> 1..5
   <223> /mol_type="protein" /note="Non-structuring sequence or spacer chain" /organism="artificial sequences"
<400> 5
   Gly Gly Gly Gly Ser
   1 5
<210> 6
   <211> 43
   <212> DNA
   <213> artificial sequences
<220>
   <221> source
   <222> 1..43
   <223> /mol_type="DNA" /note="Non-structuring peptide sequence or spacer chain" /organism="artificial sequences"
<400> 6
   cttggtggag gcggttcagg cggaggtggc tctggcggtg gcg 43
<210> 7
   <211> 436
   <212> PRT
   <213> artificial sequences
<220>
   <221> SOURCE
   <222> 1..436
   <223> /mol_type="protein" /note="secreted monomeric SAP-Fc" /organism="artificial sequences"
<400> 7
   His Thr Asp Leu Ser Gly Lys Val Phe Val Phe Pro Arg Glu Ser Val
   1               5                   10                  15
   Thr Asp His Val Asn Leu Ile Thr Pro Leu Glu Lys Pro Leu Gln Asn
               20                  25                  30
   Phe Thr Leu Cys Phe Arg Ala Tyr Ser Asp Leu Ser Arg Ala Tyr Ser
           35                  40                  45
   Leu Phe Ser Tyr Asn Thr Gln Gly Arg Asp Asn Glu Leu Leu Val Tyr
       50                  55                  60
   Lys Glu Arg Val Gly Glu Tyr Ser Leu Tyr Ile Gly Arg His Lys Val
   65                  70                  75                  80
   Thr Ser Lys Val Ile Glu Lys Phe Pro Ala Pro Val His Ile Cys Val
                   85                  90                  95
   Ser Trp Glu Ser Ser Ser Gly Ile Ala Glu Phe Trp Ile Asn Gly Thr
               100                 105                 110
   Pro Leu Val Lys Lys Gly Leu Arg Gln Gly Tyr Phe Val Glu Ala Gln
           115                 120                 125
   Pro Lys Ile Val Leu Gly Gln Glu Gln Asp Ser Tyr Gly Gly Lys Phe
       130                 135                 140
   Asp Arg Ser Gln Ser Phe Val Gly Glu Ile Gly Asp Leu Tyr Met Trp
   145                 150                 155                 160
   Asp Ser Val Leu Pro Pro Glu Asn Ile Leu Ser Ala Tyr Gln Gly Thr
                   165                 170                 175
   Pro Leu Pro Ala Asn Ile Leu Asp Trp Gln Ala Leu Asn Tyr Glu Ile
               180                 185                 190
   Arg Gly Tyr Val Ile Ile Lys Pro Leu Val Trp Val Glu Pro Lys Ser
           195                 200                 205
   Cys Asp Lys Thr His Thr Cys Pro Pro Cys Pro Ala Pro Glu Leu Leu
       210                 215                 220
   Gly Gly Pro Ser Val Phe Leu Phe Pro Pro Lys Pro Lys Asp Thr Leu
   225                 230                 235                 240
   Met Ile Ser Arg Thr Pro Glu Val Thr Cys Val Val Val Asp Val Ser
                   245                 250                 255
   His Glu Asp Pro Glu Val Lys Phe Asn Trp Tyr Val Asp Gly Val Glu
               260                 265                 270
   Val His Asn Ala Lys Thr Lys Pro Arg Glu Glu Gln Tyr Asn Ser Thr
           275                 280                 285
   Tyr Arg Val Val Ser Val Leu Thr Val Leu His Gln Asp Trp Leu Asn
       290                 295                 300
   Gly Lys Glu Tyr Lys Cys Lys Val Ser Asn Lys Ala Leu Pro Ala Pro
   305                 310                 315                 320
   Ile Glu Lys Thr Ile Ser Lys Ala Lys Gly Gln Pro Arg Glu Pro Gln
                   325                 330                 335
   Val Tyr Thr Leu Pro Pro Ser Arg Glu Glu Met Thr Lys Asn Gln Val
               340                 345                 350
   Ser Leu Thr Cys Leu Val Lys Gly Phe Tyr Pro Ser Asp Ile Ala Val
           355                 360                 365
   Glu Trp Glu Ser Asn Gly Gln Pro Glu Asn Asn Tyr Lys Thr Thr Pro
       370                 375                 380
   Pro Val Leu Asp Ser Asp Gly Ser Phe Phe Leu Tyr Ser Lys Leu Thr
   385                 390                 395                 400
   Val Asp Lys Ser Arg Trp Gln Gln Gly Asn Val Phe Ser Cys Ser Val
                   405                 410                 415
   Met His Glu Ala Leu His Asn His Tyr Thr Gln Lys Ser Leu Ser Leu
               420                 425                 430
   Ser Pro Gly Lys
           435
<210> 8
   <211> 1311
   <212> DNA
   <213> artificial sequences
<220>
   <221> source
   <222> 1..1311
   <223> /mol_type="DNA" /note="secreted monomeric SAP-Fc" /organism="artificial sequences"
<400> 8
   cacacagacc tcagtgggaa ggtgtttgta tttcctagag aatctgttac tgatcatgta      60
   aacttgatca caccgctgga gaagcctcta cagaacttta ccttgtgttt tcgagcctat     120
   agtgatctct ctcgtgccta cagcctcttc tcctacaata cccaaggcag ggataatgag     180
   ctactagttt ataaagaaag agttggagag tatagtctat acattggaag acacaaagtt     240
   acatccaaag ttatcgaaaa gttcccggct ccagtgcaca tctgtgtgag ctgggagtcc     300
   tcatcaggta ttgctgaatt ttggatcaat gggacacctt tggtgaaaaa gggtctgcga     360
   cagggttact ttgtggaagc tcagcccaag attgtcctgg ggcaggaaca ggattcctat     420
   gggggcaagt ttgataggag ccagtccttt gtgggagaga ttggggattt gtacatgtgg     480
   gactctgtgc tgcccccaga aaatatcctg tctgcctatc agggtacccc tctccctgcc     540
   aatatcctgg actggcaggc tctgaactat gaaatcagag gatatgtcat catcaaaccc     600
   ttggtgtggg tcgagcccaa atcttgtgac aaaactcaca catgcccacc gtgcccagca     660
   cctgaactcc tggggggacc gtcagtcttc ctcttccccc caaaacccaa ggacaccctc     720
   atgatctccc ggacccctga ggtcacatgc gtggtggtgg acgtgagcca cgaagaccct     780
   gaggtcaagt tcaactggta cgtggacggc gtggaggtgc ataatgccaa gacaaagccg     840
   cgggaggagc agtacaacag cacgtaccgt gtggtcagcg tcctcaccgt cctgcaccag     900
   gactggctga atggcaagga gtacaagtgc aaggtctcca acaaagccct cccagccccc     960
   atcgagaaaa ccatctccaa agccaaaggg cagccccgag aaccacaggt gtacaccctg    1020
   cccccatccc gggaggagat gaccaagaac caggtcagcc tgacctgcct ggtcaaaggc    1080
   ttctatccca gcgacatcgc cgtggagtgg gagagcaatg ggcagccgga gaacaactac    1140
   aagaccacgc ctcccgtgct ggactccgac ggctccttct tcctctacag caagctcacc    1200
   gtggacaaga gcaggtggca gcaggggaac gtcttctcat gctccgtgat gcatgaggct    1260
   ctgcacaacc actacacgca gaagagcctc tccctgtctc cgggtaaatg a             1311
<210> 9
   <211> 684
   <212> PRT
   <213> artificial sequences
<220>
   <221> SOURCE
   <222> 1..684
   <223> /mol_type="protein" /note="Secreted monomeric SAP-ScFc" /organism="artificial sequences"
<400> 9
   His Thr Asp Leu Ser Gly Lys Val Phe Val Phe Pro Arg Glu Ser Val
   1               5                   10                  15
   Thr Asp His Val Asn Leu Ile Thr Pro Leu Glu Lys Pro Leu Gln Asn
               20                  25                  30
   Phe Thr Leu Cys Phe Arg Ala Tyr Ser Asp Leu Ser Arg Ala Tyr Ser
           35                  40                  45
   Leu Phe Ser Tyr Asn Thr Gln Gly Arg Asp Asn Glu Leu Leu Val Tyr
       50                  55                  60
   Lys Glu Arg Val Gly Glu Tyr Ser Leu Tyr Ile Gly Arg His Lys Val
   65                  70                  75                  80
   Thr Ser Lys Val Ile Glu Lys Phe Pro Ala Pro Val His Ile Cys Val
                   85                  90                  95
   Ser Trp Glu Ser Ser Ser Gly Ile Ala Glu Phe Trp Ile Asn Gly Thr
               100                 105                 110
   Pro Leu Val Lys Lys Gly Leu Arg Gln Gly Tyr Phe Val Glu Ala Gln
           115                 120                 125
   Pro Lys Ile Val Leu Gly Gln Glu Gln Asp Ser Tyr Gly Gly Lys Phe
       130                 135                 140
   Asp Arg Ser Gln Ser Phe Val Gly Glu Ile Gly Asp Leu Tyr Met Trp
   145                 150                 155                 160
   Asp Ser Val Leu Pro Pro Glu Asn Ile Leu Ser Ala Tyr Gln Gly Thr
                   165                 170                 175
   Pro Leu Pro Ala Asn Ile Leu Asp Trp Gln Ala Leu Asn Tyr Glu Ile
               180                 185                 190
   Arg Gly Tyr Val Ile Ile Lys Pro Leu Val Trp Val Glu Pro Lys Ser
           195                 200                 205
   Cys Asp Lys Thr His Thr Cys Pro Pro Cys Pro Ala Pro Glu Leu Leu
       210                 215                 220
   Gly Gly Pro Ser Val Phe Leu Phe Pro Pro Lys Pro Lys Asp Thr Leu
   225                 230                 235                 240
   Met Ile Ser Arg Thr Pro Glu Val Thr Cys Val Val Val Asp Val Ser
                   245                 250                 255
   His Glu Asp Pro Glu Val Lys Phe Asn Trp Tyr Val Asp Gly Val Glu
               260                 265                 270
   Val His Asn Ala Lys Thr Lys Pro Arg Glu Glu Gln Tyr Asn Ser Thr
           275                 280                 285
   Tyr Arg Val Val Ser Val Leu Thr Val Leu His Gln Asp Trp Leu Asn
       290                 295                 300
   Gly Lys Glu Tyr Lys Cys Lys Val Ser Asn Lys Ala Leu Pro Ala Pro
   305                 310                 315                 320
   Ile Glu Lys Thr Ile Ser Lys Ala Lys Gly Gln Pro Arg Glu Pro Gln
                   325                 330                 335
   Val Tyr Thr Leu Pro Pro Ser Arg Glu Glu Met Thr Lys Asn Gln Val
               340                 345                 350
   Ser Leu Thr Cys Leu Val Lys Gly Phe Tyr Pro Ser Asp Ile Ala Val
           355                 360                 365
   Glu Trp Glu Ser Asn Gly Gln Pro Glu Asn Asn Tyr Lys Thr Thr Pro
       370                 375                 380
   Pro Val Leu Asp Ser Asp Gly Ser Phe Phe Leu Tyr Ser Lys Leu Thr
   385                 390                 395                 400
   Val Asp Lys Ser Arg Trp Gln Gln Gly Asn Val Phe Ser Cys Ser Val
                   405                 410                 415
   Met His Glu Ala Leu His Asn His Tyr Thr Gln Lys Ser Leu Ser Leu
               420                 425                 430
   Ser Pro Gly Lys Leu Gly Gly Gly Gly Ser Gly Gly Gly Gly Ser Gly
           435                 440                 445
   Gly Gly Gly Ser Glu Pro Lys Ser Cys Asp Lys Thr His Thr Cys Pro
       450                 455                 460
   Pro Cys Pro Ala Pro Glu Leu Leu Gly Gly Pro Ser Val Phe Leu Phe
   465                 470                 475                 480
   Pro Pro Lys Pro Lys Asp Thr Leu Met Ile Ser Arg Thr Pro Glu Val
                   485                 490                 495
   Thr Cys Val Val Val Asp Val Ser His Glu Asp Pro Glu Val Lys Phe
               500                 505                 510
   Asn Trp Tyr Val Asp Gly Val Glu Val His Asn Ala Lys Thr Lys Pro
           515                 520                 525
   Arg Glu Glu Gln Tyr Asn Ser Thr Tyr Arg Val Val Ser Val Leu Thr
       530                 535                 540
   Val Leu His Gln Asp Trp Leu Asn Gly Lys Glu Tyr Lys Cys Lys Val
   545                 550                 555                 560
   Ser Asn Lys Ala Leu Pro Ala Pro Ile Glu Lys Thr Ile Ser Lys Ala
                   565                 570                 575
   Lys Gly Gln Pro Arg Glu Pro Gln Val Tyr Thr Leu Pro Pro Ser Arg
               580                 585                 590
   Glu Glu Met Thr Lys Asn Gln Val Ser Leu Thr Cys Leu Val Lys Gly
           595                 600                 605
   Phe Tyr Pro Ser Asp Ile Ala Val Glu Trp Glu Ser Asn Gly Gln Pro
       610                 615                 620
   Glu Asn Asn Tyr Lys Thr Thr Pro Pro Val Leu Asp Ser Asp Gly Ser
   625                 630                 635                 640
   Phe Phe Leu Tyr Ser Lys Leu Thr Val Asp Lys Ser Arg Trp Gln Gln
                   645                 650                 655
   Gly Asn Val Phe Ser Cys Ser Val Met His Glu Ala Leu His Asn His
               660                 665                 670
   Tyr Thr Gln Lys Ser Leu Ser Leu Ser Pro Gly Lys
           675                 680
<210> 10
   <211> 2055
   <212> DNA
   <213> artificial sequences
<220>
   <221> source
   <222> 1..2055
   <223> /mol_type="DNA" /note="Secreted monomeric SAP-ScFc" /organism="artificial sequences"
<400> 10
   cacacagacc tcagtgggaa ggtgtttgta tttcctagag aatctgttac tgatcatgta 60
   aacttgatca caccgctgga gaagcctcta cagaacttta ccttgtgttt tcgagcctat      120
   agtgatctct ctcgtgccta cagcctcttc tcctacaata cccaaggcag ggataatgag      180
   ctactagttt ataaagaaag agttggagag tatagtctat acattggaag acacaaagtt      240
   acatccaaag ttatcgaaaa gttcccggct ccagtgcaca tctgtgtgag ctgggagtcc      300
   tcatcaggta ttgctgaatt ttggatcaat gggacacctt tggtgaaaaa gggtctgcga      360
   cagggttact ttgtggaagc tcagcccaag attgtcctgg ggcaggaaca ggattcctat      420
   gggggcaagt ttgataggag ccagtccttt gtgggagaga ttggggattt gtacatgtgg      480
   gactctgtgc tgcccccaga aaatatcctg tctgcctatc agggtacccc tctccctgcc      540
   aatatcctgg actggcaggc tctgaactat gaaatcagag gatatgtcat catcaaaccc      600
   ttggtgtggg tcgagcccaa atcttgtgac aaaactcaca catgcccacc gtgcccagca      660
   cctgaactcc tggggggacc gtcagtcttc ctcttccccc caaaacccaa ggacaccctc      720
   atgatctccc ggacccctga ggtcacatgc gtggtggtgg acgtgagcca cgaagaccct      780
   gaggtcaagt tcaactggta cgtggacggc gtggaggtgc ataatgccaa gacaaagccg      840
   cgggaggagc agtacaacag cacgtaccgt gtggtcagcg tcctcaccgt cctgcaccag      900
   gactggctga atggcaagga gtacaagtgc aaggtctcca acaaagccct cccagccccc      960
   atcgagaaaa ccatctccaa agccaaaggg cagccccgag aaccacaggt gtacaccctg     1020
   cccccatccc gggaggagat gaccaagaac caggtcagcc tgacctgcct ggtcaaaggc     1080
   ttctatccca gcgacatcgc cgtggagtgg gagagcaatg ggcagccgga gaacaactac     1140
   aagaccacgc ctcccgtgct ggactccgac ggctccttct tcctctatag caagctcacc     1200
   gtggacaaga gcaggtggca gcaggggaac gtcttctcat gctccgtgat gcatgaggct     1260
   ctgcacaacc actacacgca gaagagcctc tccctgtctc cgggtaagct tggtggaggc     1320
   ggttcaggcg gaggtggctc tggcggtggc ggatctgagc ccaaatcttg tgacaaaact     1380
   cacacatgcc caccgtgccc agcacctgaa ctcctggggg gaccgtcagt cttcctcttc     1440
   cccccaaaac ccaaggacac cctcatgatc tcccggaccc ctgaggtcac atgcgtggtg     1500
   gtggacgtga gccacgaaga ccctgaggtc aagttcaact ggtacgtgga cggcgtggag     1560
   gtgcataatg ccaagacaaa gccgcgggag gagcagtaca acagcacgta ccgtgtggtc     1620
   agcgtcctca ccgtcctgca ccaggactgg ctgaatggca aggagtacaa gtgcaaggtc     1680
   tccaacaaag ccctcccagc ccccatcgag aaaaccatct ccaaagccaa agggcagccc     1740
   cgagaaccac aggtgtacac cctgccccca tcccgggagg agatgaccaa gaaccaggtc     1800
   agcctgacct gcctggtcaa aggcttctat cccagcgaca tcgccgtgga gtgggagagc     1860
   aatgggcagc cggagaacaa ctacaagacc acgcctcccg tgctggactc cgacggctcc     1920
   ttcttcctct acagcaagct caccgtggac aagagcaggt ggcagcaggg gaacgtcttc     1980
   tcatgctccg tgatgcatga ggctctgcac aaccactaca cgcagaagag cctctccctg 2040
   tctccgggta aatga 2055
<210> 11
   <211> 480
   <212> PRT
   <213> artificial sequences
<220>
   <221> SOURCE
   <222> 1..480
   <223> /mol_type="protein" /note="Hinge + two fragments of Fc region of human antibody of SA P-ScFc" /organism="artificial sequences"
<400> 11
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Glu Glu Met Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys Leu Gly Gly Gly Gly Ser Gly Gly
   225                 230                 235                 240
   Gly Gly Ser Gly Gly Gly Gly Ser Glu Pro Lys Ser Cys Asp Lys Thr
                   245                 250                 255
   His Thr Cys Pro Pro Cys Pro Ala Pro Glu Leu Leu Gly Gly Pro Ser
               260                 265                 270
   Val Phe Leu Phe Pro Pro Lys Pro Lys Asp Thr Leu Met Ile Ser Arg
           275                 280                 285
   Thr Pro Glu Val Thr Cys Val Val Val Asp Val Ser His Glu Asp Pro
       290                 295                 300
   Glu Val Lys Phe Asn Trp Tyr Val Asp Gly Val Glu Val His Asn Ala
   305                 310                 315                 320
   Lys Thr Lys Pro Arg Glu Glu Gln Tyr Asn Ser Thr Tyr Arg Val Val
                   325                 330                 335
   Ser Val Leu Thr Val Leu His Gln Asp Trp Leu Asn Gly Lys Glu Tyr
                   340                 345                 350
   Lys Cys Lys Val Ser Asn Lys Ala Leu Pro Ala Pro Ile Glu Lys Thr
               355                 360                 365
   Ile Ser Lys Ala Lys Gly Gln Pro Arg Glu Pro Gln Val Tyr Thr Leu
           370                 375                 380
   Pro Pro Ser Arg Glu Glu Met Thr Lys Asn Gln Val Ser Leu Thr Cys
   385                 390                 395                 400
   Leu Val Lys Gly Phe Tyr Pro Ser Asp Ile Ala Val Glu Trp Glu Ser
                   405                 410                 415
   Asn Gly Gln Pro Glu Asn Asn Tyr Lys Thr Thr Pro Pro Val Leu Asp
               420                 425                 430
   Ser Asp Gly Ser Phe Phe Leu Tyr Ser Lys Leu Thr Val Asp Lys Ser
           435                 440                 445
   Arg Trp Gln Gln Gly Asn Val Phe Ser Cys Ser Val Met His Glu Ala
       450                 455                 460
   Leu His Asn His Tyr Thr Gln Lys Ser Leu Ser Leu Ser Pro Gly Lys
   465                 470                 475                 480
<210> 12
   <211> 1443
   <212> DNA
   <213> artificial sequences
<220>
   <221> source
   <222> 1..1443
   <223> /mol_type="DNA" /note="Hinge + two fragments of Fc region of human antibody of SA P-ScFc" /organism="artificial sequences"
<400> 12
   gagcccaaat cttgtgacaa aactcacaca tgcccaccgt gcccagcacc tgaactcctg      60
   gggggaccgt cagtcttcct cttcccccca aaacccaagg acaccctcat gatctcccgg     120
   acccctgagg tcacatgcgt ggtggtggac gtgagccacg aagaccctga ggtcaagttc     180
   aactggtacg tggacggcgt ggaggtgcat aatgccaaga caaagccgcg ggaggagcag     240
   tacaacagca cgtaccgtgt ggtcagcgtc ctcaccgtcc tgcaccagga ctggctgaat     300
   ggcaaggagt acaagtgcaa ggtctccaac aaagccctcc cagcccccat cgagaaaacc     360
   atctccaaag ccaaagggca gccccgagaa ccacaggtgt acaccctgcc cccatcccgg     420
   gaggagatga ccaagaacca ggtcagcctg acctgcctgg tcaaaggctt ctatcccagc     480
   gacatcgccg tggagtggga gagcaatggg cagccggaga acaactacaa gaccacgcct     540
   cccgtgctgg actccgacgg ctccttcttc ctctatagca agctcaccgt ggacaagagc     600
   aggtggcagc aggggaacgt cttctcatgc tccgtgatgc atgaggctct gcacaaccac     660
   tacacgcaga agagcctctc cctgtctccg ggtaagcttg gtggaggcgg ttcaggcgga     720
   ggtggctctg gcggtggcgg atctgagccc aaatcttgtg acaaaactca cacatgccca     780
   ccgtgcccag cacctgaact cctgggggga ccgtcagtct tcctcttccc cccaaaaccc     840
   aaggacaccc tcatgatctc ccggacccct gaggtcacat gcgtggtggt ggacgtgagc     900
   cacgaagacc ctgaggtcaa gttcaactgg tacgtggacg gcgtggaggt gcataatgcc     960
   aagacaaagc cgcgggagga gcagtacaac agcacgtacc gtgtggtcag cgtcctcacc    1020
   gtcctgcacc aggactggct gaatggcaag gagtacaagt gcaaggtctc caacaaagcc    1080
   ctcccagccc ccatcgagaa aaccatctcc aaagccaaag ggcagccccg agaaccacag    1140
   gtgtacaccc tgcccccatc ccgggaggag atgaccaaga accaggtcag cctgacctgc    1200
   ctggtcaaag gcttctatcc cagcgacatc gccgtggagt gggagagcaa tgggcagccg    1260
   gagaacaact acaagaccac gcctcccgtg ctggactccg acggctcctt cttcctctac    1320
   agcaagctca ccgtggacaa gagcaggtgg cagcagggga acgtcttctc atgctccgtg    1380
   atgcatgagg ctctgcacaa ccactacacg cagaagagcc tctccctgtc tccgggtaaa    1440
   tga                                                                  1443
<210> 13
   <211> 15
   <212> PRT
   <213> Homo sapiens
<220>
   <221> SOURCE
   <222> 1..15
   <223> /mol_type="protein" /note="Hinge region of human IgG1" /organism="Homo sapiens"
<400> 13
   Glu Pro Lys Ser Cys Asp Lys Thr His Thr Cys Pro Pro Cys Pro
   1               5                   10                  15
<210> 14
   <211> 23
   <212> DNA
   <213> Homo sapiens
<220>
   <221> source
   <222> 1..23
   <223> /mol_type="DNA" /note="Hinge region of human IgG1" /organism="Homo sapiens"
<400> 14
   ctcacacatg cccaccgtgc cca 23
<210> 15
   <211> 12
   <212> PRT
   <213> Homo sapiens
<220>
   <221> SOURCE
   <222> 1..12
   <223> /mol_type="protein" /note="Hinge region of human IgG2" /organism="Homo sapiens"
<400> 15
   Glu Arg Lys Cys Cys Val Glu Cys Pro Pro Cys Pro
   1               5                   10
<210> 16
   <211> 17
   <212> PRT
   <213> Homo sapiens
<220>
   <221> SOURCE
   <222> 1..17
   <223> /mol_type="protein" /note="Hinge region of human IgG3" /organism="Homo sapiens"
<400> 16
   Glu Leu Lys Thr Pro Leu Gly Asp Thr Thr His Thr Cys Pro Arg Cys
   1               5                   10                  15
   Pro
<210> 17
   <211> 15
   <212> PRT
   <213> Homo sapiens
<220>
   <221> SOURCE
   <222> 1..15
   <223> /mol_type="protein" /note="Hinge region of human IgG3" /organism="Homo sapiens"
<400> 17
   Glu Pro Lys Ser Cys Asp Thr Pro Pro Pro Cys Pro Arg Cys Pro
   1               5                   10                  15
<210> 18
   <211> 12
   <212> PRT
   <213> Homo sapiens
<220>
   <221> SOURCE
   <222> 1..12
   <223> /mol_type="protein" /note="Hinge region of human IgG4" /organism="Homo sapiens"
<400> 18
   Glu Ser Lys Tyr Gly Pro Pro Cys Pro Ser Cys Pro
   1               5                   10
<210> 19
   <211> 10
   <212> PRT
   <213> Homo sapiens
<220>
   <221> SOURCE
   <222> 1..10
   <223> /mol_type="protein" /note="Shortened hinge region of IgG1" /organism="Homo sapiens"
<400> 19
   Asp Lys Thr His Thr Cys Pro Pro Cys Pro
   1               5                   10
<210> 20
   <211> 19
   <212> PRT
   <213> Homo sapiens
<220>
   <221> SOURCE
   <222> 1..19
   <223> /mol_type="protein" /note="Natural signal peptide of SAP" /organism="Homo sapiens"
<400> 20
   Met Asn Lys Pro Leu Leu Trp Ile Ser Val Leu Thr Ser Leu Leu Glu
   1               5                   10                  15
   Ala Phe Ala
<210> 21
   <211> 57
   <212> DNA
   <213> Homo sapiens
<220>
   <221> source
   <222> 1..57
   <223> /mol_type="DNA" /note="Natural signal peptide of SAP" /organism="Homo sapiens"
<400> 21
   atgaacaagc cgctcctttg gatctctgtc ctcaccagcc tcctggaagc ctttgct 57
<210> 22
   <211> 23
   <212> PRT
   <213> Homo sapiens
<220>
   <221> SOURCE
   <222> 1..23
   <223> /mol_type="protein" /note="Human TIMP signal peptide MMP1" /organism="Homo sapiens"
<400> 22
   Met Ala Pro Phe Glu Pro Leu Ala Ser Gly Ile Leu Leu Leu Leu Trp
   1               5                   10                  15
   Leu Ile Ala Pro Ser Arg Ala
               20
<210> 23
   <211> 24
   <212> PRT
   <213> Homo sapiens
<220>
   <221> SOURCE
   <222> 1..24
   <223> /mol_type="protein" /note="human insulin signal peptide" /organism="Homo sapiens"
<400> 23
   Met Ala Leu Trp Met Arg Leu Leu Pro Leu Leu Ala Leu Leu Ala Leu
   1               5                   10                  15
   Trp Gly Pro Asp Pro Ala Ala Ala
               20
<210> 24
   <211> 27
   <212> PRT
   <213> Homo sapiens
<220>
   <221> SOURCE
   <222> 1..27
   <223> /mol_type="protein" /note="human EPO signal peptide" /organism="Homo sapiens"
<400> 24
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly
               20                  25
<210> 25
   <211> 18
   <212> PRT
   <213> artificial sequences
<220>
   <221> SOURCE
   <222> 1..18
   <223> /mol_type="protein" /note="MB7 signal peptide " /organism="artificial sequences"
<400> 25
   Met Arg Trp Ser Trp Ile Phe Leu Leu Leu Leu Ser Ile Thr Ser Ala
   1               5                   10                  15
   Asn Ala
<210> 26
   <211> 19
   <212> PRT
   <213> artificial sequences
<220>
   <221> SOURCE
   <222> 1..19
   <223> /mol_type="protein" /note="AMHRII signal peptide " /organism="artificial sequences"
<400> 26
   Met Arg Trp Ser Trp Ile Phe Leu Phe Leu Leu Ser Ile Thr Ala Ser
   1               5                   10                  15
   Val His Cys
<210> 27
   <211> 19
   <212> PRT
   <213> artificial sequences
<220>
   <221> SOURCE
   <222> 1..19
   <223> /mol_type="protein" /note="XXII49 signal peptide " /organism="artificial sequences"
<400> 27
   Met Ala Trp Val Trp Thr Leu Leu Phe Leu Met Ala Ala Ala Gln Ser
   1               5                   10                  15
   Ala Gln Ala
<210> 28
   <211> 680
   <212> DNA
   <213> artificial sequences
<220>
   <221> SOURCE
   <222> 1..680
   <223> /mol_type="DNA" /note="SAP" /organism="artificial sequences"
<400> 28
   gtcgacacca tgaacaagcc gctctttgga tctctgtcct caccagcctc ctggaagcct      60
   ttgctcacac agacctcagt gggaaggtgt ttgtatttcc tagagaatct gttactgatc     120
   atgtaaactt gatcacaccg ctggagaagc ctctacagaa ctttaccttg tgttttcgag     180
   cctatagtga tctctctcgt gcctacagcc tcttctccta caatacccaa ggcagggata     240
   atgagctact agtttataaa gaaagagttg gagagtatag tctatacatt ggaagacaca     300
   aagttacatc caaagttatc gaaaagttcc cggctccagt gcacatctgt gtgagctggg     360
   agtcctcatc aggtattgct gaattttgga tcaatgggac acctttggtg aaaaagggtc     420
   tgcgacaggg ttactttgtg gaagctcagc ccaagattgt cctggggcag gaacaggatt     480
   cctatggggg caagtttgat aggagccagt cctttgtggg agagattggg gatttgtaca     540
   tgtgggactc tgtgctgccc ccagaaaata tcctgtctgc ctatcagggt acccctctcc     600
   ctgccaatat cctggactgg caggctctga actatgaaat cagaggatat gtcatcatca     660
   aacccttggt gtgggtcgac                                                 680
<210> 29
   <211> 722
   <212> DNA
   <213> artificial sequences
<220>
   <221> source
   <222> 1..722
   <223> /mol_type="DNA" /note="Fragment of the Fc region of a human antibody" /organism="artificial sequences"
<400> 29
   atactctcga gcccaaatct tgtgacaaaa ctcacacatg cccaccgtgc ccagcacctg 60
   aactcctggg gggaccgtca gtcttcctct tccccccaaa acccaaggac accctcatga 120
   tctcccggac ccctgaggtc acatgcgtgg tggtggacgt gagccacgaa gaccctgagg 180
   tcaagttcaa ctggtacgtg gacggcgtgg aggtgcataa tgccaagaca aagccgcggg 240
   aggagcagta caacagcacg taccgtgtgg tcagcgtcct caccgtcctg caccaggact 300
   ggctgaatgg caaggagtac aagtgcaagg tctccaacaa agccctccca gcccccatcg 360
   agaaaaccat ctccaaagcc aaagggcagc cccgagaacc acaggtgtac accctgcccc 420
   catcccggga ggagatgacc aagaaccagg tcagcctgac ctgcctggtc aaaggcttct 480
   atcccagcga catcgccgtg gagtgggaga gcaatgggca gccggagaac aactacaaga 540
   ccacgcctcc cgtgctggac tccgacggct ccttcttcct ctacagcaag ctcaccgtgg 600
   acaagagcag gtggcagcag gggaacgtct tctcatgctc cgtgatgcat gaggctctgc 660
   acaaccacta cacgcagaag agcctctccc tgtctccggg taaatgagtg ctccggacag 720
   at 722
<210> 30
   <211> 1368
   <212> DNA
   <213> artificial sequences
<220>
   <221> source
   <222> 1..1368
   <223> /mol_type="DNA" /note="Monomeric SAP-Fc" /organism="artificial sequences"
<400> 30
   atgaacaagc cgctcctttg gatctctgtc ctcaccagcc tcctggaagc ctttgctcac 60
   acagacctca gtgggaaggt gtttgtattt cctagagaat ctgttactga tcatgtaaac 120
   ttgatcacac cgctggagaa gcctctacag aactttacct tgtgttttcg agcctatagt 180
   gatctctctc gtgcctacag cctcttctcc tacaataccc aaggcaggga taatgagcta 240
   ctagtttata aagaaagagt tggagagtat agtctataca ttggaagaca caaagttaca 300
   tccaaagtta tcgaaaagtt cccggctcca gtgcacatct gtgtgagctg ggagtcctca 360
   tcaggtattg ctgaattttg gatcaatggg acacctttgg tgaaaaaggg tctgcgacag 420
   ggttactttg tggaagctca gcccaagatt gtcctggggc aggaacagga ttcctatggg 480
   ggcaagtttg ataggagcca gtcctttgtg ggagagattg gggatttgta catgtgggac 540
   tctgtgctgc ccccagaaaa tatcctgtct gcctatcagg gtacccctct ccctgccaat 600
   atcctggact ggcaggctct gaactatgaa atcagaggat atgtcatcat caaacccttg 660
   gtgtgggtcg agcccaaatc ttgtgacaaa actcacacat gcccaccgtg cccagcacct 720
   gaactcctgg ggggaccgtc agtcttcctc ttccccccaa aacccaagga caccctcatg 780
   atctcccgga cccctgaggt cacatgcgtg gtggtggacg tgagccacga agaccctgag 840
   gtcaagttca actggtacgt ggacggcgtg gaggtgcata atgccaagac aaagccgcgg 900
   gaggagcagt acaacagcac gtaccgtgtg gtcagcgtcc tcaccgtcct gcaccaggac 960
   tggctgaatg gcaaggagta caagtgcaag gtctccaaca aagccctccc agcccccatc 1020
   gagaaaacca tctccaaagc caaagggcag ccccgagaac cacaggtgta caccctgccc 1080
   ccatcccggg aggagatgac caagaaccag gtcagcctga cctgcctggt caaaggcttc 1140
   tatcccagcg acatcgccgt ggagtgggag agcaatgggc agccggagaa caactacaag 1200
   accacgcctc ccgtgctgga ctccgacggc tccttcttcc tctacagcaa gctcaccgtg 1260
   gacaagagca ggtggcagca ggggaacgtc ttctcatgct ccgtgatgca tgaggctctg 1320
   cacaaccact acacgcagaa gagcctctcc ctgtctccgg gtaaatga 1368
<210> 31
   <211> 455
   <212> PRT
   <213> artificial sequences
<220>
   <221> SOURCE
   <222> 1..455
   <223> /mol_type="protein" /note="Monomeric SAP-Fc" /organism="artificial sequences"
<400> 31
   Met Asn Lys Pro Leu Leu Trp Ile Ser Val Leu Thr Ser Leu Leu Glu
   1               5                   10                  15
   Ala Phe Ala His Thr Asp Leu Ser Gly Lys Val Phe Val Phe Pro Arg
               20                  25                  30
   Glu Ser Val Thr Asp His Val Asn Leu Ile Thr Pro Leu Glu Lys Pro
           35                  40                  45
   Leu Gln Asn Phe Thr Leu Cys Phe Arg Ala Tyr Ser Asp Leu Ser Arg
       50                  55                  60
   Ala Tyr Ser Leu Phe Ser Tyr Asn Thr Gln Gly Arg Asp Asn Glu Leu
   65                  70                  75                  80
   Leu Val Tyr Lys Glu Arg Val Gly Glu Tyr Ser Leu Tyr Ile Gly Arg
                   85                  90                  95
   His Lys Val Thr Ser Lys Val Ile Glu Lys Phe Pro Ala Pro Val His
               100                 105                 110
   Ile Cys Val Ser Trp Glu Ser Ser Ser Gly Ile Ala Glu Phe Trp Ile
           115                 120                 125
   Asn Gly Thr Pro Leu Val Lys Lys Gly Leu Arg Gln Gly Tyr Phe Val
       130                 135                 140
   Glu Ala Gln Pro Lys Ile Val Leu Gly Gln Glu Gln Asp Ser Tyr Gly
   145                 150                 155                 160
   Gly Lys Phe Asp Arg Ser Gln Ser Phe Val Gly Glu Ile Gly Asp Leu
                   165                 170                 175
   Tyr Met Trp Asp Ser Val Leu Pro Pro Glu Asn Ile Leu Ser Ala Tyr
               180                 185                 190
   Gln Gly Thr Pro Leu Pro Ala Asn Ile Leu Asp Trp Gln Ala Leu Asn
           195                 200                 205
   Tyr Glu Ile Arg Gly Tyr Val Ile Ile Lys Pro Leu Val Trp Val Glu
       210                 215                 220
   Pro Lys Ser Cys Asp Lys Thr His Thr Cys Pro Pro Cys Pro Ala Pro
   225                 230                 235                 240
   Glu Leu Leu Gly Gly Pro Ser Val Phe Leu Phe Pro Pro Lys Pro Lys
                   245                 250                 255
   Asp Thr Leu Met Ile Ser Arg Thr Pro Glu Val Thr Cys Val Val Val
               260                 265                 270
   Asp Val Ser His Glu Asp Pro Glu Val Lys Phe Asn Trp Tyr Val Asp
           275                 280                 285
   Gly Val Glu Val His Asn Ala Lys Thr Lys Pro Arg Glu Glu Gln Tyr
       290                 295                 300
   Asn Ser Thr Tyr Arg Val Val Ser Val Leu Thr Val Leu His Gln Asp
   305                 310                 315                 320
   Trp Leu Asn Gly Lys Glu Tyr Lys Cys Lys Val Ser Asn Lys Ala Leu
                   325                 330                 335
   Pro Ala Pro Ile Glu Lys Thr Ile Ser Lys Ala Lys Gly Gln Pro Arg
               340                 345                 350
   Glu Pro Gln Val Tyr Thr Leu Pro Pro Ser Arg Glu Glu Met Thr Lys
           355                 360                 365
   Asn Gln Val Ser Leu Thr Cys Leu Val Lys Gly Phe Tyr Pro Ser Asp
       370                 375                 380
   Ile Ala Val Glu Trp Glu Ser Asn Gly Gln Pro Glu Asn Asn Tyr Lys
   385                 390                 395                 400
   Thr Thr Pro Pro Val Leu Asp Ser Asp Gly Ser Phe Phe Leu Tyr Ser
                   405                 410                 415
   Lys Leu Thr Val Asp Lys Ser Arg Trp Gln Gln Gly Asn Val Phe Ser
               420                 425                 430
   Cys Ser Val Met His Glu Ala Leu His Asn His Tyr Thr Gln Lys Ser
           435                 440                 445
   Leu Ser Leu Ser Pro Gly Lys
       450                 455
<210> 32
   <211> 654
   <212> DNA
   <213> artificial sequences
<220>
   <221> source
   <222> 1..654
   <223> /mol_type="DNA" /note="First fragment of Fc region of a human antibody" /organism="artificial sequences"
<400> 32
   gcacctgaac tcctgggggg accgtcagtc ttcctcttcc ccccaaaacc caaggacacc      60
   ctcatgatct cccggacccc tgaggtcaca tgcgtggtgg tggacgtgag ccacgaagac     120
   cctgaggtca agttcaactg gtacgtggac ggcgtggagg tgcataatgc caagacaaag     180
   ccgcgggagg agcagtacaa cagcacgtac cgtgtggtca gcgtcctcac cgtcctgcac     240
   caggactggc tgaatggcaa ggagtacaag tgcaaggtct ccaacaaagc cctcccagcc     300
   cccatcgaga aaaccatctc caaagccaaa gggcagcccc gagaaccaca ggtgtacacc     360
   ctgcccccat cccgggagga gatgaccaag aaccaggtca gcctgacctg cctggtcaaa     420
   ggcttctatc ccagcgacat cgccgtggag tgggagagca atgggcagcc ggagaacaac     480
   tacaagacca cgcctcccgt gctggactcc gacggctcct tcttcctcta tagcaagctc     540
   accgtggaca agagcaggtg gcagcagggg aacgtcttct catgctccgt gatgcatgag     600
   gctctgcaca accactacac gcagaagagc ctctccctgt ctccgggtaa gctt           654
<210> 33
   <211> 715
   <212> DNA
   <213> artificial sequences
<220>
   <221> source
   <222> 1..715
   <223> /mol_type="DNA" /note="Second fragment of Fc region of a human antibody" /organism="artificial sequences"
<400> 33
   agatctgagc ccaaatcttg tgacaaaact cacacatgcc caccgtgccc agcacctgaa      60
   ctcctggggg gaccgtcagt cttcctcttc cccccaaaac ccaaggacac cctcatgatc     120
   tcccggaccc ctgaggtcac atgcgtggtg gtggacgtga gccacgaaga ccctgaggtc     180
   aagttcaact ggtacgtgga cggcgtggag gtgcataatg ccaagacaaa gccgcgggag     240
   gagcagtaca acagcacgta ccgtgtggtc agcgtcctca ccgtcctgca ccaggactgg     300
   ctgaatggca aggagtacaa gtgcaaggtc tccaacaaag ccctcccagc ccccatcgag     360
   aaaaccatct ccaaagccaa agggcagccc cgagaaccac aggtgtacac cctgccccca     420
   tcccgggagg agatgaccaa gaaccaggtc agcctgacct gcctggtcaa aggcttctat     480
   cccagcgaca tcgccgtgga gtgggagagc aatgggcagc cggagaacaa ctacaagacc     540
   acgcctcccg tgctggactc cgacggctcc ttcttcctct acagcaagct caccgtggac     600
   aagagcaggt ggcagcaggg gaacgtcttc tcatgctccg tgatgcatga ggctctgcac     660
   aaccactaca cgcagaagag cctctccctg tctccgggta aatgagtgct ccgga          715
<210> 34
   <211> 51
   <212> DNA
   <213> artificial sequences
<220>
   <221> source
   <222> 1..51
   <223> /mol_type="DNA" /note="spacer chain" /organism="artificial sequences"
<400> 34
   aagcttggtg gaggcggttc aggcggaggt ggctctggcg gtggcggatc c 51
<210> 35
   <211> 2112
   <212> DNA
   <213> artificial sequences
<220>
   <221> source
   <222> 1..2112
   <223> /mol_type="DNA" /note="Monomeric SAP-ScFc" /organism="artificial sequences"
<400> 35
   atgaacaagc cgctcctttg gatctctgtc ctcaccagcc tcctggaagc ctttgctcac 60
   acagacctca gtgggaaggt gtttgtattt cctagagaat ctgttactga tcatgtaaac 120
   ttgatcacac cgctggagaa gcctctacag aactttacct tgtgttttcg agcctatagt 180
   gatctctctc gtgcctacag cctcttctcc tacaataccc aaggcaggga taatgagcta 240
   ctagtttata aagaaagagt tggagagtat agtctataca ttggaagaca caaagttaca 300
   tccaaagtta tcgaaaagtt cccggctcca gtgcacatct gtgtgagctg ggagtcctca 360
   tcaggtattg ctgaattttg gatcaatggg acacctttgg tgaaaaaggg tctgcgacag 420
   ggttactttg tggaagctca gcccaagatt gtcctggggc aggaacagga ttcctatggg 480
   ggcaagtttg ataggagcca gtcctttgtg ggagagattg gggatttgta catgtgggac 540
   tctgtgctgc ccccagaaaa tatcctgtct gcctatcagg gtacccctct ccctgccaat 600
   atcctggact ggcaggctct gaactatgaa atcagaggat atgtcatcat caaacccttg 660
   gtgtgggtcg agcccaaatc ttgtgacaaa actcacacat gcccaccgtg cccagcacct 720
   gaactcctgg ggggaccgtc agtcttcctc ttccccccaa aacccaagga caccctcatg 780
   atctcccgga cccctgaggt cacatgcgtg gtggtggacg tgagccacga agaccctgag 840
   gtcaagttca actggtacgt ggacggcgtg gaggtgcata atgccaagac aaagccgcgg 900
   gaggagcagt acaacagcac gtaccgtgtg gtcagcgtcc tcaccgtcct gcaccaggac 960
   tggctgaatg gcaaggagta caagtgcaag gtctccaaca aagccctccc agcccccatc 1020
   gagaaaacca tctccaaagc caaagggcag ccccgagaac cacaggtgta caccctgccc 1080
   ccatcccggg aggagatgac caagaaccag gtcagcctga cctgcctggt caaaggcttc 1140
   tatcccagcg acatcgccgt ggagtgggag agcaatgggc agccggagaa caactacaag 1200
   accacgcctc ccgtgctgga ctccgacggc tccttcttcc tctatagcaa gctcaccgtg 1260
   gacaagagca ggtggcagca ggggaacgtc ttctcatgct ccgtgatgca tgaggctctg    1320
   cacaaccact acacgcagaa gagcctctcc ctgtctccgg gtaagcttgg tggaggcggt    1380
   tcaggcggag gtggctctgg cggtggcgga tctgagccca aatcttgtga caaaactcac    1440
   acatgcccac cgtgcccagc acctgaactc ctggggggac cgtcagtctt cctcttcccc    1500
   ccaaaaccca aggacaccct catgatctcc cggacccctg aggtcacatg cgtggtggtg    1560
   gacgtgagcc acgaagaccc tgaggtcaag ttcaactggt acgtggacgg cgtggaggtg    1620
   cataatgcca agacaaagcc gcgggaggag cagtacaaca gcacgtaccg tgtggtcagc    1680
   gtcctcaccg tcctgcacca ggactggctg aatggcaagg agtacaagtg caaggtctcc    1740
   aacaaagccc tcccagcccc catcgagaaa accatctcca aagccaaagg gcagccccga    1800
   gaaccacagg tgtacaccct gcccccatcc cgggaggaga tgaccaagaa ccaggtcagc    1860
   ctgacctgcc tggtcaaagg cttctatccc agcgacatcg ccgtggagtg ggagagcaat    1920
   gggcagccgg agaacaacta caagaccacg cctcccgtgc tggactccga cggctccttc    1980
   ttcctctaca gcaagctcac cgtggacaag agcaggtggc agcaggggaa cgtcttctca    2040
   tgctccgtga tgcatgaggc tctgcacaac cactacacgc agaagagcct ctccctgtct    2100
   ccgggtaaat ga                                                        2112
<210> 36
   <211> 703
   <212> PRT
   <213> artificial sequences
<220>
   <221> SOURCE
   <222> 1..703
   <223> /mol_type="protein" /note="Monomeric SAP-ScFc" /organism="artificial sequences"
<400> 36
   Met Asn Lys Pro Leu Leu Trp Ile Ser Val Leu Thr Ser Leu Leu Glu
   1               5                   10                  15
   Ala Phe Ala His Thr Asp Leu Ser Gly Lys Val Phe Val Phe Pro Arg
               20                  25                  30
   Glu Ser Val Thr Asp His Val Asn Leu Ile Thr Pro Leu Glu Lys Pro
           35                  40                  45
   Leu Gln Asn Phe Thr Leu Cys Phe Arg Ala Tyr Ser Asp Leu Ser Arg
       50                  55                  60
   Ala Tyr Ser Leu Phe Ser Tyr Asn Thr Gln Gly Arg Asp Asn Glu Leu
   65                  70                  75                  80
   Leu Val Tyr Lys Glu Arg Val Gly Glu Tyr Ser Leu Tyr Ile Gly Arg
                   85                  90                  95
   His Lys Val Thr Ser Lys Val Ile Glu Lys Phe Pro Ala Pro Val His
               100                 105                 110
   Ile Cys Val Ser Trp Glu Ser Ser Ser Gly Ile Ala Glu Phe Trp Ile
           115                 120                 125
   Asn Gly Thr Pro Leu Val Lys Lys Gly Leu Arg Gln Gly Tyr Phe Val
       130                 135                 140
   Glu Ala Gln Pro Lys Ile Val Leu Gly Gln Glu Gln Asp Ser Tyr Gly
   145 150 155 160
   Gly Lys Phe Asp Arg Ser Gln Ser Phe Val Gly Glu Ile Gly Asp Leu
   165 170 175
   Tyr Met Trp Asp Ser Val Leu Pro Pro Glu Asn Ile Leu Ser Ala Tyr
   180 185 190
   Gln Gly Thr Pro Leu Pro Ala Asn Ile Leu Asp Trp Gln Ala Leu Asn
   195 200 205
   Tyr Glu Ile Arg Gly Tyr Val Ile Ile Lys Pro Leu Val Trp Val Glu
   210 215 220
   Pro Lys Ser Cys Asp Lys Thr His Thr Cys Pro Pro Cys Pro Ala Pro
   225 230 235 240
   Glu Leu Leu Gly Gly Pro Ser Val Phe Leu Phe Pro Pro Lys Pro Lys
   245 250 255
   Asp Thr Leu Met Ile Ser Arg Thr Pro Glu Val Thr Cys Val Val Val
   260 265 270
   Asp Val Ser His Glu Asp Pro Glu Val Lys Phe Asn Trp Tyr Val Asp
   275 280 285
   Gly Val Glu Val His Asn Ala Lys Thr Lys Pro Arg Glu Glu Gln Tyr
   290 295 300
   Asn Ser Thr Tyr Arg Val Val Ser Val Leu Thr Val Leu His Gln Asp
   305 310 315 320
   Trp Leu Asn Gly Lys Glu Tyr Lys Cys Lys Val Ser Asn Lys Ala Leu
   325 330 335
   Pro Ala Pro Ile Glu Lys Thr Ile Ser Lys Ala Lys Gly Gln Pro Arg
   340 345 350
   Glu Pro Gln Val Tyr Thr Leu Pro Pro Ser Arg Glu Glu Met Thr Lys
   355 360 365
   Asn Gln Val Ser Leu Thr Cys Leu Val Lys Gly Phe Tyr Pro Ser Asp
   370 375 380
   Ile Ala Val Glu Trp Glu Ser Asn Gly Gln Pro Glu Asn Asn Tyr Lys
   385 390 395 400
   Thr Thr Pro Pro Val Leu Asp Ser Asp Gly Ser Phe Phe Leu Tyr Ser
   405 410 415
   Lys Leu Thr Val Asp Lys Ser Arg Trp Gln Gln Gly Asn Val Phe Ser
   420 425 430
   Cys Ser Val Met His Glu Ala Leu His Asn His Tyr Thr Gln Lys Ser
   435 440 445
   Leu Ser Leu Ser Pro Gly Lys Leu Gly Gly Gly Gly Ser Gly Gly Gly
   450 455 460
   Gly Ser Gly Gly Gly Gly Ser Glu Pro Lys Ser Cys Asp Lys Thr His
   465 470 475 480
   Thr Cys Pro Pro Cys Pro Ala Pro Glu Leu Leu Gly Gly Pro Ser Val
   485 490 495
   Phe Leu Phe Pro Pro Lys Pro Lys Asp Thr Leu Met Ile Ser Arg Thr
   500 505 510
   Pro Glu Val Thr Cys Val Val Val Asp Val Ser His Glu Asp Pro Glu
   515 520 525
   Val Lys Phe Asn Trp Tyr Val Asp Gly Val Glu Val His Asn Ala Lys
   530 535 540
   Thr Lys Pro Arg Glu Glu Gln Tyr Asn Ser Thr Tyr Arg Val Val Ser
   545 550 555 560
   Val Leu Thr Val Leu His Gln Asp Trp Leu Asn Gly Lys Glu Tyr Lys
   565 570 575
   Cys Lys Val Ser Asn Lys Ala Leu Pro Ala Pro Ile Glu Lys Thr Ile
   580 585 590
   Ser Lys Ala Lys Gly Gln Pro Arg Glu Pro Gln Val Tyr Thr Leu Pro
   595 600 605
   Pro Ser Arg Glu Glu Met Thr Lys Asn Gln Val Ser Leu Thr Cys Leu
   610 615 620
   Val Lys Gly Phe Tyr Pro Ser Asp Ile Ala Val Glu Trp Glu Ser Asn
   625 630 635 640
   Gly Gln Pro Glu Asn Asn Tyr Lys Thr Thr Pro Pro Val Leu Asp Ser
                           645                 650                 655
   Asp Gly Ser Phe Phe Leu Tyr Ser Lys Leu Thr Val Asp Lys Ser Arg
               660                 665                 670
   Trp Gln Gln Gly Asn Val Phe Ser Cys Ser Val Met His Glu Ala Leu
           675                 680                 685
   His Asn His Tyr Thr Gln Lys Ser Leu Ser Leu Ser Pro Gly Lys
       690                 695                 700
<210> 37
   <211> 34
   <212> DNA
   <213> artificial sequences
<220>
   <221> source
   <222> 1..34
   <223> /mol_type="DNA" /note="First primer for the amplification of the SAP " /organism="artificial sequences"
<400> 37
   acttggtcga caccatgaac aagccgctgc tttg 34
<210> 38
   <211> 28
   <212> DNA
   <213> artificial sequences
<220>
   <221> source
   <222> 1..28
   <223> /mol_type="DNA" /note="Second primer for the amplification of the SAP" /organism="artificial sequences"
<400> 38
   actaggtcga cccacaccaa gggtttga 28
<210> 39
   <211> 23
   <212> DNA
   <213> artificial sequences
<220>
   <221> source
   <222> 1..23
   <223> /mol_type="DNA" /note="First primer for the amplification of the fragment of Fc r egion of a human antibody" /organism="artificial sequences"
<400> 39
   ctcgagccca aatcttgtga caa 23
<210> 40
   <211> 26
   <212> DNA
   <213> artificial sequences
<220>
   <221> source
   <222> 1..26
   <223> /mol_type="DNA" /note="Second primer for the amplification of the fragment of Fc region of a human antibody" /organism="artificial sequences"
<400> 40
   tccggagcac tcatttaccc ggagac 26
<210> 41
   <211> 28
   <212> DNA
   <213> artificial sequences
<220>
   <221> source
   <222> 1..28
   <223> /mol_type="DNA" /note="First primer for the amplification of the first fragment o f Fc region of a human antibody" /organism="artificial sequences"
<400> 41
   atactctcga gcccaaatct tgtgacaa 28
<210> 42
   <211> 28
   <212> DNA
   <213> artificial sequences
<220>
   <221> source
   <222> 1..28
   <223> /mol_type="DNA" /note="Second primer for the amplification of the first fragment of Fc region of a human antibody" /organism="artificial sequences"
<400> 42
   atctgaagct tacccggaga cagggaga 28
<210> 43
   <211> 31
   <212> DNA
   <213> artificial sequences
<220>
   <221> source
   <222> 1..31
   <223> /mol_type="DNA" /note="First primer for the amplification of the second fragment of Fc region of a human antibody" /organism="artificial sequences"
<400> 43
   aagtaagatc tgagcccaaa tcttgtgaca a 31
<210> 44
   <211> 31
   <212> DNA
   <213> artificial sequences
<220>
   <221> source
   <222> 1..31
   <223> /mol_type="DNA" /note="Second primer for the amplification of the second fragment of Fc region of a human antibody" /organism="artificial sequences"
<400> 44
   atctgtccgg agcactcatt tacccggaga c 31
<210> 45
   <211> 23
   <212> DNA
   <213> artificial sequences
<220>
   <221> source
   <222> 1..23
   <223> /mol_type="DNA" /note="First primer for the amplification of the spacer chain" /organism="artificial sequences"
<400> 45
   aagcttggtg gaggcggttc agg 23
<210> 46
   <211> 22
   <212> DNA
   <213> artificial sequences
<220>
   <221> source
   <222> 1..22
   <223> /mol_type="DNA" /note="Second primer for the amplification of the spacer chain" /organism="artificial sequences"
<400> 46
   ggatccgcca ccgccagagc ca 22